# EUROPEAN PATENT APPLICATION

(11) **EP 2 471 918 A1**
(43) Date of publication of application: **04.07.2012**
(21) Application number: 10820536.0
(22) Date of filing: 28.09.2010
(51) Int. Cl.: C12N 15/09, A61K 31/395, A61K 45/00, A61P 13/08, A61P 35/00, A61P 43/00, C07K 14/72, C12N 1/15, C12N 1/19, C12N 1/21, C12N 5/10, C12Q 1/02, C12Q 1/68, G01N 33/15, G01N 33/50, C07D 227/087

(54) **SCREENING METHOD**

(30) Priority: 29.09.2009 JP 2009225541
(71) Applicant: Takeda Pharmaceutical Company Limited, Osaka-shi, Osaka 541-0045 (JP)
(72) Inventor: WATANABE, Tatsuya, Kanagawa 251-0012 (JP); NAKAYAMA, Kazuhide, Kanagawa 251-0012 (JP); NAKATA, Daisuke, Kanagawa 251-0012 (JP); KUSAKA, Masami, Osaka-shi Osaka 532-8686 (JP)
(74) Representative: König, Gregor Sebastian
(86) International application number: PCT/JP2010/066874
(87) International publication number: WO 2011/040421

(57) **Abstract**

The present invention provides a novel variant androgen receptor (ARaiv) lacking ligand binding domain, a nucleic acid encoding the same and use thereof. That is, the present invention provides a method of screening for a substance for the prophylaxis or treatment of cancer, which includes contacting an ARaiv protein or ARaiv-producing cell with a test compound, measuring the activity of ARaiv (e.g., transcription regulating action of androgen responsive gene) or expression level thereof, and selecting a compound that suppresses the activity or expression level.

SEQUENCE LISTING

<110>  Takeda Pharmaceutical Company Limited

<120>  Screening Method

<130>  091606

<150>  JP 2009-225541
<151>  2009-09-29

<160>  52

<170>  PatentIn version 3.5

<210>  1
<211>  16
<212>  PRT
<213>  Homo sapiens

<400>  1

Glu Lys Phe Arg Val Gly Asn Cys Lys His Leu Lys Met Thr Arg Pro
1               5                   10                  15


<210>  2
<211>  15
<212>  PRT
<213>  Homo sapiens

<400>  2

Gly Ser Ser Leu Leu Pro Ala Thr Ser Lys Lys Met Cys Leu Ser
1               5                   10                  15


<210>  3
<211>  4422
<212>  DNA
<213>  Homo sapiens


<220>
<221>  CDS
<222>  (1116)..(3047)

<400>  3
cgagatcccg gggagccagc ttgctgggag agcgggacgg tccggagcaa gcccagaggc      60

agaggaggcg acagagggaa aaagggccga gctagccgct ccagtgctgt acaggagccg     120

aagggacgca ccacgccagc cccagcccgg ctccagcgac agccaacgcc tcttgcagcg     180

cggcggcttc gaagccgccg cccggagctg ccctttcctc ttcggtgaag tttttaaaag     240

ctgctaaaga ctcggaggaa gcaaggaaag tgcctggtag gactgacggc tgcctttgtc     300

ctcctcctct ccaccccgcc tccccccacc ctgccttccc cccctccccc gtcttctctc     360

ccgcagctgc ctcagtcggc tactctcagc caacccccct caccaccctt ctccccaccc     420

gcccccccgc ccccgtcggc ccagcgctgc cagcccgagt ttgcagagag gtaactccct     480

ttggctgcga gcgggcgagc tagctgcaca ttgcaaagaa ggctcttagg agccaggcga     540

ctggggagcg gcttcagcac tgcagccacg acccgcctgg ttaggctgca cgcggagaga 600

accctctgtt ttcccccact ctctctccac ctcctcctgc cttccccacc ccgagtgcgg 660

agccagagat caaaagatga aaaggcagtc aggtcttcag tagccaaaaa acaaaacaaa 720

caaaaacaaa aaagccgaaa taaaagaaaa agataataac tcagttctta tttgcaccta 780

cttcagtgga cactgaattt ggaaggtgga ggattttgtt tttttctttt aagatctggg 840

catcttttga atctaccctt caagtattaa gagacagact gtgagcctag cagggcagat 900

cttgtccacc gtgtgtcttc ttctgcacga gactttgagg ctgtcagagc gctttttgcg 960

tggttgctcc cgcaagtttc cttctctgga gcttcccgca ggtgggcagc tagctgcagc 1020

gactaccgca tcatcacagc ctgttgaact cttctgagca agagaagggg aggcggggta 1080

agggaagtag gtggaagatt cagccaagct caagg atg gaa gtg cag tta ggg 1133
Met Glu Val Gln Leu Gly
1               5

ctg gga agg gtc tac cct cgg ccg ccg tcc aag acc tac cga gga gct 1181
Leu Gly Arg Val Tyr Pro Arg Pro Pro Ser Lys Thr Tyr Arg Gly Ala
            10                  15                  20

ttc cag aat ctg ttc cag agc gtg cgc gaa gtg atc cag aac ccg ggc 1229
Phe Gln Asn Leu Phe Gln Ser Val Arg Glu Val Ile Gln Asn Pro Gly
        25                  30                  35

ccc agg cac cca gag gcc gcg agc gca gca cct ccc ggc gcc agt ttg 1277
Pro Arg His Pro Glu Ala Ala Ser Ala Ala Pro Pro Gly Ala Ser Leu
    40                  45                  50

ctg ctg ctg cag cag cag cag cag cag cag cag cag cag cag cag cag 1325
Leu Leu Leu Gln Gln Gln Gln Gln Gln Gln Gln Gln Gln Gln Gln Gln
55                  60                  65                  70

cag cag cag cag cag cag cag cag cag cag cag cag caa gag act agc 1373
Gln Gln Gln Gln Gln Gln Gln Gln Gln Gln Gln Gln Gln Glu Thr Ser
                75                  80                  85

ccc agg cag cag cag cag cag cag ggt gag gat ggt tct ccc caa gcc 1421
Pro Arg Gln Gln Gln Gln Gln Gln Gly Glu Asp Gly Ser Pro Gln Ala
            90                  95                  100

cat cgt aga ggc ccc aca ggc tac ctg gtc ctg gat gag gaa cag caa 1469
His Arg Arg Gly Pro Thr Gly Tyr Leu Val Leu Asp Glu Glu Gln Gln
        105                 110                 115

cct tca cag ccg cag tcg gcc ctg gag tgc cac ccc gag aga ggt tgc 1517
Pro Ser Gln Pro Gln Ser Ala Leu Glu Cys His Pro Glu Arg Gly Cys
    120                 125                 130

gtc cca gag cct gga gcc gcc gtg gcc gcc agc aag ggg ctg ccg cag 1565
Val Pro Glu Pro Gly Ala Ala Val Ala Ala Ser Lys Gly Leu Pro Gln
135                 140                 145                 150

cag ctg cca gca cct ccg gac gag gat gac tca gct gcc cca tcc acg 1613
Gln Leu Pro Ala Pro Pro Asp Glu Asp Asp Ser Ala Ala Pro Ser Thr
                155                 160                 165

ttg tcc ctg ctg ggc ccc act ttc ccc ggc tta agc agc tgc tcc gct 1661
Leu Ser Leu Leu Gly Pro Thr Phe Pro Gly Leu Ser Ser Cys Ser Ala

            170                 175                 180

gac ctt aaa gac atc ctg agc gag gcc agc acc atg caa ctc ctt cag 1709
Asp Leu Lys Asp Ile Leu Ser Glu Ala Ser Thr Met Gln Leu Leu Gln
        185                 190                 195

caa cag cag cag gaa gca gta tcc gaa ggc agc agc agc ggg aga gcg 1757
Gln Gln Gln Gln Glu Ala Val Ser Glu Gly Ser Ser Ser Gly Arg Ala
    200                 205                 210

agg gag gcc tcg ggg gct ccc act tcc tcc aag gac aat tac tta ggg 1805
Arg Glu Ala Ser Gly Ala Pro Thr Ser Ser Lys Asp Asn Tyr Leu Gly
215                 220                 225                 230

ggc act tcg acc att tct gac aac gcc aag gag ttg tgt aag gca gtg 1853
Gly Thr Ser Thr Ile Ser Asp Asn Ala Lys Glu Leu Cys Lys Ala Val
                235                 240                 245

tcg gtg tcc atg ggc ctg ggt gtg gag gcg ttg gag cat ctg agt cca 1901
Ser Val Ser Met Gly Leu Gly Val Glu Ala Leu Glu His Leu Ser Pro
            250                 255                 260

ggg gaa cag ctt cgg ggg gat tgc atg tac gcc cca ctt ttg gga gtt 1949
Gly Glu Gln Leu Arg Gly Asp Cys Met Tyr Ala Pro Leu Leu Gly Val
        265                 270                 275

cca ccc gct gtg cgt ccc act cct tgt gcc cca ttg gcc gaa tgc aaa 1997
Pro Pro Ala Val Arg Pro Thr Pro Cys Ala Pro Leu Ala Glu Cys Lys
    280                 285                 290

ggt tct ctg cta gac gac agc gca ggc aag agc act gaa gat act gct 2045
Gly Ser Leu Leu Asp Asp Ser Ala Gly Lys Ser Thr Glu Asp Thr Ala
295                 300                 305                 310

gag tat tcc cct ttc aag gga ggt tac acc aaa ggg cta gaa ggc gag 2093
Glu Tyr Ser Pro Phe Lys Gly Gly Tyr Thr Lys Gly Leu Glu Gly Glu
                315                 320                 325

agc cta ggc tgc tct ggc agc gct gca gca ggg agc tcc ggg aca ctt 2141
Ser Leu Gly Cys Ser Gly Ser Ala Ala Ala Gly Ser Ser Gly Thr Leu
            330                 335                 340

gaa ctg ccg tct acc ctg tct ctc tac aag tcc gga gca ctg gac gag 2189
Glu Leu Pro Ser Thr Leu Ser Leu Tyr Lys Ser Gly Ala Leu Asp Glu
        345                 350                 355

gca gct gcg tac cag agt cgc gac tac tac aac ttt cca ctg gct ctg 2237
Ala Ala Ala Tyr Gln Ser Arg Asp Tyr Tyr Asn Phe Pro Leu Ala Leu
    360                 365                 370

gcc gga ccg ccg ccc cct ccg ccg cct ccc cat ccc cac gct cgc atc 2285
Ala Gly Pro Pro Pro Pro Pro Pro Pro Pro His Pro His Ala Arg Ile
375                 380                 385                 390

aag ctg gag aac ccg ctg gac tac ggc agc gcc tgg gcg gct gcg gcg 2333
Lys Leu Glu Asn Pro Leu Asp Tyr Gly Ser Ala Trp Ala Ala Ala Ala
                395                 400                 405

gcg cag tgc cgc tat ggg gac ctg gcg agc ctg cat ggc gcg ggt gca 2381
Ala Gln Cys Arg Tyr Gly Asp Leu Ala Ser Leu His Gly Ala Gly Ala
            410                 415                 420

gcg gga ccc ggt tct ggg tca ccc tca gcc gcc gct tcc tca tcc tgg 2429
Ala Gly Pro Gly Ser Gly Ser Pro Ser Ala Ala Ala Ser Ser Ser Trp
        425                 430                 435

cac act ctc ttc aca gcc gaa gaa ggc cag ttg tat gga ccg tgt ggt 2477
His Thr Leu Phe Thr Ala Glu Glu Gly Gln Leu Tyr Gly Pro Cys Gly
    440                 445                 450

ggt ggt ggg ggt ggt ggc ggc ggc ggc ggc ggc ggc ggc ggc ggc ggc 2525
Gly Gly Gly Gly Gly Gly Gly Gly Gly Gly Gly Gly Gly Gly Gly Gly
455                 460                 465                 470

ggc ggc gag gcg gga gct gta gcc ccc tac ggc tac act cgg ccc cct 2573
Gly Gly Glu Ala Gly Ala Val Ala Pro Tyr Gly Tyr Thr Arg Pro Pro
                475                 480                 485

cag ggg ctg gcg ggc cag gaa agc gac ttc acc gca cct gat gtg tgg 2621
Gln Gly Leu Ala Gly Gln Glu Ser Asp Phe Thr Ala Pro Asp Val Trp
            490                 495                 500

tac cct ggc ggc atg gtg agc aga gtg ccc tat ccc agt ccc act tgt 2669
Tyr Pro Gly Gly Met Val Ser Arg Val Pro Tyr Pro Ser Pro Thr Cys
        505                 510                 515

gtc aaa agc gaa atg ggc ccc tgg atg gat agc tac tcc gga cct tac 2717
Val Lys Ser Glu Met Gly Pro Trp Met Asp Ser Tyr Ser Gly Pro Tyr
    520                 525                 530

ggg gac atg cgt ttg gag act gcc agg gac cat gtt ttg ccc att gac 2765
Gly Asp Met Arg Leu Glu Thr Ala Arg Asp His Val Leu Pro Ile Asp
535                 540                 545                 550

tat tac ttt cca ccc cag aag acc tgc ctg atc tgt gga gat gaa gct 2813
Tyr Tyr Phe Pro Pro Gln Lys Thr Cys Leu Ile Cys Gly Asp Glu Ala
                555                 560                 565

tct ggg tgt cac tat gga gct ctc aca tgt gga agc tgc aag gtc ttc 2861
Ser Gly Cys His Tyr Gly Ala Leu Thr Cys Gly Ser Cys Lys Val Phe
            570                 575                 580

ttc aaa aga gcc gct gaa ggg aaa cag aag tac ctg tgc gcc agc aga 2909
Phe Lys Arg Ala Ala Glu Gly Lys Gln Lys Tyr Leu Cys Ala Ser Arg
        585                 590                 595

aat gat tgc act att gat aaa ttc cga agg aaa aat tgt cca tct tgt 2957
Asn Asp Cys Thr Ile Asp Lys Phe Arg Arg Lys Asn Cys Pro Ser Cys
    600                 605                 610

cgt ctt cgg aaa tgt tat gaa gca ggg atg act ctg gga gaa aaa ttc 3005
Arg Leu Arg Lys Cys Tyr Glu Ala Gly Met Thr Leu Gly Glu Lys Phe
615                 620                 625                 630

cgg gtt ggc aat tgc aag cat ctc aaa atg acc aga ccc tga 3047
Arg Val Gly Asn Cys Lys His Leu Lys Met Thr Arg Pro
                635                 640

agaaaggctg acttgcctca ttcaaaatga gggctctaga gggctctagt ggatagtctg 3107

gagaaacctg gcgtctgagg cttaggagct taggtttttg ctcctcaaca cagactttga 3167

cgttggggtt gggggctact ctcttgattg ctgactccct ccagcgggac caatagtgtt 3227

ttcctacctc acagggatgt tgtgaggacg ggctgtagaa gtaatagtgg ttaccattca 3287

tgtagttgtg agtatcatga ttattgtttc ctgtaatgtg gcttggcatt ggcaaagtgc 3347

tttttgattg ttcttgatca catatgatgg gggccaggca ctgactcagg cggatgcagt 3407

gaagctctgg ctcagtcgct tgcttttcgt ggtgtgctgc caggaagaaa ctttgctgat 3467

gggactcaag gtgtcacctt ggacaagaag caactgtgtc tgtctgaggt tcctgtggcc 3527

atctttattt gtgtattagg caattcgtat ttccccctta ggttctagcc ttctggatcc 3587

cagccagtga cctagatctt agcctcaggc cctgtcactg agctgaaggt agtagctgat 3647

ccacagaagt tcagtaaaca aggaccagat ttctgcttct ccaggagaag aagccagcca 3707

acccctctct tcaaacacac tgagagacta cagtccgact ttccctctta catctagcct 3767

tactgtagcc acactccttg attgctctct cacatcacat gcttctcttc atcagttgta 3827

agcctctcat tcttctccca agccagactc aaatattgta ttgatgtcaa agaagaatca 3887

cttagagttt ggaatatctt gttctctctc tgctccatag cttccatatt gacaccagtt 3947

tctttctagt ggagaagtgg agtctgtgaa gccagggaaa cacacatgtg agagtcagaa 4007

ggactctccc tgacttgcct ggggcctgtc tttcccacct tctccagtct gtctaaacac 4067

acacacacac acacacacac acacacacac gctctctctc tctctccccc cccaacacac 4127

acacactctc tctctctctc acacacacac acatacacac acacttcttt ctctttcccc 4187

tgactcagca acattctgga gaaaagccaa ggaaggactt caggagggga gtttccccct 4247

tctcagggca gaattttaat ctccagacca acaagaagtt ccctaatgtg gattgaaagg 4307

ctaatgaggt ttatttttaa ctactttcta tttgtttgaa tgttgcatat ttctactagt 4367

gaaattttcc cttaataaag ccattaatac accaaaaaaa aaaaaaaaaa aaaaa 4422

<210> 4
<211> 643
<212> PRT
<213> Homo sapiens

<400> 4
   Met Glu Val Gln Leu Gly Leu Gly Arg Val Tyr Pro Arg Pro Pro Ser
   1 5 10 15
   Lys Thr Tyr Arg Gly Ala Phe Gln Asn Leu Phe Gln Ser Val Arg Glu
   20 25 30
   Val Ile Gln Asn Pro Gly Pro Arg His Pro Glu Ala Ala Ser Ala Ala
   35 40 45
   Pro Pro Gly Ala Ser Leu Leu Leu Leu Gln Gln Gln Gln Gln Gln Gln
   50 55 60
   Gln Gln Gln Gln Gln Gln Gln Gln Gln Gln Gln Gln Gln Gln Gln Gln
   65 70 75 80
   Gln Gln Gln Glu Thr Ser Pro Arg Gln Gln Gln Gln Gln Gln Gly Glu
   85 90 95
   Asp Gly Ser Pro Gln Ala His Arg Arg Gly Pro Thr Gly Tyr Leu Val
               100                 105                 110
   Leu Asp Glu Glu Gln Gln Pro Ser Gln Pro Gln Ser Ala Leu Glu Cys
           115                 120                 125
   His Pro Glu Arg Gly Cys Val Pro Glu Pro Gly Ala Ala Val Ala Ala
       130                 135                 140
   Ser Lys Gly Leu Pro Gln Gln Leu Pro Ala Pro Pro Asp Glu Asp Asp
   145                 150                 155                 160
   Ser Ala Ala Pro Ser Thr Leu Ser Leu Leu Gly Pro Thr Phe Pro Gly
                   165                 170                 175
   Leu Ser Ser Cys Ser Ala Asp Leu Lys Asp Ile Leu Ser Glu Ala Ser
               180                 185                 190
   Thr Met Gln Leu Leu Gln Gln Gln Gln Gln Glu Ala Val Ser Glu Gly
           195                 200                 205
   Ser Ser Ser Gly Arg Ala Arg Glu Ala Ser Gly Ala Pro Thr Ser Ser
       210                 215                 220
   Lys Asp Asn Tyr Leu Gly Gly Thr Ser Thr Ile Ser Asp Asn Ala Lys
   225                 230                 235                 240
   Glu Leu Cys Lys Ala Val Ser Val Ser Met Gly Leu Gly Val Glu Ala
                   245                 250                 255
   Leu Glu His Leu Ser Pro Gly Glu Gln Leu Arg Gly Asp Cys Met Tyr
               260                 265                 270
   Ala Pro Leu Leu Gly Val Pro Pro Ala Val Arg Pro Thr Pro Cys Ala
           275                 280                 285
   Pro Leu Ala Glu Cys Lys Gly Ser Leu Leu Asp Asp Ser Ala Gly Lys
       290                 295                 300
   Ser Thr Glu Asp Thr Ala Glu Tyr Ser Pro Phe Lys Gly Gly Tyr Thr
   305                 310                 315                 320
   Lys Gly Leu Glu Gly Glu Ser Leu Gly Cys Ser Gly Ser Ala Ala Ala
                   325                 330                 335
   Gly Ser Ser Gly Thr Leu Glu Leu Pro Ser Thr Leu Ser Leu Tyr Lys
               340                 345                 350
   Ser Gly Ala Leu Asp Glu Ala Ala Ala Tyr Gln Ser Arg Asp Tyr Tyr
           355                 360                 365
   Asn Phe Pro Leu Ala Leu Ala Gly Pro Pro Pro Pro Pro Pro Pro Pro
       370                 375                 380
   His Pro His Ala Arg Ile Lys Leu Glu Asn Pro Leu Asp Tyr Gly Ser
   385                 390                 395                 400
   Ala Trp Ala Ala Ala Ala Ala Gln Cys Arg Tyr Gly Asp Leu Ala Ser
                   405                 410                 415
   Leu His Gly Ala Gly Ala Ala Gly Pro Gly Ser Gly Ser Pro Ser Ala
               420                 425                 430
   Ala Ala Ser Ser Ser Trp His Thr Leu Phe Thr Ala Glu Glu Gly Gln
           435                 440                 445
   Leu Tyr Gly Pro Cys Gly Gly Gly Gly Gly Gly Gly Gly Gly Gly Gly
       450                 455                 460
   Gly Gly Gly Gly Gly Gly Gly Gly Glu Ala Gly Ala Val Ala Pro Tyr
   465                 470                 475                 480
   Gly Tyr Thr Arg Pro Pro Gln Gly Leu Ala Gly Gln Glu Ser Asp Phe
                   485                 490                 495
   Thr Ala Pro Asp Val Trp Tyr Pro Gly Gly Met Val Ser Arg Val Pro
               500                 505                 510
   Tyr Pro Ser Pro Thr Cys Val Lys Ser Glu Met Gly Pro Trp Met Asp
           515                 520                 525
   Ser Tyr Ser Gly Pro Tyr Gly Asp Met Arg Leu Glu Thr Ala Arg Asp
       530                 535                 540
   His Val Leu Pro Ile Asp Tyr Tyr Phe Pro Pro Gln Lys Thr Cys Leu
   545                 550                 555                 560
   Ile Cys Gly Asp Glu Ala Ser Gly Cys His Tyr Gly Ala Leu Thr Cys
                   565                 570                 575
   Gly Ser Cys Lys Val Phe Phe Lys Arg Ala Ala Glu Gly Lys Gln Lys
               580                 585                 590
   Tyr Leu Cys Ala Ser Arg Asn Asp Cys Thr Ile Asp Lys Phe Arg Arg
           595                 600                 605
   Lys Asn Cys Pro Ser Cys Arg Leu Arg Lys Cys Tyr Glu Ala Gly Met
   610                 615                 620
   Thr Leu Gly Glu Lys Phe Arg Val Gly Asn Cys Lys His Leu Lys Met
   625                 630                 635                 640
   Thr Arg Pro

<210>  5
<211>  4174
<212>  DNA
<213>  Homo sapiens

<220>
<221>  CDS
<222>  (1116)..(3044)

<400>  5
cgagatcccg gggagccagc ttgctgggag agcgggacgg tccggagcaa gcccagaggc      60

agaggaggcg acagagggaa aaagggccga gctagccgct ccagtgctgt acaggagccg     120

aagggacgca ccacgccagc cccagcccgg ctccagcgac agccaacgcc tcttgcagcg     180

cggcggcttc gaagccgccg cccggagctg ccctttcctc ttcggtgaag tttttaaaag     240

ctgctaaaga ctcggaggaa gcaaggaaag tgcctggtag gactgacggc tgcctttgtc     300

ctcctcctct ccaccccgcc tccccccacc ctgccttccc cccctccccc gtcttctctc     360

ccgcagctgc ctcagtcggc tactctcagc caacccccct caccaccctt ctccccaccc     420

gcccccccgc ccccgtcggc ccagcgctgc cagcccgagt ttgcagagag gtaactccct     480

ttggctgcga gcgggcgagc tagctgcaca ttgcaaagaa ggctcttagg agccaggcga     540

ctggggagcg gcttcagcac tgcagccacg acccgcctgg ttaggctgca cgcggagaga     600

accctctgtt ttcccccact ctctctccac ctcctcctgc cttccccacc ccgagtgcgg     660

agccagagat caaaagatga aaaggcagtc aggtcttcag tagccaaaaa acaaaacaaa     720

caaaaacaaa aaagccgaaa taaaagaaaa agataataac tcagttctta tttgcaccta     780

cttcagtgga cactgaattt ggaaggtgga ggattttgtt tttttctttt aagatctggg     840

catcttttga atctaccctt caagtattaa gagacagact gtgagcctag cagggcagat     900

cttgtccacc gtgtgtcttc ttctgcacga gactttgagg ctgtcagagc gctttttgcg     960

tggttgctcc cgcaagtttc cttctctgga gcttcccgca ggtgggcagc tagctgcagc    1020

gactaccgca tcatcacagc ctgttgaact cttctgagca agagaagggg aggcggggta    1080

agggaagtag gtggaagatt cagccaagct caagg atg gaa gtg cag tta ggg       1133
                                       Met Glu Val Gln Leu Gly
                                       1               5

ctg gga agg gtc tac cct cgg ccg ccg tcc aag acc tac cga gga gct      1181
Leu Gly Arg Val Tyr Pro Arg Pro Pro Ser Lys Thr Tyr Arg Gly Ala
            10                  15                  20

ttc cag aat ctg ttc cag agc gtg cgc gaa gtg atc cag aac ccg ggc 1229
Phe Gln Asn Leu Phe Gln Ser Val Arg Glu Val Ile Gln Asn Pro Gly
        25                  30                  35

ccc agg cac cca gag gcc gcg agc gca gca cct ccc ggc gcc agt ttg 1277
Pro Arg His Pro Glu Ala Ala Ser Ala Ala Pro Pro Gly Ala Ser Leu
    40                  45                  50

ctg ctg ctg cag cag cag cag cag cag cag cag cag cag cag cag cag 1325
Leu Leu Leu Gln Gln Gln Gln Gln Gln Gln Gln Gln Gln Gln Gln Gln
55                  60                  65                  70

cag cag cag cag cag cag cag cag cag cag cag cag caa gag act agc 1373
Gln Gln Gln Gln Gln Gln Gln Gln Gln Gln Gln Gln Gln Glu Thr Ser
                75                  80                  85

ccc agg cag cag cag cag cag cag ggt gag gat ggt tct ccc caa gcc 1421
Pro Arg Gln Gln Gln Gln Gln Gln Gly Glu Asp Gly Ser Pro Gln Ala
            90                  95                  100

cat cgt aga ggc ccc aca ggc tac ctg gtc ctg gat gag gaa cag caa 1469
His Arg Arg Gly Pro Thr Gly Tyr Leu Val Leu Asp Glu Glu Gln Gln
        105                 110                 115

cct tca cag ccg cag tcg gcc ctg gag tgc cac ccc gag aga ggt tgc 1517
Pro Ser Gln Pro Gln Ser Ala Leu Glu Cys His Pro Glu Arg Gly Cys
    120                 125                 130

gtc cca gag cct gga gcc gcc gtg gcc gcc agc aag ggg ctg ccg cag 1565
Val Pro Glu Pro Gly Ala Ala Val Ala Ala Ser Lys Gly Leu Pro Gln
135                 140                 145                 150

cag ctg cca gca cct ccg gac gag gat gac tca gct gcc cca tcc acg 1613
Gln Leu Pro Ala Pro Pro Asp Glu Asp Asp Ser Ala Ala Pro Ser Thr
                155                 160                 165

ttg tcc ctg ctg ggc ccc act ttc ccc ggc tta agc agc tgc tcc gct 1661
Leu Ser Leu Leu Gly Pro Thr Phe Pro Gly Leu Ser Ser Cys Ser Ala
            170                 175                 180

gac ctt aaa gac atc ctg agc gag gcc agc acc atg caa ctc ctt cag 1709
Asp Leu Lys Asp Ile Leu Ser Glu Ala Ser Thr Met Gln Leu Leu Gln
        185                 190                 195

caa cag cag cag gaa gca gta tcc gaa ggc agc agc agc ggg aga gcg 1757
Gln Gln Gln Gln Glu Ala Val Ser Glu Gly Ser Ser Ser Gly Arg Ala
    200                 205                 210

agg gag gcc tcg ggg gct ccc act tcc tcc aag gac aat tac tta ggg 1805
Arg Glu Ala Ser Gly Ala Pro Thr Ser Ser Lys Asp Asn Tyr Leu Gly
215                 220                 225                 230

ggc act tcg acc att tct gac aac gcc aag gag ttg tgt aag gca gtg 1853
Gly Thr Ser Thr Ile Ser Asp Asn Ala Lys Glu Leu Cys Lys Ala Val
                235                 240                 245

tcg gtg tcc atg ggc ctg ggt gtg gag gcg ttg gag cat ctg agt cca 1901
Ser Val Ser Met Gly Leu Gly Val Glu Ala Leu Glu His Leu Ser Pro
            250                 255                 260

ggg gaa cag ctt cgg ggg gat tgc atg tac gcc cca ctt ttg gga gtt 1949
Gly Glu Gln Leu Arg Gly Asp Cys Met Tyr Ala Pro Leu Leu Gly Val
        265                 270                 275

cca ccc gct gtg cgt ccc act cct tgt gcc cca ttg gcc gaa tgc aaa 1997
Pro Pro Ala Val Arg Pro Thr Pro Cys Ala Pro Leu Ala Glu Cys Lys
    280                 285                 290

ggt tct ctg cta gac gac agc gca ggc aag agc act gaa gat act gct 2045
Gly Ser Leu Leu Asp Asp Ser Ala Gly Lys Ser Thr Glu Asp Thr Ala
295                 300                 305                 310

gag tat tcc cct ttc aag gga ggt tac acc aaa ggg cta gaa ggc gag 2093
Glu Tyr Ser Pro Phe Lys Gly Gly Tyr Thr Lys Gly Leu Glu Gly Glu
                315                 320                 325

agc cta ggc tgc tct ggc agc gct gca gca ggg agc tcc ggg aca ctt 2141
Ser Leu Gly Cys Ser Gly Ser Ala Ala Ala Gly Ser Ser Gly Thr Leu
            330                 335                 340

gaa ctg ccg tct acc ctg tct ctc tac aag tcc gga gca ctg gac gag 2189
Glu Leu Pro Ser Thr Leu Ser Leu Tyr Lys Ser Gly Ala Leu Asp Glu
        345                 350                 355

gca gct gcg tac cag agt cgc gac tac tac aac ttt cca ctg gct ctg 2237
Ala Ala Ala Tyr Gln Ser Arg Asp Tyr Tyr Asn Phe Pro Leu Ala Leu
    360                 365                 370

gcc gga ccg ccg ccc cct ccg ccg cct ccc cat ccc cac gct cgc atc 2285
Ala Gly Pro Pro Pro Pro Pro Pro Pro Pro His Pro His Ala Arg Ile
375                 380                 385                 390

aag ctg gag aac ccg ctg gac tac ggc agc gcc tgg gcg gct gcg gcg 2333
Lys Leu Glu Asn Pro Leu Asp Tyr Gly Ser Ala Trp Ala Ala Ala Ala
                395                 400                 405

gcg cag tgc cgc tat ggg gac ctg gcg agc ctg cat ggc gcg ggt gca 2381
Ala Gln Cys Arg Tyr Gly Asp Leu Ala Ser Leu His Gly Ala Gly Ala
            410                 415                 420

gcg gga ccc ggt tct ggg tca ccc tca gcc gcc gct tcc tca tcc tgg 2429
Ala Gly Pro Gly Ser Gly Ser Pro Ser Ala Ala Ala Ser Ser Ser Trp
        425                 430                 435

cac act ctc ttc aca gcc gaa gaa ggc cag ttg tat gga ccg tgt ggt 2477
His Thr Leu Phe Thr Ala Glu Glu Gly Gln Leu Tyr Gly Pro Cys Gly
    440                 445                 450

ggt ggt ggg ggt ggt ggc ggc ggc ggc ggc ggc ggc ggc ggc ggc ggc 2525
Gly Gly Gly Gly Gly Gly Gly Gly Gly Gly Gly Gly Gly Gly Gly Gly
455                 460                 465                 470

ggc ggc gag gcg gga gct gta gcc ccc tac ggc tac act cgg ccc cct 2573
Gly Gly Glu Ala Gly Ala Val Ala Pro Tyr Gly Tyr Thr Arg Pro Pro
                475                 480                 485

cag ggg ctg gcg ggc cag gaa agc gac ttc acc gca cct gat gtg tgg 2621
Gln Gly Leu Ala Gly Gln Glu Ser Asp Phe Thr Ala Pro Asp Val Trp
            490                 495                 500

tac cct ggc ggc atg gtg agc aga gtg ccc tat ccc agt ccc act tgt 2669
Tyr Pro Gly Gly Met Val Ser Arg Val Pro Tyr Pro Ser Pro Thr Cys
        505                 510                 515

gtc aaa agc gaa atg ggc ccc tgg atg gat agc tac tcc gga cct tac 2717
Val Lys Ser Glu Met Gly Pro Trp Met Asp Ser Tyr Ser Gly Pro Tyr
    520                 525                 530

ggg gac atg cgt ttg gag act gcc agg gac cat gtt ttg ccc att gac 2765

Gly Asp Met Arg Leu Glu Thr Ala Arg Asp His Val Leu Pro Ile Asp
535                 540                 545                 550

tat tac ttt cca ccc cag aag acc tgc ctg atc tgt gga gat gaa gct      2813
Tyr Tyr Phe Pro Pro Gln Lys Thr Cys Leu Ile Cys Gly Asp Glu Ala
                555                 560                 565

tct ggg tgt cac tat gga gct ctc aca tgt gga agc tgc aag gtc ttc      2861
Ser Gly Cys His Tyr Gly Ala Leu Thr Cys Gly Ser Cys Lys Val Phe
            570                 575                 580

ttc aaa aga gcc gct gaa ggg aaa cag aag tac ctg tgc gcc agc aga      2909
Phe Lys Arg Ala Ala Glu Gly Lys Gln Lys Tyr Leu Cys Ala Ser Arg
        585                 590                 595

aat gat tgc act att gat aaa ttc cga agg aaa aat tgt cca tct tgt      2957
Asn Asp Cys Thr Ile Asp Lys Phe Arg Arg Lys Asn Cys Pro Ser Cys
    600                 605                 610

cgt ctt cgg aaa tgt tat gaa gca ggg atg act ctg gga ggg tcc tcg      3005
Arg Leu Arg Lys Cys Tyr Glu Ala Gly Met Thr Leu Gly Gly Ser Ser
615                 620                 625                 630

ctg ctg cct gct act tcc aaa aag atg tgt ctt tca tga gaaaaacaag      3054
Leu Leu Pro Ala Thr Ser Lys Lys Met Cys Leu Ser
                635                 640

atcattaatc cacttcgatt tggaaatgga atttgaagaa aggcaagcct atttctgagt    3114

gcctgcaact gtagcctcat acccaattat tcattattag cctggaaaac ccaagtgcct    3174

agaatccaac cctctcccct ctcctcttaa gtctaattta gaccagttgt ctatctctgg    3234

ctttctgtga ggtgttcaat accttgtctg cctatgtgca catttataga caacaactag    3294

ttctcttatc ctggagcagg gccatgtgtg gatcttcata tagataacta tatcctcccc    3354

atcctcacag ggcagtagta ttatttaaac agaacaaagt acctcacatg aattgaccca    3414

ggctggatga gagacaattt caaaagaatc atctcaagta gcgtccagta ctcccaaaca    3474

tcacaggtag atgttctgtg agtggctttc caagcatcca catcaaatga gactcagata    3534

tctgagaaaa ctcaaccttg ttttggtttg cttggtgcac cccaaagaaa tccaacaatt    3594

gaggtctaca gtggagaaga agtaggactg gggtcaggga gtacagaggc aaaggcagga    3654

agggtgacaa agtgattgac aagaaaaaat gttctccata tgaatgttgc agccccatgt    3714

tgagggttct tatacactca actgtcaatt atttagcctt ctgtgaatta tgtatagtat    3774

aaaagatagg gactctcaag tagggaacct cttggcttgc catctggcaa tatgaattgc    3834

aagtccactt tgatgcaggt aaagtttaat ggtaacaaaa gtcctcataa catttggatg    3894

caaatcttaa cattaattcc atgtctcagc caacattctc cattattaag cagcctgtga    3954

tgtgattaca gtgaaccact tttgaaaagg agcctgtgta taacagatag tttcactata    4014

ctatataacc gtcagatgca ggcttgtaaa ttaatttgtt ggtgacaatg tttcagtaca    4074

ttttcaaatt gattcattgg tatagtactc aaatttgagt gggcttggtg aacacaatga    4134

agacaagctg agaagtgcaa aaaaaaaaaa aaaaaaaaaa                          4174

<210>  6
<211>  642
<212>  PRT
<213>  Homo sapiens

<400>  6

Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Gln Gln Gln Glu Thr Ser Pro Arg Gln Gln Gln Gln Gln Gln Gly Glu
                85                  90                  95

Asp Gly Ser Pro Gln Ala His Arg Arg Gly Pro Thr Gly Tyr Leu Val
            100                 105                 110

Leu Asp Glu Glu Gln Gln Pro Ser Gln Pro Gln Ser Ala Leu Glu Cys
        115                 120                 125

His Pro Glu Arg Gly Cys Val Pro Glu Pro Gly Ala Ala Val Ala Ala
    130                 135                 140

Ser Lys Gly Leu Pro Gln Gln Leu Pro Ala Pro Pro Asp Glu Asp Asp
145                 150                 155                 160

Ser Ala Ala Pro Ser Thr Leu Ser Leu Leu Gly Pro Thr Phe Pro Gly
                165                 170                 175

Leu Ser Ser Cys Ser Ala Asp Leu Lys Asp Ile Leu Ser Glu Ala Ser
            180                 185                 190

Thr Met Gln Leu Leu Gln Gln Gln Gln Gln Glu Ala Val Ser Glu Gly
        195                 200                 205

Ser Ser Ser Gly Arg Ala Arg Glu Ala Ser Gly Ala Pro Thr Ser Ser
    210                 215                 220

Lys Asp Asn Tyr Leu Gly Gly Thr Ser Thr Ile Ser Asp Asn Ala Lys

225                 230                 235                 240

Glu Leu Cys Lys Ala Val Ser Val Ser Met Gly Leu Gly Val Glu Ala
                245                 250                 255

Leu Glu His Leu Ser Pro Gly Glu Gln Leu Arg Gly Asp Cys Met Tyr
            260                 265                 270

Ala Pro Leu Leu Gly Val Pro Pro Ala Val Arg Pro Thr Pro Cys Ala
        275                 280                 285

Pro Leu Ala Glu Cys Lys Gly Ser Leu Leu Asp Asp Ser Ala Gly Lys
    290                 295                 300

Ser Thr Glu Asp Thr Ala Glu Tyr Ser Pro Phe Lys Gly Gly Tyr Thr
305                 310                 315                 320

Lys Gly Leu Glu Gly Glu Ser Leu Gly Cys Ser Gly Ser Ala Ala Ala
                325                 330                 335

Gly Ser Ser Gly Thr Leu Glu Leu Pro Ser Thr Leu Ser Leu Tyr Lys
            340                 345                 350

Ser Gly Ala Leu Asp Glu Ala Ala Ala Tyr Gln Ser Arg Asp Tyr Tyr
        355                 360                 365

Asn Phe Pro Leu Ala Leu Ala Gly Pro Pro Pro Pro Pro Pro Pro Pro
    370                 375                 380

His Pro His Ala Arg Ile Lys Leu Glu Asn Pro Leu Asp Tyr Gly Ser
385                 390                 395                 400

Ala Trp Ala Ala Ala Ala Ala Gln Cys Arg Tyr Gly Asp Leu Ala Ser
                405                 410                 415

Leu His Gly Ala Gly Ala Ala Gly Pro Gly Ser Gly Ser Pro Ser Ala
            420                 425                 430

Ala Ala Ser Ser Ser Trp His Thr Leu Phe Thr Ala Glu Glu Gly Gln
        435                 440                 445

Leu Tyr Gly Pro Cys Gly Gly Gly Gly Gly Gly Gly Gly Gly Gly Gly
    450                 455                 460

Gly Gly Gly Gly Gly Gly Gly Gly Glu Ala Gly Ala Val Ala Pro Tyr
465                 470                 475                 480

Gly Tyr Thr Arg Pro Pro Gln Gly Leu Ala Gly Gln Glu Ser Asp Phe
                485                 490                 495

Thr Ala Pro Asp Val Trp Tyr Pro Gly Gly Met Val Ser Arg Val Pro
            500                 505                 510

Tyr Pro Ser Pro Thr Cys Val Lys Ser Glu Met Gly Pro Trp Met Asp
        515                 520                 525

Ser Tyr Ser Gly Pro Tyr Gly Asp Met Arg Leu Glu Thr Ala Arg Asp
    530                 535                 540

His Val Leu Pro Ile Asp Tyr Tyr Phe Pro Pro Gln Lys Thr Cys Leu
545                 550                 555                 560

Ile Cys Gly Asp Glu Ala Ser Gly Cys His Tyr Gly Ala Leu Thr Cys
                565                 570                 575

Gly Ser Cys Lys Val Phe Phe Lys Arg Ala Ala Glu Gly Lys Gln Lys
            580                 585                 590

Tyr Leu Cys Ala Ser Arg Asn Asp Cys Thr Ile Asp Lys Phe Arg Arg
        595                 600                 605

Lys Asn Cys Pro Ser Cys Arg Leu Arg Lys Cys Tyr Glu Ala Gly Met
    610                 615                 620

Thr Leu Gly Gly Ser Ser Leu Leu Pro Ala Thr Ser Lys Lys Met Cys
625                 630                 635                 640

Leu Ser

<210>  7
<211>  4026
<212>  DNA
<213>  Homo sapiens

<220>
<221>  CDS
<222>  (1116)..(2651)

<400>  7
cgagatcccg gggagccagc ttgctgggag agcgggacgg tccggagcaa gcccagaggc      60

agaggaggcg acagagggaa aaagggccga gctagccgct ccagtgctgt acaggagccg     120

aagggacgca ccacgccagc cccagcccgg ctccagcgac agccaacgcc tcttgcagcg     180

cggcggcttc gaagccgccg cccggagctg ccctttcctc ttcggtgaag tttttaaaag     240

ctgctaaaga ctcggaggaa gcaaggaaag tgcctggtag gactgacggc tgcctttgtc     300

ctcctcctct ccaccccgcc tccccccacc ctgccttccc cccctccccc gtcttctctc     360

ccgcagctgc ctcagtcggc tactctcagc caacccccct caccaccctt ctccccaccc 420

gcccccccgc ccccgtcggc ccagcgctgc cagcccgagt ttgcagagag gtaactccct 480

ttggctgcga gcgggcgagc tagctgcaca ttgcaaagaa ggctcttagg agccaggcga 540

ctggggagcg gcttcagcac tgcagccacg acccgcctgg ttaggctgca cgcggagaga 600

accctctgtt ttcccccact ctctctccac ctcctcctgc cttccccacc ccgagtgcgg 660

agccagagat caaaagatga aaaggcagtc aggtcttcag tagccaaaaa acaaaacaaa 720

caaaaacaaa aaagccgaaa taaaagaaaa agataataac tcagttctta tttgcaccta 780

cttcagtgga cactgaattt ggaaggtgga ggattttgtt tttttctttt aagatctggg 840

catcttttga atctaccctt caagtattaa gagacagact gtgagcctag cagggcagat 900

cttgtccacc gtgtgtcttc ttctgcacga gactttgagg ctgtcagagc gctttttgcg 960

tggttgctcc cgcaagtttc cttctctgga gcttcccgca ggtgggcagc tagctgcagc 1020

gactaccgca tcatcacagc ctgttgaact cttctgagca agagaagggg aggcggggta 1080

agggaagtag gtggaagatt cagccaagct caagg atg gaa gtg cag tta ggg 1133
Met Glu Val Gln Leu Gly
1               5

ctg gga agg gtc tac cct cgg ccg ccg tcc aag acc tac cga gga gct 1181
Leu Gly Arg Val Tyr Pro Arg Pro Pro Ser Lys Thr Tyr Arg Gly Ala
            10                  15                  20

ttc cag aat ctg ttc cag agc gtg cgc gaa gtg atc cag aac ccg ggc 1229
Phe Gln Asn Leu Phe Gln Ser Val Arg Glu Val Ile Gln Asn Pro Gly
        25                  30                  35

ccc agg cac cca gag gcc gcg agc gca gca cct ccc ggc gcc agt ttg 1277
Pro Arg His Pro Glu Ala Ala Ser Ala Ala Pro Pro Gly Ala Ser Leu
    40                  45                  50

ctg ctg ctg cag cag cag cag cag cag cag cag cag cag cag cag cag 1325
Leu Leu Leu Gln Gln Gln Gln Gln Gln Gln Gln Gln Gln Gln Gln Gln
55                  60                  65                  70

cag cag cag cag cag cag cag cag cag caa gag act agc ccc agg cag 1373
Gln Gln Gln Gln Gln Gln Gln Gln Gln Gln Glu Thr Ser Pro Arg Gln
                75                  80                  85

cag cag cag cag cag ggt gag gat ggt tct ccc caa gcc cat cgt aga 1421
Gln Gln Gln Gln Gln Gly Glu Asp Gly Ser Pro Gln Ala His Arg Arg
            90                  95                  100

ggc ccc aca ggc tac ctg gtc ctg gat gag gaa cag caa cct tca cag 1469
Gly Pro Thr Gly Tyr Leu Val Leu Asp Glu Glu Gln Gln Pro Ser Gln
        105                 110                 115

ccg cag tcg gcc ctg gag tgc cac ccc gag aga ggt tgc gtc cca gag 1517
Pro Gln Ser Ala Leu Glu Cys His Pro Glu Arg Gly Cys Val Pro Glu
    120                 125                 130

cct gga gcc gcc gtg gcc gcc agc aag ggg ctg ccg cag cag ctg cca 1565
Pro Gly Ala Ala Val Ala Ala Ser Lys Gly Leu Pro Gln Gln Leu Pro
135                 140                 145                 150

gca cct ccg gac gag gat gac tca gct gcc cca tcc acg ttg tcc ctg 1613

Ala Pro Pro Asp Glu Asp Asp Ser Ala Ala Pro Ser Thr Leu Ser Leu
                155                 160                 165

ctg ggc ccc act ttc ccc ggc tta agc agc tgc tcc gct gac ctt aaa 1661
Leu Gly Pro Thr Phe Pro Gly Leu Ser Ser Cys Ser Ala Asp Leu Lys
            170                 175                 180

gac atc ctg agc gag gcc agc acc atg caa ctc ctt cag caa cag cag 1709
Asp Ile Leu Ser Glu Ala Ser Thr Met Gln Leu Leu Gln Gln Gln Gln
        185                 190                 195

cag gaa gca gta tcc gaa ggc agc agc agc ggg aga gcg agg gag gcc 1757
Gln Glu Ala Val Ser Glu Gly Ser Ser Ser Gly Arg Ala Arg Glu Ala
    200                 205                 210

tcg ggg gct ccc act tcc tcc aag gac aat tac tta ggg ggc act tcg 1805
Ser Gly Ala Pro Thr Ser Ser Lys Asp Asn Tyr Leu Gly Gly Thr Ser
215                 220                 225                 230

acc att tct gac aac gcc aag gag ttg tgt aag gca gtg tcg gtg tcc 1853
Thr Ile Ser Asp Asn Ala Lys Glu Leu Cys Lys Ala Val Ser Val Ser
                235                 240                 245

atg ggc ctg ggt gtg gag gcg ttg gag cat ctg agt cca ggg gaa cag 1901
Met Gly Leu Gly Val Glu Ala Leu Glu His Leu Ser Pro Gly Glu Gln
            250                 255                 260

ctt cgg ggg gat tgc atg tac gcc cca ctt ttg gga gtt cca ccc gct 1949
Leu Arg Gly Asp Cys Met Tyr Ala Pro Leu Leu Gly Val Pro Pro Ala
        265                 270                 275

gtg cgt ccc act cct tgt gcc cca ttg gcc gaa tgc aaa ggt tct ctg 1997
Val Arg Pro Thr Pro Cys Ala Pro Leu Ala Glu Cys Lys Gly Ser Leu
    280                 285                 290

cta gac gac agc gca ggc aag agc act gaa gat act gct gag tat tcc 2045
Leu Asp Asp Ser Ala Gly Lys Ser Thr Glu Asp Thr Ala Glu Tyr Ser
295                 300                 305                 310

cct ttc aag gga ggt tac acc aaa ggg cta gaa ggc gag agc cta ggc 2093
Pro Phe Lys Gly Gly Tyr Thr Lys Gly Leu Glu Gly Glu Ser Leu Gly
                315                 320                 325

tgc tct ggc agc gct gca gca ggg agc tcc ggg aca ctt gaa ctg ccg 2141
Cys Ser Gly Ser Ala Ala Ala Gly Ser Ser Gly Thr Leu Glu Leu Pro
            330                 335                 340

tct acc ctg tct ctc tac aag tcc gga gca ctg gac gag gca gct gcg 2189
Ser Thr Leu Ser Leu Tyr Lys Ser Gly Ala Leu Asp Glu Ala Ala Ala
        345                 350                 355

tac cag agt cgc gac ttc acc gca cct gat gtg tgg tac cct ggc ggc 2237
Tyr Gln Ser Arg Asp Phe Thr Ala Pro Asp Val Trp Tyr Pro Gly Gly
    360                 365                 370

atg gtg agc aga gtg ccc tat ccc agt ccc act tgt gtc aaa agc gaa 2285
Met Val Ser Arg Val Pro Tyr Pro Ser Pro Thr Cys Val Lys Ser Glu
375                 380                 385                 390

atg ggc ccc tgg atg gat agc tac tcc gga cct tac ggg gac atg cgt 2333
Met Gly Pro Trp Met Asp Ser Tyr Ser Gly Pro Tyr Gly Asp Met Arg
                395                 400                 405

ttg gag act gcc agg gac cat gtt ttg ccc att gac tat tac ttt cca 2381
Leu Glu Thr Ala Arg Asp His Val Leu Pro Ile Asp Tyr Tyr Phe Pro

            410                 415                 420

ccc cag aag acc tgc ctg atc tgt gga gat gaa gct tct ggg tgt cac 2429
Pro Gln Lys Thr Cys Leu Ile Cys Gly Asp Glu Ala Ser Gly Cys His
        425                 430                 435

tat gga gct ctc aca tgt gga agc tgc aag gtc ttc ttc aaa aga gcc 2477
Tyr Gly Ala Leu Thr Cys Gly Ser Cys Lys Val Phe Phe Lys Arg Ala
    440                 445                 450

gct gaa ggg aaa cag aag tac ctg tgc gcc agc aga aat gat tgc act 2525
Ala Glu Gly Lys Gln Lys Tyr Leu Cys Ala Ser Arg Asn Asp Cys Thr
455                 460                 465                 470

att gat aaa ttc cga agg aaa aat tgt cca tct tgt cgt ctt cgg aaa 2573
Ile Asp Lys Phe Arg Arg Lys Asn Cys Pro Ser Cys Arg Leu Arg Lys
                475                 480                 485

tgt tat gaa gca ggg atg act ctg gga gaa aaa ttc cgg gtt ggc aat 2621
Cys Tyr Glu Ala Gly Met Thr Leu Gly Glu Lys Phe Arg Val Gly Asn
            490                 495                 500

tgc aag cat ctc aaa atg acc aga ccc tga agaaaggctg acttgcctca 2671
Cys Lys His Leu Lys Met Thr Arg Pro
        505                 510

ttcaaaatga gggctctaga gggctctagt ggatagtctg gagaaacctg gcgtctgagg 2731

cttaggagct taggtttttg ctcctcaaca cagactttga cgttggggtt gggggctact 2791

ctcttgattg ctgactccct ccagcgggac caatagtgtt ttcctacctc acagggatgt 2851

tgtgaggacg ggctgtagaa gtaatagtgg ttaccattca tgtagttgtg agtatcatga 2911

ttattgtttc ctgtaatgtg gcttggcatt ggcaaagtgc tttttgattg ttcttgatca 2971

catatgatgg gggccaggca ctgactcagg cggatgcagt gaagctctgg ctcagtcgct 3031

tgcttttcgt ggtgtgctgc caggaagaaa ctttgctgat gggactcaag gtgtcacctt 3091

ggacaagaag caactgtgtc tgtctgaggt tcctgtggcc atctttattt gtgtattagg 3151

caattcgtat ttccccctta ggttctagcc ttctggatcc cagccagtga cctagatctt 3211

agcctcaggc cctgtcactg agctgaaggt agtagctgat ccacagaagt tcagtaaaca 3271

aggaccagat ttctgcttct ccaggagaag aagccagcca acccctctct tcaaacacac 3331

tgagagacta cagtccgact ttccctctta catctagcct tactgtagcc acactccttg 3391

attgctctct cacatcacat gcttctcttc atcagttgta agcctctcat tcttctccca 3451

agccagactc aaatattgta ttgatgtcaa agaagaatca cttagagttt ggaatatctt 3511

gttctctctc tgctccatag cttccatatt gacaccagtt tctttctagt ggagaagtgg 3571

agtctgtgaa gccagggaaa cacacatgtg agagtcagaa ggactctccc tgacttgcct 3631

ggggcctgtc tttcccacct tctccagtct gtctaaacac acacacacac acacacacac 3691

acacacacac gctctctctc tctctccccc cccaacacac acacactctc tctctctctc 3751

acacacacac acatacacac acacttcttt ctctttcccc tgactcagca acattctgga 3811

gaaaagccaa ggaaggactt caggagggga gtttccccct tctcagggca gaattttaat 3871

ctccagacca acaagaagtt ccctaatgtg gattgaaagg ctaatgaggt ttatttttaa 3931

ctactttcta tttgtttgaa tgttgcatat ttctactagt gaaattttcc cttaataaag 3991

ccattaatac accaaaaaaa aaaaaaaaaa aaaaa 4026

<210> 8
<211> 511
<212> PRT
<213> Homo sapiens

<400> 8

Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Glu Thr Ser Pro Arg Gln Gln Gln Gln Gln Gln Gly Glu Asp Gly Ser
                85                  90                  95

Pro Gln Ala His Arg Arg Gly Pro Thr Gly Tyr Leu Val Leu Asp Glu
            100                 105                 110

Glu Gln Gln Pro Ser Gln Pro Gln Ser Ala Leu Glu Cys His Pro Glu
        115                 120                 125

Arg Gly Cys Val Pro Glu Pro Gly Ala Ala Val Ala Ala Ser Lys Gly
    130                 135                 140

Leu Pro Gln Gln Leu Pro Ala Pro Pro Asp Glu Asp Asp Ser Ala Ala
145                 150                 155                 160

Pro Ser Thr Leu Ser Leu Leu Gly Pro Thr Phe Pro Gly Leu Ser Ser
                165                 170                 175

Cys Ser Ala Asp Leu Lys Asp Ile Leu Ser Glu Ala Ser Thr Met Gln
            180                 185                 190

Leu Leu Gln Gln Gln Gln Gln Glu Ala Val Ser Glu Gly Ser Ser Ser
        195                 200                 205

Gly Arg Ala Arg Glu Ala Ser Gly Ala Pro Thr Ser Ser Lys Asp Asn
    210                 215                 220

Tyr Leu Gly Gly Thr Ser Thr Ile Ser Asp Asn Ala Lys Glu Leu Cys
225                 230                 235                 240

Lys Ala Val Ser Val Ser Met Gly Leu Gly Val Glu Ala Leu Glu His
                245                 250                 255

Leu Ser Pro Gly Glu Gln Leu Arg Gly Asp Cys Met Tyr Ala Pro Leu
            260                 265                 270

Leu Gly Val Pro Pro Ala Val Arg Pro Thr Pro Cys Ala Pro Leu Ala
        275                 280                 285

Glu Cys Lys Gly Ser Leu Leu Asp Asp Ser Ala Gly Lys Ser Thr Glu
    290                 295                 300

Asp Thr Ala Glu Tyr Ser Pro Phe Lys Gly Gly Tyr Thr Lys Gly Leu
305                 310                 315                 320

Glu Gly Glu Ser Leu Gly Cys Ser Gly Ser Ala Ala Ala Gly Ser Ser
                325                 330                 335

Gly Thr Leu Glu Leu Pro Ser Thr Leu Ser Leu Tyr Lys Ser Gly Ala
            340                 345                 350

Leu Asp Glu Ala Ala Ala Tyr Gln Ser Arg Asp Phe Thr Ala Pro Asp
        355                 360                 365

Val Trp Tyr Pro Gly Gly Met Val Ser Arg Val Pro Tyr Pro Ser Pro
    370                 375                 380

Thr Cys Val Lys Ser Glu Met Gly Pro Trp Met Asp Ser Tyr Ser Gly
385                 390                 395                 400

Pro Tyr Gly Asp Met Arg Leu Glu Thr Ala Arg Asp His Val Leu Pro
                405                 410                 415

Ile Asp Tyr Tyr Phe Pro Pro Gln Lys Thr Cys Leu Ile Cys Gly Asp
            420                 425                 430

Glu Ala Ser Gly Cys His Tyr Gly Ala Leu Thr Cys Gly Ser Cys Lys
        435                 440                 445

Val Phe Phe Lys Arg Ala Ala Glu Gly Lys Gln Lys Tyr Leu Cys Ala
    450                 455                 460

Ser Arg Asn Asp Cys Thr Ile Asp Lys Phe Arg Arg Lys Asn Cys Pro
465                 470                 475                 480

Ser Cys Arg Leu Arg Lys Cys Tyr Glu Ala Gly Met Thr Leu Gly Glu
                485                 490                 495

Lys Phe Arg Val Gly Asn Cys Lys His Leu Lys Met Thr Arg Pro
            500                 505                 510

<210>  9
<211>  4167
<212>  DNA
<213>  Homo sapiens

<220>
<221>  CDS
<222>  (1116)..(2792)

<400>  9
cgagatcccg gggagccagc ttgctgggag agcgggacgg tccggagcaa gcccagaggc      60

agaggaggcg acagagggaa aaagggccga gctagccgct ccagtgctgt acaggagccg     120

aagggacgca ccacgccagc cccagcccgg ctccagcgac agccaacgcc tcttgcagcg     180

cggcggcttc gaagccgccg cccggagctg ccctttcctc ttcggtgaag tttttaaaag     240

ctgctaaaga ctcggaggaa gcaaggaaag tgcctggtag gactgacggc tgcctttgtc     300

ctcctcctct ccaccccgcc tccccccacc ctgccttccc cccctccccc gtcttctctc     360

ccgcagctgc ctcagtcggc tactctcagc caacccccct caccaccctt ctccccaccc     420

gcccccccgc ccccgtcggc ccagcgctgc cagcccgagt ttgcagagag gtaactccct     480

ttggctgcga gcgggcgagc tagctgcaca ttgcaaagaa ggctcttagg agccaggcga     540

ctggggagcg gcttcagcac tgcagccacg acccgcctgg ttaggctgca cgcggagaga     600

accctctgtt ttcccccact ctctctccac ctcctcctgc cttccccacc ccgagtgcgg     660

agccagagat caaaagatga aaaggcagtc aggtcttcag tagccaaaaa acaaaacaaa     720

caaaaacaaa aaagccgaaa taaaagaaaa agataataac tcagttctta tttgcaccta     780

cttcagtgga cactgaattt ggaaggtgga ggattttgtt tttttctttt aagatctggg     840

catcttttga atctaccctt caagtattaa gagacagact gtgagcctag cagggcagat     900

cttgtccacc gtgtgtcttc ttctgcacga gactttgagg ctgtcagagc gctttttgcg     960

tggttgctcc cgcaagtttc cttctctgga gcttcccgca ggtgggcagc tagctgcagc    1020

gactaccgca tcatcacagc ctgttgaact cttctgagca agagaagggg aggcggggta    1080

agggaagtag gtggaagatt cagccaagct caagg atg gaa gtg cag tta ggg       1133
                                       Met Glu Val Gln Leu Gly
                                       1               5

ctg gga agg gtc tac cct cgg ccg ccg tcc aag acc tac cga gga gct      1181

Leu Gly Arg Val Tyr Pro Arg Pro Pro Ser Lys Thr Tyr Arg Gly Ala
            10                  15                  20

ttc cag aat ctg ttc cag agc gtg cgc gaa gtg atc cag aac ccg ggc      1229
Phe Gln Asn Leu Phe Gln Ser Val Arg Glu Val Ile Gln Asn Pro Gly
        25                  30                  35

ccc agg cac cca gag gcc gcg agc gca gca cct ccc ggc gcc agt ttg      1277
Pro Arg His Pro Glu Ala Ala Ser Ala Ala Pro Pro Gly Ala Ser Leu
    40                  45                  50

ctg ctg ctg cag cag cag cag cag cag cag cag cag cag cag cag cag      1325
Leu Leu Leu Gln Gln Gln Gln Gln Gln Gln Gln Gln Gln Gln Gln Gln
55                  60                  65                  70

cag cag cag cag cag cag cag cag cag caa gag act agc ccc agg cag      1373
Gln Gln Gln Gln Gln Gln Gln Gln Gln Gln Glu Thr Ser Pro Arg Gln
                75                  80                  85

cag cag cag cag cag ggt gag gat ggt tct ccc caa gcc cat cgt aga      1421
Gln Gln Gln Gln Gln Gly Glu Asp Gly Ser Pro Gln Ala His Arg Arg
            90                  95                  100

ggc ccc aca ggc tac ctg gtc ctg gat gag gaa cag caa cct tca cag      1469
Gly Pro Thr Gly Tyr Leu Val Leu Asp Glu Glu Gln Gln Pro Ser Gln
        105                 110                 115

ccg cag tcg gcc ctg gag tgc cac ccc gag aga ggt tgc gtc cca gag      1517
Pro Gln Ser Ala Leu Glu Cys His Pro Glu Arg Gly Cys Val Pro Glu
    120                 125                 130

cct gga gcc gcc gtg gcc gcc agc aag ggg ctg ccg cag cag ctg cca      1565
Pro Gly Ala Ala Val Ala Ala Ser Lys Gly Leu Pro Gln Gln Leu Pro
135                 140                 145                 150

gca cct ccg gac gag gat gac tca gct gcc cca tcc acg ttg tcc ctg      1613
Ala Pro Pro Asp Glu Asp Asp Ser Ala Ala Pro Ser Thr Leu Ser Leu
                155                 160                 165

ctg ggc ccc act ttc ccc ggc tta agc agc tgc tcc gct gac ctt aaa      1661
Leu Gly Pro Thr Phe Pro Gly Leu Ser Ser Cys Ser Ala Asp Leu Lys
            170                 175                 180

gac atc ctg agc gag gcc agc acc atg caa ctc ctt cag caa cag cag      1709
Asp Ile Leu Ser Glu Ala Ser Thr Met Gln Leu Leu Gln Gln Gln Gln
        185                 190                 195

cag gaa gca gta tcc gaa ggc agc agc agc ggg aga gcg agg gag gcc      1757
Gln Glu Ala Val Ser Glu Gly Ser Ser Ser Gly Arg Ala Arg Glu Ala
    200                 205                 210

tcg ggg gct ccc act tcc tcc aag gac aat tac tta ggg ggc act tcg      1805
Ser Gly Ala Pro Thr Ser Ser Lys Asp Asn Tyr Leu Gly Gly Thr Ser
215                 220                 225                 230

acc att tct gac aac gcc aag gag ttg tgt aag gca gtg tcg gtg tcc      1853
Thr Ile Ser Asp Asn Ala Lys Glu Leu Cys Lys Ala Val Ser Val Ser
                235                 240                 245

atg ggc ctg ggt gtg gag gcg ttg gag cat ctg agt cca ggg gaa cag      1901
Met Gly Leu Gly Val Glu Ala Leu Glu His Leu Ser Pro Gly Glu Gln
            250                 255                 260

ctt cgg ggg gat tgc atg tac gcc cca ctt ttg gga gtt cca ccc gct      1949
Leu Arg Gly Asp Cys Met Tyr Ala Pro Leu Leu Gly Val Pro Pro Ala

        265                 270                 275

gtg cgt ccc act cct tgt gcc cca ttg gcc gaa tgc aaa ggt tct ctg 1997
Val Arg Pro Thr Pro Cys Ala Pro Leu Ala Glu Cys Lys Gly Ser Leu
    280                 285                 290

cta gac gac agc gca ggc aag agc act gaa gat act gct gag tat tcc 2045
Leu Asp Asp Ser Ala Gly Lys Ser Thr Glu Asp Thr Ala Glu Tyr Ser
295                 300                 305                 310

cct ttc aag gga ggt tac acc aaa ggg cta gaa ggc gag agc cta ggc 2093
Pro Phe Lys Gly Gly Tyr Thr Lys Gly Leu Glu Gly Glu Ser Leu Gly
                315                 320                 325

tgc tct ggc agc gct gca gca ggg agc tcc ggg aca ctt gaa ctg ccg 2141
Cys Ser Gly Ser Ala Ala Ala Gly Ser Ser Gly Thr Leu Glu Leu Pro
            330                 335                 340

tct acc ctg tct ctc tac aag tcc gga gca ctg gac gag gca gct gcg 2189
Ser Thr Leu Ser Leu Tyr Lys Ser Gly Ala Leu Asp Glu Ala Ala Ala
        345                 350                 355

tac cag agt cgc gac tac tac aac ttt cca ctg gct ctg gcc gga ccg 2237
Tyr Gln Ser Arg Asp Tyr Tyr Asn Phe Pro Leu Ala Leu Ala Gly Pro
    360                 365                 370

ccg ccc cct ccg ccg cct ccc cat ccc cac gct cgc atc aag ctg gag 2285
Pro Pro Pro Pro Pro Pro Pro His Pro His Ala Arg Ile Lys Leu Glu
375                 380                 385                 390

aac ccg ctg gac tac ggc tac act cgg ccc cct cag ggg ctg gcg ggc 2333
Asn Pro Leu Asp Tyr Gly Tyr Thr Arg Pro Pro Gln Gly Leu Ala Gly
                395                 400                 405

cag gaa agc gac ttc acc gca cct gat gtg tgg tac cct ggc ggc atg 2381
Gln Glu Ser Asp Phe Thr Ala Pro Asp Val Trp Tyr Pro Gly Gly Met
            410                 415                 420

gtg agc aga gtg ccc tat ccc agt ccc act tgt gtc aaa agc gaa atg 2429
Val Ser Arg Val Pro Tyr Pro Ser Pro Thr Cys Val Lys Ser Glu Met
        425                 430                 435

ggc ccc tgg atg gat agc tac tcc gga cct tac ggg gac atg cgt ttg 2477
Gly Pro Trp Met Asp Ser Tyr Ser Gly Pro Tyr Gly Asp Met Arg Leu
    440                 445                 450

gag act gcc agg gac cat gtt ttg ccc att gac tat tac ttt cca ccc 2525
Glu Thr Ala Arg Asp His Val Leu Pro Ile Asp Tyr Tyr Phe Pro Pro
455                 460                 465                 470

cag aag acc tgc ctg atc tgt gga gat gaa gct tct ggg tgt cac tat 2573
Gln Lys Thr Cys Leu Ile Cys Gly Asp Glu Ala Ser Gly Cys His Tyr
                475                 480                 485

gga gct ctc aca tgt gga agc tgc aag gtc ttc ttc aaa aga gcc gct 2621
Gly Ala Leu Thr Cys Gly Ser Cys Lys Val Phe Phe Lys Arg Ala Ala
            490                 495                 500

gaa ggg aaa cag aag tac ctg tgc gcc agc aga aat gat tgc act att 2669
Glu Gly Lys Gln Lys Tyr Leu Cys Ala Ser Arg Asn Asp Cys Thr Ile
        505                 510                 515

gat aaa ttc cga agg aaa aat tgt cca tct tgt cgt ctt cgg aaa tgt 2717
Asp Lys Phe Arg Arg Lys Asn Cys Pro Ser Cys Arg Leu Arg Lys Cys
    520                 525                 530

tat gaa gca ggg atg act ctg gga gaa aaa ttc cgg gtt ggc aat tgc      2765
Tyr Glu Ala Gly Met Thr Leu Gly Glu Lys Phe Arg Val Gly Asn Cys
535                 540                 545                 550

aag cat ctc aaa atg acc aga ccc tga agaaaggctg acttgcctca           2812
Lys His Leu Lys Met Thr Arg Pro
                555

ttcaaaatga gggctctaga gggctctagt ggatagtctg gagaaacctg gcgtctgagg    2872

cttaggagct taggtttttg ctcctcaaca cagactttga cgttggggtt gggggctact    2932

ctcttgattg ctgactccct ccagcgggac caatagtgtt ttcctacctc acagggatgt    2992

tgtgaggacg ggctgtagaa gtaatagtgg ttaccattca tgtagttgtg agtatcatga    3052

ttattgtttc ctgtaatgtg gcttggcatt ggcaaagtgc tttttgattg ttcttgatca    3112

catatgatgg gggccaggca ctgactcagg cggatgcagt gaagctctgg ctcagtcgct    3172

tgcttttcgt ggtgtgctgc caggaagaaa ctttgctgat gggactcaag gtgtcacctt    3232

ggacaagaag caactgtgtc tgtctgaggt tcctgtggcc atctttattt gtgtattagg    3292

caattcgtat ttccccctta ggttctagcc ttctggatcc cagccagtga cctagatctt    3352

agcctcaggc cctgtcactg agctgaaggt agtagctgat ccacagaagt tcagtaaaca    3412

aggaccagat ttctgcttct ccaggagaag aagccagcca acccctctct tcaaacacac    3472

tgagagacta cagtccgact ttccctctta catctagcct tactgtagcc acactccttg    3532

attgctctct cacatcacat gcttctcttc atcagttgta agcctctcat tcttctccca    3592

agccagactc aaatattgta ttgatgtcaa agaagaatca cttagagttt ggaatatctt    3652

gttctctctc tgctccatag cttccatatt gacaccagtt tctttctagt ggagaagtgg    3712

agtctgtgaa gccagggaaa cacacatgtg agagtcagaa ggactctccc tgacttgcct    3772

ggggcctgtc tttcccacct tctccagtct gtctaaacac acacacacac acacacacac    3832

acacacacac gctctctctc tctctccccc cccaacacac acacactctc tctctctctc    3892

acacacacac acatacacac acacttcttt ctctttcccc tgactcagca acattctgga    3952

gaaaagccaa ggaaggactt caggagggga gtttccccct tctcagggca gaattttaat    4012

ctccagacca acaagaagtt ccctaatgtg gattgaaagg ctaatgaggt ttatttttaa    4072

ctactttcta tttgtttgaa tgttgcatat ttctactagt gaaattttcc cttaataaag    4132

ccattaatac accaaaaaaa aaaaaaaaaa aaaaa                               4167


<210>  10
<211>  558
<212>  PRT
<213>  Homo sapiens

<400>  10

Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Glu Thr Ser Pro Arg Gln Gln Gln Gln Gln Gln Gly Glu Asp Gly Ser
                85                  90                  95

Pro Gln Ala His Arg Arg Gly Pro Thr Gly Tyr Leu Val Leu Asp Glu
            100                 105                 110

Glu Gln Gln Pro Ser Gln Pro Gln Ser Ala Leu Glu Cys His Pro Glu
        115                 120                 125

Arg Gly Cys Val Pro Glu Pro Gly Ala Ala Val Ala Ala Ser Lys Gly
    130                 135                 140

Leu Pro Gln Gln Leu Pro Ala Pro Pro Asp Glu Asp Asp Ser Ala Ala
145                 150                 155                 160

Pro Ser Thr Leu Ser Leu Leu Gly Pro Thr Phe Pro Gly Leu Ser Ser
                165                 170                 175

Cys Ser Ala Asp Leu Lys Asp Ile Leu Ser Glu Ala Ser Thr Met Gln
            180                 185                 190

Leu Leu Gln Gln Gln Gln Gln Glu Ala Val Ser Glu Gly Ser Ser Ser
        195                 200                 205

Gly Arg Ala Arg Glu Ala Ser Gly Ala Pro Thr Ser Ser Lys Asp Asn
    210                 215                 220

Tyr Leu Gly Gly Thr Ser Thr Ile Ser Asp Asn Ala Lys Glu Leu Cys
225                 230                 235                 240

Lys Ala Val Ser Val Ser Met Gly Leu Gly Val Glu Ala Leu Glu His
                245                 250                 255

Leu Ser Pro Gly Glu Gln Leu Arg Gly Asp Cys Met Tyr Ala Pro Leu
            260                 265                 270

Leu Gly Val Pro Pro Ala Val Arg Pro Thr Pro Cys Ala Pro Leu Ala
        275                 280                 285

Glu Cys Lys Gly Ser Leu Leu Asp Asp Ser Ala Gly Lys Ser Thr Glu
    290                 295                 300

Asp Thr Ala Glu Tyr Ser Pro Phe Lys Gly Gly Tyr Thr Lys Gly Leu
305                 310                 315                 320

Glu Gly Glu Ser Leu Gly Cys Ser Gly Ser Ala Ala Ala Gly Ser Ser
                325                 330                 335

Gly Thr Leu Glu Leu Pro Ser Thr Leu Ser Leu Tyr Lys Ser Gly Ala
            340                 345                 350

Leu Asp Glu Ala Ala Ala Tyr Gln Ser Arg Asp Tyr Tyr Asn Phe Pro
        355                 360                 365

Leu Ala Leu Ala Gly Pro Pro Pro Pro Pro Pro Pro Pro His Pro His
    370                 375                 380

Ala Arg Ile Lys Leu Glu Asn Pro Leu Asp Tyr Gly Tyr Thr Arg Pro
385                 390                 395                 400

Pro Gln Gly Leu Ala Gly Gln Glu Ser Asp Phe Thr Ala Pro Asp Val
                405                 410                 415

Trp Tyr Pro Gly Gly Met Val Ser Arg Val Pro Tyr Pro Ser Pro Thr
            420                 425                 430

Cys Val Lys Ser Glu Met Gly Pro Trp Met Asp Ser Tyr Ser Gly Pro
        435                 440                 445

Tyr Gly Asp Met Arg Leu Glu Thr Ala Arg Asp His Val Leu Pro Ile
    450                 455                 460

Asp Tyr Tyr Phe Pro Pro Gln Lys Thr Cys Leu Ile Cys Gly Asp Glu
465                 470                 475                 480

Ala Ser Gly Cys His Tyr Gly Ala Leu Thr Cys Gly Ser Cys Lys Val
                485                 490                 495

Phe Phe Lys Arg Ala Ala Glu Gly Lys Gln Lys Tyr Leu Cys Ala Ser
            500                 505                 510

Arg Asn Asp Cys Thr Ile Asp Lys Phe Arg Arg Lys Asn Cys Pro Ser
        515                 520                 525

Cys Arg Leu Arg Lys Cys Tyr Glu Ala Gly Met Thr Leu Gly Glu Lys
    530                 535                 540

Phe Arg Val Gly Asn Cys Lys His Leu Lys Met Thr Arg Pro
545                 550                 555

<210>  11
<211>  4287
<212>  DNA
<213>  Homo sapiens

<220>
<221>  CDS
<222>  (1116)..(2912)

<400>  11
cgagatcccg gggagccagc ttgctgggag agcgggacgg tccggagcaa gcccagaggc      60

agaggaggcg acagagggaa aaagggccga gctagccgct ccagtgctgt acaggagccg     120

aagggacgca ccacgccagc cccagcccgg ctccagcgac agccaacgcc tcttgcagcg     180

cggcggcttc gaagccgccg cccggagctg ccctttcctc ttcggtgaag tttttaaaag     240

ctgctaaaga ctcggaggaa gcaaggaaag tgcctggtag gactgacggc tgcctttgtc     300

ctcctcctct ccaccccgcc tccccccacc ctgccttccc cccctccccc gtcttctctc     360

ccgcagctgc ctcagtcggc tactctcagc caacccccct caccaccctt ctccccaccc     420

gcccccccgc ccccgtcggc ccagcgctgc cagcccgagt ttgcagagag gtaactccct     480

ttggctgcga gcgggcgagc tagctgcaca ttgcaaagaa ggctcttagg agccaggcga     540

ctggggagcg gcttcagcac tgcagccacg acccgcctgg ttaggctgca cgcggagaga     600

accctctgtt ttcccccact ctctctccac ctcctcctgc cttccccacc ccgagtgcgg     660

agccagagat caaaagatga aaaggcagtc aggtcttcag tagccaaaaa acaaaaacaa     720

caaaaacaaa aaagccgaaa taaaagaaaa agataataac tcagttctta tttgcaccta     780

cttcagtgga cactgaattt ggaaggtgga ggattttgtt tttttctttt aagatctggg     840

catcttttga atctaccctt caagtattaa gagacagact gtgagcctag cagggcagat     900

cttgtccacc gtgtgtcttc ttctgcacga gactttgagg ctgtcagagc gctttttgcg     960

tggttgctcc cgcaagtttc cttctctgga gcttcccgca ggtgggcagc tagctgcagc    1020

gactaccgca tcatcacagc ctgttgaact cttctgagca agagaagggg aggcggggta    1080

agggaagtag gtggaagatt cagccaagct caagg atg gaa gtg cag tta ggg       1133
                                       Met Glu Val Gln Leu Gly
                                       1               5

ctg gga agg gtc tac cct cgg ccg ccg tcc aag acc tac cga gga gct      1181
Leu Gly Arg Val Tyr Pro Arg Pro Pro Ser Lys Thr Tyr Arg Gly Ala
            10                  15                  20

ttc cag aat ctg ttc cag agc gtg cgc gaa gtg atc cag aac ccg ggc      1229
Phe Gln Asn Leu Phe Gln Ser Val Arg Glu Val Ile Gln Asn Pro Gly

        25                  30                  35

ccc agg cac cca gag gcc gcg agc gca gca cct ccc ggc gcc agt ttg 1277
Pro Arg His Pro Glu Ala Ala Ser Ala Ala Pro Pro Gly Ala Ser Leu
    40                  45                  50

ctg ctg ctg cag cag cag cag cag cag cag cag cag cag cag cag cag 1325
Leu Leu Leu Gln Gln Gln Gln Gln Gln Gln Gln Gln Gln Gln Gln Gln
55                  60                  65                  70

cag cag cag cag cag cag cag cag cag cag caa gag act agc ccc agg 1373
Gln Gln Gln Gln Gln Gln Gln Gln Gln Gln Gln Glu Thr Ser Pro Arg
                75                  80                  85

cag cag cag cag cag cag ggt gag gat ggt tct ccc caa gcc cat cgt 1421
Gln Gln Gln Gln Gln Gln Gly Glu Asp Gly Ser Pro Gln Ala His Arg
            90                  95                  100

aga ggc ccc aca ggc tac ctg gtc ctg gat gag gaa cag caa cct tca 1469
Arg Gly Pro Thr Gly Tyr Leu Val Leu Asp Glu Glu Gln Gln Pro Ser
        105                 110                 115

cag ccg cag tcg gcc ctg gag tgc cac ccc gag aga ggt tgc gtc cca 1517
Gln Pro Gln Ser Ala Leu Glu Cys His Pro Glu Arg Gly Cys Val Pro
    120                 125                 130

gag cct gga gcc gcc gtg gcc gcc agc aag ggg ctg ccg cag cag ctg 1565
Glu Pro Gly Ala Ala Val Ala Ala Ser Lys Gly Leu Pro Gln Gln Leu
135                 140                 145                 150

cca gca cct ccg gac gag gat gac tca gct gcc cca tcc acg ttg tcc 1613
Pro Ala Pro Pro Asp Glu Asp Asp Ser Ala Ala Pro Ser Thr Leu Ser
                155                 160                 165

ctg ctg ggc ccc act ttc ccc ggc tta agc agc tgc tcc gct gac ctt 1661
Leu Leu Gly Pro Thr Phe Pro Gly Leu Ser Ser Cys Ser Ala Asp Leu
            170                 175                 180

aaa gac atc ctg agc gag gcc agc acc atg caa ctc ctt cag caa cag 1709
Lys Asp Ile Leu Ser Glu Ala Ser Thr Met Gln Leu Leu Gln Gln Gln
        185                 190                 195

cag cag gaa gca gta tcc gaa ggc agc agc agc ggg aga gcg agg gag 1757
Gln Gln Glu Ala Val Ser Glu Gly Ser Ser Ser Gly Arg Ala Arg Glu
    200                 205                 210

gcc tcg ggg gct ccc act tcc tcc aag gac aat tac tta ggg ggc act 1805
Ala Ser Gly Ala Pro Thr Ser Ser Lys Asp Asn Tyr Leu Gly Gly Thr
215                 220                 225                 230

tcg acc att tct gac aac gcc aag gag ttg tgt aag gca gtg tcg gtg 1853
Ser Thr Ile Ser Asp Asn Ala Lys Glu Leu Cys Lys Ala Val Ser Val
                235                 240                 245

tcc atg ggc ctg ggt gtg gag gcg ttg gag cat ctg agt cca ggg gaa 1901
Ser Met Gly Leu Gly Val Glu Ala Leu Glu His Leu Ser Pro Gly Glu
            250                 255                 260

cag ctt cgg ggg gat tgc atg tac gcc cca ctt ttg gga gtt cca ccc 1949
Gln Leu Arg Gly Asp Cys Met Tyr Ala Pro Leu Leu Gly Val Pro Pro
        265                 270                 275

gct gtg cgt ccc act cct tgt gcc cca ttg gcc gaa tgc aaa ggt tct 1997
Ala Val Arg Pro Thr Pro Cys Ala Pro Leu Ala Glu Cys Lys Gly Ser
    280                 285                 290

ctg cta gac gac agc gca ggc aag agc act gaa gat act gct gag tat 2045
Leu Leu Asp Asp Ser Ala Gly Lys Ser Thr Glu Asp Thr Ala Glu Tyr
295                 300                 305                 310

tcc cct ttc aag gga ggt tac acc aaa ggg cta gaa ggc gag agc cta 2093
Ser Pro Phe Lys Gly Gly Tyr Thr Lys Gly Leu Glu Gly Glu Ser Leu
                315                 320                 325

ggc tgc tct ggc agc gct gca gca ggg agc tcc ggg aca ctt gaa ctg 2141
Gly Cys Ser Gly Ser Ala Ala Ala Gly Ser Ser Gly Thr Leu Glu Leu
            330                 335                 340

ccg tct acc ctg tct ctc tac aag tcc gga gca ctg gac gag gca gct 2189
Pro Ser Thr Leu Ser Leu Tyr Lys Ser Gly Ala Leu Asp Glu Ala Ala
        345                 350                 355

gcg tac cag agt cgc gac tac tac aac ttt cca ctg gct ctg gcc gga 2237
Ala Tyr Gln Ser Arg Asp Tyr Tyr Asn Phe Pro Leu Ala Leu Ala Gly
    360                 365                 370

ccg ccg ccc cct ccg ccg cct ccc cat ccc cac gct cgc atc aag ctg 2285
Pro Pro Pro Pro Pro Pro Pro Pro His Pro His Ala Arg Ile Lys Leu
375                 380                 385                 390

gag aac ccg ctg gac tac ggc agc gcc tgg gcg gct gcg gcg gcg cag 2333
Glu Asn Pro Leu Asp Tyr Gly Ser Ala Trp Ala Ala Ala Ala Ala Gln
                395                 400                 405

tgc cgc tat ggg gac ctg gcg agc ctg cat ggc gcg ggt gca gcg gga 2381
Cys Arg Tyr Gly Asp Leu Ala Ser Leu His Gly Ala Gly Ala Ala Gly
            410                 415                 420

ccc ggt tct ggg tca ccc tca gcc gcc gct tcc tca tcc tgg cac act 2429
Pro Gly Ser Gly Ser Pro Ser Ala Ala Ala Ser Ser Ser Trp His Thr
        425                 430                 435

cgg ccc cct cag ggg ctg gcg ggc cag gaa agc gac ttc acc gca cct 2477
Arg Pro Pro Gln Gly Leu Ala Gly Gln Glu Ser Asp Phe Thr Ala Pro
    440                 445                 450

gat gtg tgg tac cct ggc ggc atg gtg agc aga gtg ccc tat ccc agt 2525
Asp Val Trp Tyr Pro Gly Gly Met Val Ser Arg Val Pro Tyr Pro Ser
455                 460                 465                 470

ccc act tgt gtc aaa agc gaa atg ggc ccc tgg atg gat agc tac tcc 2573
Pro Thr Cys Val Lys Ser Glu Met Gly Pro Trp Met Asp Ser Tyr Ser
                475                 480                 485

gga cct tac ggg gac atg cgt ttg gag act gcc agg gac cat gtt ttg 2621
Gly Pro Tyr Gly Asp Met Arg Leu Glu Thr Ala Arg Asp His Val Leu
            490                 495                 500

ccc att gac tat tac ttt cca ccc cag aag acc tgc ctg atc tgt gga 2669
Pro Ile Asp Tyr Tyr Phe Pro Pro Gln Lys Thr Cys Leu Ile Cys Gly
        505                 510                 515

gat gaa gct tct ggg tgt cac tat gga gct ctc aca tgt gga agc tgc 2717
Asp Glu Ala Ser Gly Cys His Tyr Gly Ala Leu Thr Cys Gly Ser Cys
    520                 525                 530

aag gtc ttc ttc aaa aga gcc gct gaa ggg aaa cag aag tac ctg tgc 2765
Lys Val Phe Phe Lys Arg Ala Ala Glu Gly Lys Gln Lys Tyr Leu Cys
535                 540                 545                 550

gcc agc aga aat gat tgc act att gat aaa ttc cga agg aaa aat tgt     2813
Ala Ser Arg Asn Asp Cys Thr Ile Asp Lys Phe Arg Arg Lys Asn Cys
                555                 560                 565

cca tct tgt cgt ctt cgg aaa tgt tat gaa gca ggg atg act ctg gga     2861
Pro Ser Cys Arg Leu Arg Lys Cys Tyr Glu Ala Gly Met Thr Leu Gly
            570                 575                 580

gaa aaa ttc cgg gtt ggc aat tgc aag cat ctc aaa atg acc aga ccc     2909
Glu Lys Phe Arg Val Gly Asn Cys Lys His Leu Lys Met Thr Arg Pro
        585                 590                 595

tga agaaaggctg acttgcctca ttcaaaatga gggctctaga gggctctagt          2962

ggatagtctg gagaaacctg gcgtctgagg cttaggagct taggtttttg ctcctcaaca   3022

cagactttga cgttggggtt gggggctact ctcttgattg ctgactccct ccagcgggac   3082

caatagtgtt ttcctacctc acagggatgt tgtgaggacg ggctgtagaa gtaatagtgg   3142

ttaccattca tgtagttgtg agtatcatga ttattgtttc ctgtaatgtg gcttggcatt   3202

ggcaaagtgc tttttgattg ttcttgatca catatgatgg gggccaggca ctgactcagg   3262

cggatgcagt gaagctctgg ctcagtcgct tgcttttcgt ggtgtgctgc caggaagaaa   3322

ctttgctgat gggactcaag gtgtcacctt ggacaagaag caactgtgtc tgtctgaggt   3382

tcctgtggcc atctttattt gtgtattagg caattcgtat ttccccctta ggttctagcc   3442

ttctggatcc cagccagtga cctagatctt agcctcaggc cctgtcactg agctgaaggt   3502

agtagctgat ccacagaagt tcagtaaaca aggaccagat ttctgcttct ccaggagaag   3562

aagccagcca acccctctct tcaaacacac tgagagacta cagtccgact ttccctctta   3622

catctagcct tactgtagcc acactccttg attgctctct cacatcacat gcttctcttc   3682

atcagttgta agcctctcat tcttctccca agccagactc aaatattgta ttgatgtcaa   3742

agaagaatca cttagagttt ggaatatctt gttctctctc tgctccatag cttccatatt   3802

gacaccagtt tctttctagt ggagaagtgg agtctgtgaa gccagggaaa cacacatgtg   3862

agagtcagaa ggactctccc tgacttgcct ggggcctgtc tttcccacct tctccagtct   3922

gtctaaacac acacacacac acacacacac acacacacac gctctctctc tctctccccc   3982

cccaacacac acacactctc tctctctctc acacacacac acatacacac acacttcttt   4042

ctctttcccc tgactcagca acattctgga gaaaagccaa ggaaggactt caggagggga   4102

gtttccccct tctcagggca gaattttaat ctccagacca acaagaagtt ccctaatgtg   4162

gattgaaagg ctaatgaggt ttatttttaa ctactttcta tttgtttgaa tgttgcatat   4222

ttctactagt gaaattttcc cttaataaag ccattaatac accaaaaaaa aaaaaaaaaa   4282

aaaaa                                                               4287

<210>  12
<211>  598
<212>  PRT
<213>  Homo sapiens

<400>  12

Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Gln Glu Thr Ser Pro Arg Gln Gln Gln Gln Gln Gln Gly Glu Asp Gly
                85                  90                  95

Ser Pro Gln Ala His Arg Arg Gly Pro Thr Gly Tyr Leu Val Leu Asp
            100                 105                 110

Glu Glu Gln Gln Pro Ser Gln Pro Gln Ser Ala Leu Glu Cys His Pro
        115                 120                 125

Glu Arg Gly Cys Val Pro Glu Pro Gly Ala Ala Val Ala Ala Ser Lys
    130                 135                 140

Gly Leu Pro Gln Gln Leu Pro Ala Pro Pro Asp Glu Asp Asp Ser Ala
145                 150                 155                 160

Ala Pro Ser Thr Leu Ser Leu Leu Gly Pro Thr Phe Pro Gly Leu Ser
                165                 170                 175

Ser Cys Ser Ala Asp Leu Lys Asp Ile Leu Ser Glu Ala Ser Thr Met
            180                 185                 190

Gln Leu Leu Gln Gln Gln Gln Gln Glu Ala Val Ser Glu Gly Ser Ser
        195                 200                 205

Ser Gly Arg Ala Arg Glu Ala Ser Gly Ala Pro Thr Ser Ser Lys Asp
    210                 215                 220

Asn Tyr Leu Gly Gly Thr Ser Thr Ile Ser Asp Asn Ala Lys Glu Leu
225                 230                 235                 240

Cys Lys Ala Val Ser Val Ser Met Gly Leu Gly Val Glu Ala Leu Glu
                245                 250                 255

His Leu Ser Pro Gly Glu Gln Leu Arg Gly Asp Cys Met Tyr Ala Pro
            260                 265                 270

Leu Leu Gly Val Pro Pro Ala Val Arg Pro Thr Pro Cys Ala Pro Leu
        275                 280                 285

Ala Glu Cys Lys Gly Ser Leu Leu Asp Asp Ser Ala Gly Lys Ser Thr
    290                 295                 300

Glu Asp Thr Ala Glu Tyr Ser Pro Phe Lys Gly Gly Tyr Thr Lys Gly
305                 310                 315                 320

Leu Glu Gly Glu Ser Leu Gly Cys Ser Gly Ser Ala Ala Ala Gly Ser
                325                 330                 335

Ser Gly Thr Leu Glu Leu Pro Ser Thr Leu Ser Leu Tyr Lys Ser Gly
            340                 345                 350

Ala Leu Asp Glu Ala Ala Ala Tyr Gln Ser Arg Asp Tyr Tyr Asn Phe
        355                 360                 365

Pro Leu Ala Leu Ala Gly Pro Pro Pro Pro Pro Pro Pro Pro His Pro
    370                 375                 380

His Ala Arg Ile Lys Leu Glu Asn Pro Leu Asp Tyr Gly Ser Ala Trp
385                 390                 395                 400

Ala Ala Ala Ala Ala Gln Cys Arg Tyr Gly Asp Leu Ala Ser Leu His
                405                 410                 415

Gly Ala Gly Ala Ala Gly Pro Gly Ser Gly Ser Pro Ser Ala Ala Ala
            420                 425                 430

Ser Ser Ser Trp His Thr Arg Pro Pro Gln Gly Leu Ala Gly Gln Glu
        435                 440                 445

Ser Asp Phe Thr Ala Pro Asp Val Trp Tyr Pro Gly Gly Met Val Ser
    450                 455                 460

Arg Val Pro Tyr Pro Ser Pro Thr Cys Val Lys Ser Glu Met Gly Pro
465                 470                 475                 480

Trp Met Asp Ser Tyr Ser Gly Pro Tyr Gly Asp Met Arg Leu Glu Thr
                485                 490                 495

Ala Arg Asp His Val Leu Pro Ile Asp Tyr Tyr Phe Pro Pro Gln Lys
            500                 505                 510

Thr Cys Leu Ile Cys Gly Asp Glu Ala Ser Gly Cys His Tyr Gly Ala
        515                 520                 525

Leu Thr Cys Gly Ser Cys Lys Val Phe Phe Lys Arg Ala Ala Glu Gly
    530                 535                 540

Lys Gln Lys Tyr Leu Cys Ala Ser Arg Asn Asp Cys Thr Ile Asp Lys
545                 550                 555                 560

Phe Arg Arg Lys Asn Cys Pro Ser Cys Arg Leu Arg Lys Cys Tyr Glu
                565                 570                 575

Ala Gly Met Thr Leu Gly Glu Lys Phe Arg Val Gly Asn Cys Lys His
            580                 585                 590

Leu Lys Met Thr Arg Pro
        595

<210>  13
<211>  1403
<212>  DNA
<213>  Homo sapiens

<400>  13
aaaaattccg ggttggcaat tgcaagcatc tcaaaatgac cagaccctga agaaaggctg      60

acttgcctca ttcaaaatga gggctctaga gggctctagt ggatagtctg gagaaacctg     120

gcgtctgagg cttaggagct taggtttttg ctcctcaaca cagactttga cgttggggtt     180

gggggctact ctcttgattg ctgactccct ccagcgggac caatagtgtt ttcctacctc     240

acagggatgt tgtgaggacg ggctgtagaa gtaatagtgg ttaccattca tgtagttgtg     300

agtatcatga ttattgtttc ctgtaatgtg gcttggcatt ggcaaagtgc tttttgattg     360

ttcttgatca catatgatgg gggccaggca ctgactcagg cggatgcagt gaagctctgg     420

ctcagtcgct tgcttttcgt ggtgtgctgc caggaagaaa ctttgctgat gggactcaag     480

gtgtcacctt ggacaagaag caactgtgtc tgtctgaggt tcctgtggcc atctttattt     540

gtgtattagg caattcgtat ttccccctta ggttctagcc ttctggatcc cagccagtga     600

cctagatctt agcctcaggc cctgtcactg agctgaaggt agtagctgat ccacagaagt     660

tcagtaaaca aggaccagat ttctgcttct ccaggagaag aagccagcca acccctctct     720

tcaaacacac tgagagacta cagtccgact ttccctctta catctagcct tactgtagcc     780

acactccttg attgctctct cacatcacat gcttctcttc atcagttgta agcctctcat     840

tcttctccca agccagactc aaatattgta ttgatgtcaa agaagaatca cttagagttt     900

ggaatatctt gttctctctc tgctccatag cttccatatt gacaccagtt tctttctagt     960

ggagaagtgg agtctgtgaa gccagggaaa cacacatgtg agagtcagaa ggactctccc    1020

tgacttgcct ggggcctgtc tttcccacct tctccagtct gtctaaacac acacacacac 1080

acacacacac acacacacac gctctctctc tctctccccc cccaacacac acacactctc 1140

tctctctctc acacacacac acatacacac acacttcttt ctctttcccc tgactcagca 1200

acattctgga gaaaagccaa ggaaggactt caggagggga gtttccccct tctcagggca 1260

gaattttaat ctccagacca acaagaagtt ccctaatgtg gattgaaagg ctaatgaggt 1320

ttatttttaa ctactttcta tttgtttgaa tgttgcatat ttctactagt gaaattttcc 1380

cttaataaag ccattaatac acc 1403

<210> 14
<211> 1155
<212> DNA
<213> Homo sapiens

<400> 14
ggtcctcgct gctgcctgct acttccaaaa agatgtgtct ttcatgagaa aaacaagatc 60

attaatccac ttcgatttgg aaatggaatt tgaagaaagg caagcctatt tctgagtgcc 120

tgcaactgta gcctcatacc caattattca ttattagcct ggaaaaccca agtgcctaga 180

atccaaccct ctcccctctc ctcttaagtc taatttagac cagttgtcta tctctggctt 240

tctgtgaggt gttcaatacc ttgtctgcct atgtgcacat ttatagacaa caactagttc 300

tcttatcctg gagcagggcc atgtgtggat cttcatatag ataactatat cctccccatc 360

ctcacagggc agtagtatta tttaaacaga acaaagtacc tcacatgaat tgacccaggc 420

tggatgagag acaatttcaa aagaatcatc tcaagtagcg tccagtactc ccaaacatca 480

caggtagatg ttctgtgagt ggctttccaa gcatccacat caaatgagac tcagatatct 540

gagaaaactc aaccttgttt tggtttgctt ggtgcacccc aaagaaatcc aacaattgag 600

gtctacagtg gagaagaagt aggactgggg tcagggagta cagaggcaaa ggcaggaagg 660

gtgacaaagt gattgacaag aaaaaatgtt ctccatatga atgttgcagc cccatgttga 720

gggttcttat acactcaact gtcaattatt tagccttctg tgaattatgt atagtataaa 780

agatagggac tctcaagtag ggaacctctt ggcttgccat ctggcaatat gaattgcaag 840

tccactttga tgcaggtaaa gtttaatggt aacaaaagtc ctcataacat ttggatgcaa 900

atcttaacat taattccatg tctcagccaa cattctccat tattaagcag cctgtgatgt 960

gattacagtg aaccactttt gaaaaggagc ctgtgtataa cagatagttt cactatacta 1020

tataaccgtc agatgcaggc ttgtaaatta atttgttggt gacaatgttt cagtacattt 1080

tcaaattgat tcattggtat agtactcaaa tttgagtggg cttggtgaac acaatgaaga 1140

caagctgaga agtgc 1155

<210> 15
<211> 2763
<212> DNA

<213>  Homo sapiens

<220>
<221>  CDS
<222>  (1)..(2763)

<400>  15
atg gaa gtg cag tta ggg ctg gga agg gtc tac cct cgg ccg ccg tcc          48
aag acc tac cga gga gct ttc cag aat ctg ttc cag agc gtg cgc gaa          96
gtg atc cag aac ccg ggc ccc agg cac cca gag gcc gcg agc gca gca         144
cct ccc ggc gcc agt ttg ctg ctg ctg cag cag cag cag cag cag cag         192
cag cag cag cag cag cag cag cag cag cag cag cag cag cag cag caa         240
gag act agc ccc agg cag cag cag cag cag cag ggt gag gat ggt tct         288
ccc caa gcc cat cgt aga ggc ccc aca ggc tac ctg gtc ctg gat gag         336
gaa cag caa cct tca cag ccg cag tcg gcc ctg gag tgc cac ccc gag         384
aga ggt tgc gtc cca gag cct gga gcc gcc gtg gcc gcc agc aag ggg         432
ctg ccg cag cag ctg cca gca cct ccg gac gag gat gac tca gct gcc         480
cca tcc acg ttg tcc ctg ctg ggc ccc act ttc ccc ggc tta agc agc         528
tgc tcc gct gac ctt aaa gac atc ctg agc gag gcc agc acc atg caa         576
ctc ctt cag caa cag cag cag gaa gca gta tcc gaa ggc agc agc agc         624
ggg aga gcg agg gag gcc tcg ggg gct ccc act tcc tcc aag gac aat         672
tac tta ggg ggc act tcg acc att tct gac aac gcc aag gag ttg tgt         720
aag gca gtg tcg gtg tcc atg ggc ctg ggt gtg gag gcg ttg gag cat      768
ctg agt cca ggg gaa cag ctt cgg ggg gat tgc atg tac gcc cca ctt      816
ttg gga gtt cca ccc gct gtg cgt ccc act cct tgt gcc cca ttg gcc      864
gaa tgc aaa ggt tct ctg cta gac gac agc gca ggc aag agc act gaa      912
gat act gct gag tat tcc cct ttc aag gga ggt tac acc aaa ggg cta      960
gaa ggc gag agc cta ggc tgc tct ggc agc gct gca gca ggg agc tcc     1008
ggg aca ctt gaa ctg ccg tct acc ctg tct ctc tac aag tcc gga gca     1056
ctg gac gag gca gct gcg tac cag agt cgc gac tac tac aac ttt cca     1104
ctg gct ctg gcc gga ccg ccg ccc cct ccg ccg cct ccc cat ccc cac     1152
gct cgc atc aag ctg gag aac ccg ctg gac tac ggc agc gcc tgg gcg     1200
gct gcg gcg gcg cag tgc cgc tat ggg gac ctg gcg agc ctg cat ggc     1248
gcg ggt gca gcg gga ccc ggt tct ggg tca ccc tca gcc gcc gct tcc     1296
tca tcc tgg cac act ctc ttc aca gcc gaa gaa ggc cag ttg tat gga     1344
ccg tgt ggt ggt ggt ggg ggt ggt ggc ggc ggc ggc ggc ggc ggc ggc     1392
ggc ggc ggc ggc ggc ggc ggc ggc ggc gag gcg gga gct gta gcc ccc     1440
tac ggc tac act cgg ccc cct cag ggg ctg gcg ggc cag gaa agc gac     1488
ttc acc gca cct gat gtg tgg tac cct ggc ggc atg gtg agc aga gtg 1536
ccc tat ccc agt ccc act tgt gtc aaa agc gaa atg ggc ccc tgg atg 1584
gat agc tac tcc gga cct tac ggg gac atg cgt ttg gag act gcc agg 1632
gac cat gtt ttg ccc att gac tat tac ttt cca ccc cag aag acc tgc 1680
ctg atc tgt gga gat gaa gct tct ggg tgt cac tat gga gct ctc aca 1728
tgt gga agc tgc aag gtc ttc ttc aaa aga gcc gct gaa ggg aaa cag 1776
aag tac ctg tgc gcc agc aga aat gat tgc act att gat aaa ttc cga 1824
agg aaa aat tgt cca tct tgt cgt ctt cgg aaa tgt tat gaa gca ggg 1872
atg act ctg gga gcc cgg aag ctg aag aaa ctt ggt aat ctg aaa cta 1920
cag gag gaa gga gag gct tcc agc acc acc agc ccc act gag gag aca 1968
acc cag aag ctg aca gtg tca cac att gaa ggc tat gaa tgt cag ccc 2016
atc ttt ctg aat gtc ctg gaa gcc att gag cca ggt gta gtg tgt gct 2064
gga cac gac aac aac cag ccc gac tcc ttt gca gcc ttg ctc tct agc 2112
ctc aat gaa ctg gga gag aga cag ctt gta cac gtg gtc aag tgg gcc 2160
aag gcc ttg cct ggc ttc cgc aac tta cac gtg gac gac cag atg gct 2208
gtc att cag tac tcc tgg atg ggg ctc atg gtg ttt gcc atg ggc tgg 2256
cga tcc ttc acc aat gtc aac tcc agg atg ctc tac ttc gcc cct gat 2304
ctg gtt ttc aat gag tac cgc atg cac aag tcc cgg atg tac agc cag 2352
tgt gtc cga atg agg cac ctc tct caa gag ttt gga tgg ctc caa atc 2400
acc ccc cag gaa ttc ctg tgc atg aaa gca ctg cta ctc ttc agc att 2448
att cca gtg gat ggg ctg aaa aat caa aaa ttc ttt gat gaa ctt cga 2496
atg aac tac atc aag gaa ctc gat cgt atc att gca tgc aaa aga aaa 2544
aat ccc aca tcc tgc tca aga cgc ttc tac cag ctc acc aag ctc ctg 2592
gac tcc gtg cag cct att gcg aga gag ctg cat cag ttc act ttt gac 2640
ctg cta atc aag tca cac atg gtg agc gtg gac ttt ccg gaa atg atg 2688
gca gag atc atc tct gtg caa gtg ccc aag atc ctt tct ggg aaa gtc 2736
aag ccc atc tat ttc cac acc cag tga 2763

<210> 16
<211> 920
<212> PRT
<213> Homo sapiens

<400> 16

<210>  17
<211>  15
<212>  DNA
<213>  Homo sapiens

<400>  17
agaacagcaa gtgct                                                        15

<210>  18
<211>  35
<212>  DNA
<213>  Homo sapiens

<400>  18
gtggtgcagg gatcagggag tctcacaatc tcctg                                  35

<210>  19
<211>  23
<212>  DNA
<213>  Artificial Sequence

<220>
<223>  Primer

<400>  19
gcatggtgag cagagtgccc tat                                               23

<210>  20
<211>  23
<212>  DNA
<213>  Artificial Sequence

<220>
<223>  Primer

<400>  20
tactccggac cttacgggga cat                                               23

<210>  21
<211>  26
<212>  DNA
<213>  Artificial Sequence

<220>
<223>  Primer

<400>  21
atggaagtgc agttagggct gggaag                                          26


<210>  22
<211>  29
<212>  DNA
<213>  Artificial Sequence

<220>
<223>  Primer

<400>  22
ggtctggtca ttttgagatg cttgcaatt                                       29


<210>  23
<211>  26
<212>  DNA
<213>  Artificial Sequence

<220>
<223>  Primer

<400>  23
aaatgttatg aagcagggat gactct                                          26


<210>  24
<211>  22
<212>  DNA
<213>  Artificial Sequence

<220>
<223>  Primer

<400>  24
gcttctgggt tgtctcctca gt                                              22


<210>  25
<211>  18
<212>  DNA
<213>  Artificial Sequence

<220>
<223>  Probe

<400>  25
actacaggag gaaggaga                                                   18


<210>  26
<211>  22
<212>  DNA

<213>  Artificial Sequence

<220>
<223>  Primer

<400>  26
catcttgtcg tcttcggaaa tg                                                22


<210>  27
<211>  20
<212>  DNA
<213>  Artificial Sequence

<220>
<223>  Primer

<400>  27
tgccaacccg gaatttttct                                                   20


<210>  28
<211>  16
<212>  DNA
<213>  Artificial Sequence

<220>
<223>  Probe

<400>  28
cagggatgac tctggg                                                       16


<210>  29
<211>  19
<212>  DNA
<213>  Artificial Sequence

<220>
<223>  siRNA against ARaiv1

<400>  29
gattgttctt gatcacata                                                    19


<210>  30
<211>  19
<212>  RNA
<213>  Artificial Sequence

<220>
<223>  siRNA against AR

<400>  30
ggaacucgau cguaucauu                                                    19


<210>  31
<211>  20
<212>  DNA
<213>  Artificial Sequence

<220>
<223>  siRNA against AR and ARaiv1

<400>  31
gcagaaatgg attgcactat                                                    20


<210>  32
<211>  15
<212>  DNA
<213>  Artificial Sequence

<220>
<223>  Consensus androgen responsive element sequence


<220>
<221>  misc_feature
<222>  (7)..(9)
<223>  n is a, c, g, or t

<400>  32
agaacannnt gttct                                                         15


<210>  33
<211>  22
<212>  RNA
<213>  Homo sapiens

<400>  33
ugagguagua aguuguauug uu                                                 22


<210>  34
<211>  22
<212>  RNA
<213>  Homo sapiens

<400>  34
ugagguagua gguuguauag uu                                                 22


<210>  35
<211>  22
<212>  RNA
<213>  Homo sapiens

<400>  35
ugagguagua gguugugugg uu                                                 22


<210>  36
<211>  22
<212>  RNA
<213>  Homo sapiens

<400>  36
ugagguagua gguuguaugg uu                                                 22


<210>  37
<211>  21
<212>  RNA
<213>  Homo sapiens

<400>  37
agagguagua gguugcauag u                                                  21

<210> 38
<211> 21
<212> RNA
<213> Homo sapiens

<400> 38
ugagguagga gguuguauag u 21


<210> 39
<211> 22
<212> RNA
<213> Homo sapiens

<400> 39
ugagguagua gauuguauag uu 22


<210> 40
<211> 21
<212> RNA
<213> Homo sapiens

<400> 40
ugagguagua guuuguacag u 21


<210> 41
<211> 21
<212> RNA
<213> Homo sapiens

<400> 41
ugagguagua guuugugcug u 21


<210> 42
<211> 24
<212> RNA
<213> Homo sapiens

<400> 42
ucccugagac ccuuuaaccu guga 24


<210> 43
<211> 22
<212> RNA
<213> Homo sapiens

<400> 43
ugugacuggu ugaccagagg gg 22


<210> 44
<211> 21
<212> RNA
<213> Homo sapiens

<400> 44
agggagggac gggggcugug c 21

<210>  45
<211>  22
<212>  RNA
<213>  Homo sapiens

<400>  45
aguggggaac ccuuccauga gg                                                22


<210>  46
<211>  23
<212>  RNA
<213>  Homo sapiens

<400>  46
ugagugugug ugugugagug ugu                                               23


<210>  47
<211>  23
<212>  DNA
<213>  Homo sapiens

<400>  47
accaatagtg ttttcctacc tca                                               23


<210>  48
<211>  32
<212>  DNA
<213>  Homo sapiens

<400>  48
cttcaggagg ggagtttccc ccttctcagg gc                                     32


<210>  49
<211>  25
<212>  DNA
<213>  Homo sapiens

<400>  49
gccttctgga tcccagccag tgacc                                             25


<210>  50
<211>  25
<212>  DNA
<213>  Homo sapiens

<400>  50
acacacgctc tctctctctc tcccc                                             25


<210>  51
<211>  24
<212>  DNA
<213>  Homo sapiens

<400>  51
cttcaggagg ggagtttccc cctt                                              24


<210>  52
<211>  23

<212>  DNA
<213>  Homo sapiens

<400>  52
aaacacacac acacacacac aca                                              23

## Description

### Technical Field

The present invention relates to a novel variant androgen receptor, a method of screening for an agent for the prophylaxis and/or treatment of cancer using the variant, and the like.

### [Background of the Invention]

At present, chemical castration (endocrine therapy) by a hormone medicament has been widely used for cancer treatment. Endocrine therapy for prostate cancer and the like utilizes the mechanism of suppressing the growth of cancer cells by cutting off the function of male sex hormone since it stimulates the growth of cancer cells.
Androgen is a generic term of steroid hormone having a male sex hormone action, and includes testosterone synthesized by the testis, dehydroepiandrosterone synthesized by the adrenal cortex and the like.
For testosterone that stimulates the growth of cancer cells to enter the cells and exhibit its action, testosterone needs to be converted to dihydrotestosterone and bind to an androgen receptor (hereinafter sometimes to be abbreviated as AR) in the nucleus of the cancer cells. An anti-androgenic agent binds to AR to prevent binding between dihydrotestosterone and AR, thereby exhibiting efficacy. It is known, however, when the treatment is continued, the effect disappears and the cancer starts to grow again (androgen independent prostate cancer; AIPC). An effective prophylactic or therapeutic method for relapsed prostate cancer that acquired androgen independence has not been developed yet. While the mechanism of relapsed prostate cancer relative to the endocrine therapy has not been elucidated yet, plural factors, such as emergence or increase of cells that acquired androgen hypersensitivity by amplification or overexpression of AR gene, capability of utilizing a substance other than androgen (e.g., glucocorticoid, anti-androgenic agent per se) as an agonist due to the mutation of AR and the like, are being clarified. For example, a mutant androgen receptor wherein flutamide and bicalutamide, which are anti-androgenic agents, act as agonists, was separated from patients with relapsed prostate cancer (e.g., see patent document 1).

Hu et al. (non-patent document 1) found 7 kinds of AR splice variants (AR-V1, AR-V2, AR-V3, AR-V4, AR-V5, AR-V6, AR-V7) lacking a ligand binding domain. However, whether these AR splice variants become treatment targets of AIPC has not been demonstrated. On the other hand, Guo et al. (non-patent document 2) found 3 kinds of AR splice variants lacking a ligand binding domain, and suggest that one kind thereof (AR3; having the same amino acid sequence as the above-mentioned AR-V7) can be a treatment target of AIPC. However, as a concrete image of a therapeutic drug with the AR splice variant as a target, an example using only an shRNA expressing lentivirus for the variant is merely shown. Thus, the possibility of a treatment with a single agent of siRNA or a low-molecular-weight compound that inhibits the function of AR splice variant, and a combined therapy with other kind of active ingredient or radiation therapy etc. has not been verified at all.
In addition, the presence of variant AR other than those mentioned above (AR^{v567es}; non-patent document 4) and cDNA fragments capable of encoding variant AR (AR-V8, AR-V9, AR-V10, AR-V11; non-patent document 5) has also been reported.
Note that some compounds are known to inhibit the activity of the N-terminal domain of AR (patent document 2, non-patent document 3). However, whether or not they inhibit variant AR, represented by AR3 (AR-V7), which is inherently expressed by prostate cancer cells such as CWR22Rvl, VCaP, JDCaP/JDCaP-hr and the like, has not been verified.

Meanwhile, a 90 kDa heat shock protein (HSP90), which is a molecular chaperone, controls interaction, intracellular transport, degradation and function of various proteins that play an important role in the cell control, such as kinase, transcription factor, hormone receptor and the like, via enhancement of the folding of these proteins, thus playing multifaceted roles in the control of the growth and survival of the cells. Particularly, increased expression of HSP90 is seen in cancer cells, and the level thereof is correlated with the malignancy of the cancer. In addition, HSP90 inhibitor is expected to be a new type of anti-cancer agent, and its action to potentiate the effects of radiation and conventional anti-cancer agents is attracting attention.
While HSP90 inhibitors are known to promote degradation of mutant AR wherein a polyglutamine chain is abnormally elongated, which is a causative gene of spinobulbar muscular atrophy, their actions on mutant AR observed in relapsed androgen-independent prostate cancer and the above-mentioned variant AR remain to be elucidated.

### [Document List]

### [patent documents]

patent document 1: WO 2003/102188
patent document 2: WO 2010/000066

### [non-patent documents]

non-patent document 1: Cancer Res., 2009, 69(1): 16-22
non-patent document 2: Cancer Res., 2009, 69(6): 2305-2313
non-patent document 3: Cancer Cell., 2010, 17: 535-546
non-patent document 4: The Journal of Clinical Investigation, 2010, 120(8):2715-2730
non-patent document 5: Proc. Natl. Acad. Sci. USA, 2010 Sep 7. [Epub ahead of print]

### [SUMMARY OF THE INVENTION]

### Problems to be Solved by the Invention

A medicament effective for the prophylaxis or treatment of androgen-independent prostate cancer that relapsed after endocrine therapy, has been demanded. It is therefore an object of the present invention to identify a novel variant AR lacking ligand binding domain, clarify the involvement of the variant AR in relapsed androgen-independent prostate cancer, and provide a method of searching a novel prophylactic or therapeutic drug for relapsed androgen-independent prostate cancer, which uses the variant AR inhibitory activity as an index.

### Means of Solving the Problems

The present inventors have conducted unique studies and found that a C-terminally deficient variant AR emerges during the process of conversion of androgen dependent prostate cancer (ADPC) tumor line (JDCaP) into AIPC (JDCaP-hr). The 3' terminal sequence of mRNA was cloned and the nucleotide sequence was analyzed. As a result, two kinds of variant AR clones (ARaiv1 and ARaiv2) wherein a part of the third intron (intron 3) is linked to immediately after the third exon (exon 3) of AR gene to generate a stop codon were identified, and translation products predicted from these were constitutively active molecules lacking the ligand binding domain. ORF cDNA of ARaiv1 was acquired from CWR22Rvl cell and JDCaP-hr cell and the sequence was analyzed. As a result, ARaiv1 was found to contain a variant having a intact first exon, as well as three variants (ARaiv1Δ1 - Δ3) lacking a part of the first exon (exon 1).
These 4 kinds of ARaiv1 were all confirmed to be constitutively active molecules by a reporter gene assay. ARaiv1 without deletion in the first exon has the same amino acid sequence as one of the AR splice variants reported by Hu et al. (non-patent document 1) and Guo et al. (non-patent document 2) (AR-V7 and AR3, respectively). It has been clarified that ARaiv1 is also highly expressed in other AIPC tumor lines, and its expression is increased in 7.9% of the clinical samples of prostate cancer.
Therefore, the present inventors have specifically knocked down ARaiv1 in ARaiv1 highly expressing tumor cell line to find that the growth of tumor cell is remarkably suppressed in the absence of androgen. It has also been shown that the growth of tumor cell is more remarkably suppressed when both the wild-type AR and ARaiv1 are knocked down than when either one alone is knocked down.
Moreover, it has been found by exposure of COS7 cells transfected with ARaiv1 and androgen responsive reporter plasmid to geldanamycin (HSP90 inhibitor) that the HSP90 inhibitor inhibits activation of reporter gene transcription by ARaiv1.
Furthermore, it has been found by exposure of JDCaP-hr cell to geldanamycin (HSP90 inhibitor) that the HSP90 inhibitor inhibits expression of AR and shows a higher expression inhibitory effect on variant AR lacking a ligand binding domain.
The present inventors have conducted further investigations based on these findings, and completed the present invention.

Accordingly, the present invention provides, as one embodiment,
[1-A] a method of screening for a substance for the prophylaxis or treatment of cancer, comprising a step of contacting a cell comprising a nucleic acid encoding a variant androgen receptor protein comprising the amino acid sequence shown by SEQ ID NO: 6, 8, 10 or 12, and a reporter gene under control of an androgen responsive promoter with a test compound,
   a step of measuring the expression level of the reporter gene, and
   a step of selecting a compound that suppresses the expression of the reporter gene as compared to a control without contact with the test compound,
[2-A] a method of screening for a substance for the prophylaxis or treatment of cancer, comprising a step of contacting a variant androgen receptor protein comprising the amino acid sequence shown by SEQ ID NO: 6, 8, 10 or 12 with a test compound,
   a step of measuring the activity of the protein, and a step of selecting a compound that suppresses the activity of the protein as compared to a control without contact with the test compound,
[3-A] a variant androgen receptor protein comprising the amino acid sequence shown by SEQ ID NO: 6, 8, 10 or 12,
[4-A] a nucleic acid encoding the protein of the above-mentioned [3-A],
[5-A] a cell transduced with the nucleic acid of the above-mentioned [4-A],
[6-A] a nucleic acid of any of the following (a) - (e),
   (a) an antisense nucleic acid for a nucleic acid encoding the protein of the above-mentioned [3-A]
   (b) siRNA for RNA encoding the protein of the above-mentioned [3-A]
   (c) a nucleic acid capable of generating siRNA for RNA encoding the protein of the above-mentioned [3-A]
   (d) miRNA for RNA encoding the protein of the above-mentioned [3-A]
   (e) a nucleic acid capable of generating miRNA for RNA encoding the protein of the above-mentioned [3-A],
[7-A] a method of evaluating the efficacy of a substance for the prophylaxis or treatment of cancer, comprising a step of contacting a test compound with an animal or tissue thereof into which the nucleic acid of the above-mentioned [4-A] has been transduced, and measuring growth, differentiation or cell death, canceration or cancer growth of the tissue,
[8-A] a diagnostic reagent for cancer expressing a variant androgen receptor lacking a ligand binding domain, which comprises a substance that specifically recognizes the amino acid sequence shown by SEQ ID NO: 2 or the nucleotide sequence shown by SEQ ID NO: 14, and the like.

As another embodiment, the present invention provides
[1-B] a function inhibitor of a variant androgen receptor lacking a ligand binding domain, comprising an HSP90 inhibitor,
[2-B] the inhibitor of the above-mentioned [1-B], wherein the variant androgen receptor comprises a C-terminal amino acid sequence shown by SEQ ID NO: 1 or 2,
[3-B] the inhibitor of the above-mentioned [1-B], wherein the HSP90 inhibitor is geldanamycin or a derivative thereof,
[4-B] an agent for the prophylaxis or treatment of cancer, comprising a substance that inhibits a function of a variant androgen receptor lacking a ligand binding domain (particularly, a variant androgen receptor protein comprising the amino acid sequence shown by SEQ ID NO: 6, 8, 10 or 12),
[5-B] the agent of the above-mentioned [4-B], wherein the variant androgen receptor comprises a C-terminal amino acid sequence shown by SEQ ID NO: 1 or 2,
[6-B] the agent of the above-mentioned [4-B], wherein the aforementioned substance is an expression inhibitor of the aforementioned receptor protein,
[7- B] the agent of the above-mentioned [6-B], wherein the aforementioned substance is any of the following (a) - (e),
   (a) an antisense nucleic acid for a nucleic acid encoding the above-mentioned receptor protein
   (b) siRNA for RNA encoding the above-mentioned receptor protein
   (c) a nucleic acid capable of generating siRNA for RNA encoding the above-mentioned receptor protein
   (d) miRNA for RNA encoding the above-mentioned receptor protein
   (e) a nucleic acid capable of generating miRNA for RNA encoding the above-mentioned receptor protein [8-B] the agent of the above-mentioned [7-B], wherein the target sequence of the aforementioned nucleic acid is the entire or partial fourth exon of mRNA encoding the aforementioned receptor protein,
[9-B] the agent of the above-mentioned [4-B], wherein the cancer is prostate cancer,
[10-B] the agent of the above-mentioned [4-B], wherein the cancer is androgen independent prostate cancer,
[11-B] the agent of the above-mentioned [4-B], wherein the cancer expresses the aforementioned receptor,
[12-B] the agent of the above-mentioned [11-B], which is combined with an androgen receptor signal inhibitor,
[13-B] a method for the prophylaxis or treatment of cancer in an animal, comprising administering an effective amount of a substance that inhibits a function of a variant androgen receptor lacking a ligand binding domain to the animal,
[14-B] use of a substance that inhibits a function of a variant androgen receptor lacking a ligand binding domain for the production of an agent for the prophylaxis or treatment of cancer,
[15-B] a diagnostic reagent for cancer expressing a variant androgen receptor lacking a ligand binding domain, which comprises a substance that specifically recognizes the amino acid sequence shown by SEQ ID NO: 1 or 2 or the nucleotide sequence shown by SEQ ID NO: 13 or 14,
[16-B] the agent of the above-mentioned [4-B], which is administered using the reagent of the above-mentioned [15-B] to a patient diagnosed with cancer expressing a variant androgen receptor lacking a ligand binding domain,
[17-B] a variant androgen receptor protein comprising the amino acid sequence shown by SEQ ID NO: 6, 8, 10 or 12,
[18-B] a nucleic acid encoding the protein of the above-mentioned [17-B],
[19-B] a cell into which the nucleic acid of the above-mentioned [18-B] has been transfected,
[20-B] a method of screening for a substance for the prophylaxis or treatment of cancer, comprising contacting a test compound with a cell comprising the nucleic acid of the above-mentioned [18-B], and a reporter gene under control of an androgen responsive promoter, and measuring the expression level of the reporter gene,
[21-B] an animal transduced with the nucleic acid of the above-mentioned [18-B],
[22-B] a method of evaluating the efficacy of a substance for the prophylaxis or treatment of cancer, comprising contacting a test compound with an animal or tissue thereof into which the nucleic acid of the above-mentioned [18-B] has been transduced, and measuring growth, differentiation or cell death, canceration or cancer growth of the tissue, and the like.

### Effect of the Invention

Using the inhibition of transcription regulation by the novel variant AR of the present invention as an index, a substance effective for the prophylaxis or treatment of cancer expressing the variant AR can be searched for. In addition, detection of a C-terminal sequence specific to the variant AR or a sequence resulting from partial deletion of exon 1 enables diagnosis of cancer expressing the variant AR, thus making it possible to determine an appropriate administration subject for a substance obtained by the aforementioned search method.
Moreover, an HSP90 inhibitor can shut off AR signal that is mediated by the variant by inhibiting the function (expression and/or activity) of a ligand independent (ligand binding domain-deleted) variant AR which could be one factor of relapsed androgen-independent prostate cancer. Therefore, it is useful for the treatment and prevention of recurrence of cancer expressing the variant AR. Since other medicament that inhibits the function (expression and/or activity) of the variant AR can also inhibit transcription regulating action by the variant AR, it is similarly useful for the treatment and prevention of recurrence of cancer expressing the variant AR. Moreover, a combined use of a inhibitor of conventional AR signal via full-length AR with an expression/activity inhibitor of the above-mentioned variant AR affords an effect of increased efficacy and/or decreased side effects.

### Brief Description of the Drawings

Fig. 1 shows Western blot analysis results of AR in JDCaP and JDCaP-hr, wherein N-term. means that an antibody specific to the N-terminal of AR was used as a probe, and C-term. means that an antibody specific to the C-terminal of AR was used as a probe.
Fig. 2 shows (a) 3' terminal nucleotide sequence of ARaiv1 cDNA (SEQ ID NO: 3, the 2703-position - the 4422-position) and predicted amino acid sequence (SEQ ID NO: 4, the 530-position - 643-position) and (b) 3' terminal nucleotide sequence of ARaiv2 cDNA (SEQ ID NO: 5, the 2703-position - 4174-position) and predicted amino acid sequence (SEQ ID NO: 6, the 530-position - 642-position).
Fig. 3 is a schematic diagram showing the structures of 4 kinds of ARaiv1 ORF clones. AF1: transcription-activation domain 1; DBD: DNA binding domain; LBD: ligand binding domain
Fig. 4 shows (a) ligand independent transcription activity and (b) bicalutamide resistant transcription activity of the 4 kinds of ARaiv1 ORF clones. DHT: dihydrotestosterone; Bic: bicartamide
Fig. 5 shows expression of ARaiv1 mRNA in various prostate cancer cell lines.
Fig. 6 shows (a) knockdown efficiency of full-length AR and ARaiv1 by various siRNAs and (b) inhibition of C-terminally defective AR(ARΔC) production by ARaiv1 knockdown. NS: non-specific siRNA transfection group
Fig. 7 shows inhibition of JDCaP-hr cell growth by ARaiv1 knockdown. NS: non-specific siRNA transfection group
Fig. 8 shows inhibition of CWR22Rvl cell growth by ARaiv1 knockdown.
Fig. 9 shows expression of ARaiv1 and full-length AR in clinical prostate cancer samples. normal: normal prostate tissue, normal region: non-lesion site of prostate affected with cancer, NA: no staging information
Fig. 10 shows inhibition of transcriptional activation action of ARaiv1 by geldanamycin. Empty shows cells transfected with an empty vector without ARaiv1 gene.
Fig. 11 shows inhibition by geldanamycin of gene expression and protein production of ARaiv1 and full-length AR.
Fig. 12 shows inhibition of JDCaP-hr cell proliferation by geldanamycin.
Fig. 13 shows inhibition of transcription activity of ARaiv1 by pyrvinium pamoate.
Fig. 14 shows inhibition of JDCaP-hr cell growth by pyrvinium pamoate. PP: pyrvinium pamoate concentration

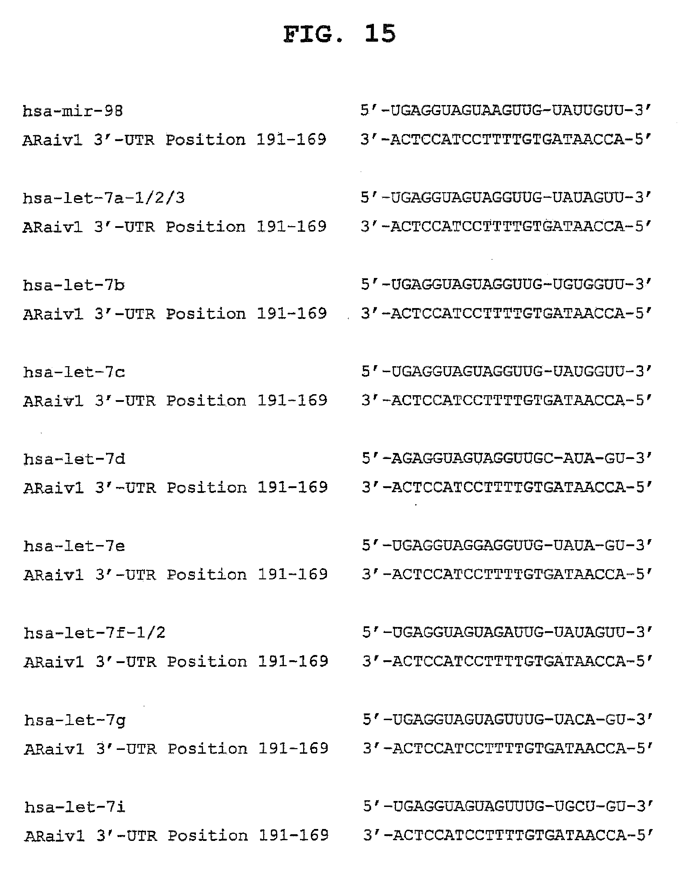

Fig. 15 shows miRNAs capable of binding to 3'-UTR sequence of ARaivl. The nucleotide sequence of each miRNA is described in the upper panel (5'→3'), and the nucleotide sequence (3'←5') of 169th - 191st from the 5'-end of 3'-UTR of ARaiv1 (ARaiv1 3'-UTR Position 191-169) to be the target is described in the lower panel.

### [Detailed Description of the Invention]

### (1) variant AR of the present invention

The variant AR (ARaiv) lacking a ligand binding domain in the present invention is a protein comprising a region at least necessary for retaining a transcriptional activation action in the amino acid sequence encoded by the first exon (5001 - 7731-position of NG_009014.1; 1 - 1616-position of SEQ ID NO: 15) of human AR gene (its nucleotide sequence is registered as NCBI RefSeq No. NG_009014.1 (GI:213385253)), and a region at least necessary for binding to a target gene in the amino acid sequence encoded by the first - third exons (the second and the third exon are the 104225 - 104376-position and the 146979 - 147095-position of NG_009014.1, respectively; the 1617 - 1768-position and the 1769 - 1885-position of SEQ ID NO: 15, respectively), and lacking a region necessary for binding to a ligand in the amino acid sequence encoded by the fourth - eighth exons (the 172371 - 172658-position, the 178447 - 178591-position, the 182802 - 182932-position, the 183796 - 183953-position and the 184655 - 185246-position of NG_009014.1, respectively; the 1886 - 2173-position, the 2174 - 2318-position, the 2319 - 2449-position, the 2450 - 2607-position and the 2608 - 2763-position of SEQ ID NO: 15, respectively). Preferably, ARaiv of the present invention includes a protein containing an amino acid sequence which is an amino acid sequence of full-length AR shown by SEQ ID NO: 16 wherein a C-terminal sequence shown by amino acid Nos. 625 - 920 is deleted and, instead, a C-terminal amino acid sequence shown by SEQ ID NO: 1 or 2 is added (i.e., amino acid sequence shown by SEQ ID NO: 4 (ARaiv1) or 6 (ARaiv2)), or a protein containing an amino acid sequence which is an amino acid sequence shown by SEQ ID NO: 4 or 6 wherein an amino acid sequence shown by amino acid Nos. 367 - 495 is entirely or partially deleted. In the present specification, the protein and peptide are described according to conventional peptide lettering, and the left end is N-terminal (amino terminal), and the right end is C-terminal (carboxyl terminal).

The amino acid sequence shown by the above-mentioned amino acid Nos. 367 - 495 corresponds to a region called transactivation unit 5 (TAU5) in the AR transcriptional activation domain (AF1), and is reported to inhibit androgen-dependent AR activity in androgen-dependent cancer cells, but potentiate androgen-independent AR activity in androgen-independent cancer cells (Cancer Res., 2007, 67: 10067-77). Even if TAU5 is completely deleted, a transcriptional activation action of AR is maintained at a level sufficient to promote growth of cancer cells. Specific examples of the TAU5-deleted ARaiv include a protein containing an amino acid sequence shown by SEQ ID NO: 8, 10 or 12 (ARaiv1Δ1, ARaiv1Δ2, ARaiv1Δ3, respectively) and the like.

The Gln repeat number of the polyglutamine region in the N-terminal sequence of AR shows individual difference (generally about 9-34 residues, repeat number in prostate cancer cells may decrease), and therefore, a protein containing an amino acid sequence which is the amino acid sequence shown by each of the above-mentioned SEQ ID NO, wherein the Gln repeat number of the polyglutamine region varies within a range generally observed, is also encompassed in the ARaiv of the present invention.

The ARaiv protein may be isolated and purified by a protein separation and purification technique known per se from human cell [e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding progenitor cell, stem cell or cancer cell thereof etc.] or any tissue in which these cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle (e.g., smooth muscle, skeletal muscle), lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue) etc.] and the like, preferably cancer cells of prostate, testis, mammary gland, ovary, placenta, uterus and the like, more preferably relapsed androgen-independent prostate cancer cells. When, for example, an ARaiv protein is used for screening for a prophylactic or therapeutic drug for cancer, and the like, it may be a protein produced by chemical synthesis or biochemical synthesis in a cell-free translation system, or a recombinant protein produced by a transformant transduced with a nucleic acid having a nucleotide sequence encoding any of the above-mentioned amino acid sequences.

The ARaiv in the present invention also encompasses a protein containing any of the above-mentioned amino acid sequences, polymorphic or natural allele variant found in human (said mutation does not influence AR function), and ortholog in a mammal other than human (e.g., mouse, rat, monkey, dog, cat, bovine, horse, swine, sheep, goat, rabbit and the like).

Preferably, the ARaiv in the present invention is a protein containing an amino acid sequence shown by SEQ ID NO: 6, 8 or 10 (i.e., ARaiv1Δ1, ARaiv1Δ2 or ARaiv1Δ3), or a protein containing an amino acid sequence shown by SEQ ID NO: 12 (i.e., ARaiv2). These ARaivs are novel variant ARs identified in the present invention.

### (2) use of variant AR of the present invention

### (2-1) screening for medicament candidate compound for disease

The present invention provides a method of screening for a substance for the prophylaxis and/or treatment of a disease involving variant AR, for example, variant AR highly expressing cancer, which uses, as an index, a function of the variant AR, particularly the above-mentioned novel ARaiv, namely, a transcription regulating action on an androgen responsive gene (e.g., transcriptional activation of prostate specific antigen (PSA) gene, TMPRSS2 gene and the like, or transcriptional repression of SERPIN B5 gene and the like), or a suppressive action on the expression of the variant AR genes.
As mentioned above, when expression and/or activity of ARaiv is inhibited, transcription regulation of an androgen responsive gene is suppressed, and AR signal is shut off to suppress cancer cell growth. Therefore, a compound that inhibits expression and/or activity of ARaiv can be used as a blocker of AR signal from ARaiv and as a prophylactic or therapeutic drug for cancer, particularly ARaiv highly expressing cancer (e.g., prostate cancer and the like), inter alia, relapsed androgen-independent prostate cancer.
Hence, an ARaiv protein or a cell that produces ARaiv can be used as a tool for screening for a substance for the prophylaxis or treatment of cancer, by using the expression level and/or activity (e.g., transcription regulation activity) of ARaiv as an index.

When a compound that inhibits the activity of ARaiv is screened for, the screening method includes
(1) contacting an ARaiv protein or a cell capable of producing ARaiv with a test compound, and
(2) measuring the activity of the protein,
(3) selecting a compound that suppresses the activity of the protein as compared to the activity of a control without contact with the test compound.
A cell capable of producing ARaiv can be contacted with a test compound by, for example, cultivating the cell in the presence of the test compound. In this case, the cell cultivated in the absence of the test compound is used as a control, and the activities of ARaiv under the both conditions are compared to evaluate the ARaiv activity suppressive effect of the test compound.
Examples of the activity of ARaiv include androgen responsive gene transcription regulation activity, ARaiv protein nuclear translocation activity, and androgen response sequence (ARE)-binding activity of the gene.

The cell capable of producing ARaiv to be used for the above-mentioned screening method is not particularly limited as long as it is a human or other mammalian cell that inherently expresses ARaiv or a biological sample containing same (e.g., blood, tissue, organ etc.). Preferred are androgen independent cancer cell line or tumor line (e.g., cell line or tumor line of AIPC prostate cancer) and the like. When blood, tissue, organ and the like derived from a non-human animal are used, they may be isolated from the living organism and cultured, or a test compound may be administered to the living organism itself, and a biological sample may be isolated after lapse of a given time.
Examples of the cell capable of producing ARaiv include various transformants produced by a conventionally known genetic engineering technique. As a host, animal cells such as H4IIE-C3 cell, HepG2 cell, HEK293 cell, COS7 cell, CHO cell and the like are preferably used. As a host, prostate cancer cells that do not inherently express ARaiv protein, for example, LNCaP-FGC cell, PC-3 cell, DU-145 cell and the like, can also be used.

Specifically, the cell can be prepared by linking a DNA encoding ARaiv (i.e., DNA encoding a protein containing the amino acid sequence shown by SEQ ID NO: 4, 6, 8, 10 or 12, particularly SEQ ID NO: 6, 8, 10 or 12, specifically, DNA containing a nucleotide sequence shown by SEQ ID NO: 3, 5, 7, 9 or 11, particularly SEQ ID NO: 5, 7, 9 or 11 (CDS sequence), or a nucleotide sequence that hybridizes to a complementary chain of said nucleotide sequence under high stringent conditions, and encodes a polypeptide having an androgen responsive gene transcription regulating action equivalent to that of a protein comprising an amino acid sequence shown by SEQ ID NO: 4, 6, 8, 10 or 12, particularly SEQ ID NO: 6, 8, 10 or 12) to the downstream of a promoter in a suitable expression vector and transducing the vector into a host animal cell.
DNA encoding ARaiv can be cloned, for example, by synthesizing a suitable oligonucleotide as a probe or primer based on the nucleotide sequence shown by SEQ ID NO: 3 or 5, and by using a hybridization method or PCR method, from a cDNA or cDNA library derived from a cell or tissue that produces the aforementioned ARaiv. The hybridization can be performed, for example, by the method described in Molecular Cloning, 2nd ed. (J. Sambrook et al., Cold Spring Harbor Lab. Press, 1989) and the like. When a commercially available library is used, the hybridization can be performed according to the instructions of the manufacturer's protocol attached to the library.

The nucleotide sequence of the DNA can be converted according to a method known per se, such as the ODA-LA PCR method, the Gapped duplex method, or the Kunkel method, or a method based thereon, using a commonly known kit, for example, Mutan^{™}-super Express Km (TAKARA SHUZO CO. LTD.), Mutan^{™}-K (TAKARA SHUZO CO. LTD.) and the like. For example, a part or the entirety of the region that does not cause disappearance of the function of ARaiv even if deleted, such as TAU5 region and the like, may be deleted from a cloned DNA or the entirety or a part of the deleted region may be inserted into or added to a DNA encoding mutant ARaiv lacking a part of the region.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added by using a suitable synthetic DNA adaptor.

As an expression vector, animal cell expression plasmid (e.g., pA1-11, pXT1, pRc/CMV, pRc/RSV, pcDNAI/Neo); bacteriophage such as λphage and the like; animal virus vectors such as retrovirus, vaccinia virus, adenovirus, lentivirus and the like; and the like are used. The promoter may be any promoter, as long as it is appropriate for the host used to express the gene. For example, SRα promoter, SV40 promoter, LTR promoter, CMV (cytomegalovirus) promoter, RSV (Rous sarcoma virus) promoter, MoMuLV (Moloney murine leukaemia virus) LTR, HSV-TK (simple herpes virus thymidine kinase) promoter and the like are used. Of these, CMV promoter, SRα promoter and the like are preferred.
Useful expression vectors include, in addition to the above, expression vectors that optionally comprises an enhancer, a splicing signal, a polyA addition signal, a selection marker, an SV40 replication origin (hereinafter also abbreviated as SV40 ori), and the like. As examples of the selection markers, the dihydrofolate reductase gene (hereinafter also abbreviated as dhfr) [methotrexate (MTX) resistance], the ampicillin resistance gene (hereinafter also abbreviated as amp^{r}), the neomycin resistance gene (hereinafter also abbreviated as neo^{r}, G418 resistance), and the like can be mentioned. In particular, when a dhfr gene defective Chinese hamster cell is used and the dhfr gene is used as the selection marker, a target gene can also be selected using a thymidine-free medium.
When a nuclear translocation activity is measured as the ARaiv activity, the vector can also be designed such that ARaiv is expressed as a fusion protein with a reporter protein, so that nuclear translocation of the ARaiv protein can be easily detected. Examples of the reporter protein include fluorescence proteins such as GFP, YFP, BFP, RFP and the like, and the like.

An ARaiv expressing cell can be produced by transforming a host with an expression vector containing the above-mentioned DNA encoding ARaiv.
As the host, mammalian cells, for example, HepG2 cell, HEK293 cell, HeLa cell, LNCaP-FGC cell, PC-3 cell, DU-145 cell, human FL cell, monkey COS-7 cell, monkey Vero cell, Chinese hamster ovary cell (hereinafter to be abbreviated as CHO cell), dhfrgene defective CHO cell (hereinafter to be abbreviated as CHO(dhfr⁻) cell), mouse L cell, mouse AtT-20 cell, mouse myeloma cell, rat H4IIE-C3 cell, rat GH3 cell and the like can be used.

Transformation can be performed by a calcium phosphate coprecipitation method, a PEG method, an electroporation method, a microinjection method, a lipofection method and the like. For example, the method described in Saibo Kogaku, extra issue 8, Shin Saibo Kogaku Jikken Protocol, 263-267 (1995), published by Shujunsha, or Virology, 52, 456 (1973) can be used.

The transformed cell obtained as mentioned above, a mammalian cell inherently having an ability to produce ARaiv, and a tissue or organ containing the cell can be cultivated in a medium, for example, minimum essential medium (MEM) containing about 5 - 20% fetal bovine serum [Science, vol. 122, 501(1952)], Dulbecco's modified Eagle medium (DMEM) [Virology, vol. 8, 396(1959)], RPMI1640 medium [The Journal of the American Medical Association, vol. 1995, 19(1967)], 199 medium [Proceeding of the Society for the Biological Medicine, vol. 73, 1(1950)] and the like. The medium preferably has a pH of about 6-8. Culture is performed generally at about 30 - 40°C, and aeration and stirring may be added as necessary.

Examples of the test compound include protein, peptide, antibody, nonpeptidic compound, synthetic compound, fermentation product, cell extract, plant extract, animal tissue extract, plasma and the like, which may be novel or known.

A test compound can be contacted with the above-mentioned cell by, for example, adding the test compound to the above-mentioned medium and various buffers (e.g., HEPES buffer, phosphate buffer, phosphate buffered saline, Tris-HCl buffer, borate buffer, acetate buffer and the like), and incubating the cell for a given time. While the concentration of the test compound to be added varies depending on the kind of the compound (solubility, toxicity etc.), it is, for example, appropriately determined within the range of about 0.1 nM - about 10000 nM, preferably about 1 nM - about 1000 nM. Examples of the incubation time include about 10 min - about 24 hr.

When an ARaiv-producing cell is provided in the form of a non-human individual mammal, the condition of the individual animal is not particularly limited. For example, it may be a model animal such as a cancer bearing mouse and the like. Such cancer bearing mouse can be produced by a conventional method. Preferably, a transgenic non-human mammal transduced with a nucleic acid containing a protein coding sequence (CDS) of ARaiv, particularly, a nucleic acid containing a CDS sequence of ARaiv2 or any of ARaiv1Δ1 - 3 (i.e., CDS sequence encoding a protein containing the amino acid sequence shown by SEQ ID NO: 6, 8, 10 or 12, specifically, a CDS sequence in a nucleotide sequence shown by SEQ ID NO: 5, 7, 9 or 11), in an expressible form can be used. Such transgenic animal can be produced by a conventional method.
While the rearing conditions of the animal to be used are not particularly limited, the animal is preferably reared in an environment of SPF grade or above. A test compound is contacted with the cell by the administration of the test compound to the animal individual. While the administration route is not particularly limited, for example, intravenous administration, intraarterial administration, subcutaneous administration, intradermal administration, intraperitoneal administration, oral administration, tracheobronchial administration, rectal administration and the like can be mentioned. While the dose is not particularly limited, either, for example, a dose of about 0.5 - 20 mg/kg can be administered 1 - 5 times, preferably 1 - 3 times, per day for 1 - 14 days.

The ARaiv activity can be measured in the above-mentioned screening method by, for example, using the expression level of an androgen responsive gene (e.g., PSA gene, TMPRSS2 gene etc.) as an index. The expression level of the gene may be of an RNA level by using RT-PCR or Northernblot analysis, or of a protein level by using an antibody to a translation product thereof. The ARaiv activity is more preferably evaluated by introducing a DNA encoding a reporter protein under the control of an androgen responsive promoter (e.g., a promoter having a native androgen response sequence (ARE) such as PSA promoter and the like, a chimera promoter wherein ARE sequence is combined with an animal cell promoter such as SRα promoter, SV40 promoter, LTR promoter, CMV promoter, HSV-TK promoter and the like, and the like) and examining the expression of the reporter protein. The "PSA promoter" here means, in the 5' upstream sequence of PSA gene, a region containing at least from the essential promoter region such as TATA box and the like to an ARE corresponding sequence in the upstream (in human PSA gene, AGAACAGCAAGTGCT; SEQ ID NO: 17), preferably, a region further containing up to 35 base pairs known as an androgen response region (ARR) in the further upstream [for human PSA gene, GTGGTGCAGGGATCAGGGAGTCTCACAATCTCCTG; SEQ ID NO: 18 (J. Biol. Chem., 271(11): 6379-6388, 1996)], more preferably a region containing about 6000 base pairs in the upstream of the transcription start point, and still more preferably a region containing about 600 base pairs in the upstream of the transcription start point. PSA promoter is useful since it reflects well the behavior of ARaiv in actual prostate cancer cell. When the activity of the promoter needs to be enhanced, plural (preferably 2 - 5, more preferably 2 or 3) promoters are preferably linked in tandem. As the reporter protein, luciferase, green fluorescence protein (GFP), β-galactosidase, chloramphenicol acetyltransferase, peroxidase, alkaline phosphatase and the like can be used. A DNA encoding a reporter protein can be inserted into the aforementioned vector for animal cell expression, and transduced according to the aforementioned gene transfer method.
When a cell that produces ARaiv is provided in the form of a non-human individual mammal, the activity of ARaiv can also be evaluated by using, as an index, the growth, differentiation, cell death or canceration, or cancer growth of the tissue (e.g., prostate tissue) collected from the animal. When such index is used, this screening system using ARaiv-producing non-human individual mammal is particularly useful not only for screening for a novel candidate substance for a prophylactic or therapeutic drug for cancer, but also an efficacy evaluation system for existing prophylactic or therapeutic drugs for cancer.

Examples of the test sample for activity measurement include:
a culture extract when the ARaiv-producing cell is provided in the form of a cell culture, tissue or organ culture; when the cell is provided as a non-human individual mammal, cell, tissue, organ extract separated from the individual, for example, homogenate of prostate, testis, mammary gland or tissue sections and the like; and the like.

When an isolated ARaiv protein is used in the above-mentioned screening method, a variant AR protein containing the amino acid sequence shown by SEQ ID NO: 6, 8, 10 or 12 is preferably used as ARaiv. The protein may be isolated and purified from a cell or tissue that produces the protein, or chemically synthesized, or produced as a recombinant protein by genetic engineering.
The ARaiv can be contacted with a test compound in, for example, water or a suitable buffer. While the concentration of the test compound to be added varies depending on the kind of the compound (solubility, toxicity etc.), it is, for example, appropriately determined within the range of about 0.1 nM - about 10000 nM, preferably 1 nM - 1000 nM. Examples of the incubation time include about 10 min - about 24 hr.
The activity of ARaiv can be measured by, for example, a gel shift assay using an oligonucleotide containing a consensus ARE sequence (e.g., AGAACAnnnTGTTCT; SEQ ID NO: 32) of an androgen responsive gene (e.g., PSA gene, TMPRSS2 gene etc.) as a probe.

When, for example, in the above-mentioned screening method, the activity of ARaiv in the presence of a test compound is inhibited by not less than about 20%, preferably not less than 30%, more preferably not less than about 50%, as compared to the activity in the absence of the test compound, the test compound can be selected as a candidate for an ARaiv activity inhibitory substance, therefore, a substance for the prophylaxis or treatment of cancer, particularly ARaiv highly expressing cancer (e.g., prostate cancer etc.), inter alia, relapsed androgen-independent prostate cancer.

The present invention also provides a method of screening for a substance for the prophylaxis or treatment of cancer, comprising comparing expression of a variant AR in a cell having an ability to produce ARaiv, particularly novel ARaiv (variant AR protein containing the amino acid sequence shown by SEQ ID NO: 6, 8, 10 or 12), between in the presence and absence of a test compound. The cell to be used for this method, the kind of the test compound, the manner of contact between the test compound and the cell and the like are the same as in the above-mentioned method using the ARaiv activity as an index.

The expression level of ARaiv can be measured at the RNA level by detecting ARaiv mRNA by using a nucleic acid which can be hybridized with the aforementioned DNA encoding ARaiv under high stringent conditions, i.e., a nucleic acid which can be hybridized with a nucleotide sequence complementary to a nucleotide sequence shown by SEQ ID NO: 3, 5, 7, 9 or 11 under high stringent conditions. Alternatively, the expression level can also be measured at the protein level, by detecting ARaiv protein by using an antibody to AR or the below-mentioned antibody to ARaiv. When a cell inherently expressing ARaiv together with full-length AR, or a cell obtained by transducing a nucleic acid encoding ARaiv into a cell expressing full-length AR alone is used, mRNA or protein of full-length AR, and mRNA or protein of ARaiv need to be measured in a distinguished manner. A nucleic acid and an antibody capable of recognizing a region specific to ARaiv, which are used in the below-mentioned "diagnostic reagent of the present invention" can be used therefor. When measured at the protein level, ARaiv and AR can be measured in a distinguished manner based on the molecular weight of the band obtained by Western Blot using an antibody to AR.

When, for example, in the above-mentioned screening method, the expression level (mRNA amount or protein amount) of ARaiv in the presence of a test compound is inhibited by not less than about 20%, preferably not less than about 30%, more preferably not less than about 50%, as compared to the activity in the absence of the test compound, the test compound can be selected as a candidate for an ARaiv expression inhibitory substance, therefore, a substance for the prophylaxis or treatment of cancer, particularly ARaiv highly expressing cancer (e.g., prostate cancer etc.), inter alia, relapsed androgen-independent prostate cancer.

### (2-2) variant AR as drug discovery target for nucleic acid medicament

ARaiv is expressed in a subset of relapsed androgen-independent prostate cancer, and is a ligand (androgen, anti-androgenic agent) independent constitutively active molecule. Therefore, AR signal from this variant is considered to be at least one cause of the growth of androgen independent cancer cells in ARaiv expressing patients. Accordingly, a substance that inhibits the function of ARaiv is effective for the prophylaxis and/or treatment of ARaiv expressing cancer.

The "substance that inhibits function of variant AR(ARaiv) lacking a ligand binding domain" is largely divided into a substance that inhibits expression of ARaiv (expression inhibitor) and a substance that inhibits activity of ARaiv (activity inhibitor). The ARaiv expression inhibitor may act at any level from ARaiv transcription level, post-transcriptional regulation level, translation into protein level, post-translational modification level, ARaiv protein intracellular transport level, ARaiv protein degradation and the like. Therefore, examples of the ARaiv expression inhibitor include a substance inhibiting transcription of ARaiv, a substance inhibiting the processing from early transcription products to mRNA, a substance inhibiting transport of mRNA to cytoplasm, a substance promoting degradation of mRNA, a substance inhibiting translation from mRNA to protein, a substance inhibiting formation of the correct structure of ARaiv polypeptide, a substance inhibiting posttranslational modification of ARaiv polypeptide and the like. The substances acting at any of those levels are preferably used, but in the sense of directly inhibiting the production of ARaiv protein, a substance inhibiting translation from mRNA to protein is preferably employed. Moreover, a substance inhibiting selective splicing that produces ARaiv mRNA and a substance selectively promoting degradation of ARaiv mRNA are also preferable.

As a substance which can specifically inhibit translation from mRNA of ARaiv to protein, a nucleic acid comprising a nucleotide sequence complementary or substantially complementary to the mRNA nucleotide sequence, or a part thereof, is preferably mentioned.
The "nucleotide sequence substantially complementary to the nucleotide sequence of ARaiv mRNA" refers to the nucleotide sequence having complementarity in an extent of inhibiting the translation by binding to a target sequence of the mRNA, under physiological conditions in the ARaiv expressing cell of a mammal. Specifically, it is, for example, the nucleotide sequence having not less than about 80% homology, preferably not less than about 90% homology, more preferably not less than about 95% homology, and particularly preferably not less than about 97% homology, to the nucleotide sequence completely complimentary to the mRNA nucleotide sequence (that is, nucleotide sequence of mRNA complementary strand) in the overlapping region.
The "homology of the nucleotide sequence" in the present invention can be calculated under the following conditions (an expectation value = 10; gaps are allowed; filtering = ON, match score=l; mismatch score=-3) using a homology scoring algorithm NCBI BLAST (National Center for Biotechnology Information Basic Local Alignment Search Tool). As other algorithm for determining the homology of nucleotide sequence, for example, the algorithm described in Karlin et al., Proc. Natl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.

More specifically, as a nucleotide sequence complementary or substantially complementary to the nucleotide sequence of ARaiv mRNA, a nucleotide sequence complementary or substantially complementary to (a) a nucleotide sequence encoding a protein containing the amino acid sequence shown by SEQ ID NO: 4, 6, 8, 10 or 12, particularly SEQ ID NO: 6, 8, 10 or 12, more specifically, a nucleotide sequence shown by SEQ ID NO: 3, 5, 7, 9 or 11, particularly SEQ ID NO: 5, 7, 9 or 11, or (b) a nucleotide sequence that hybridizes to a complementary chain of said nucleotide sequence under high stringent conditions, and that encodes a polypeptide having an activity (e.g., androgen responsive gene transcription regulating action) equivalent to that of a protein comprising an amino acid sequence shown by SEQ ID NO: 4, 6, 8, 10 or 12, particularly SEQ ID NO: 6, 8, 10 or 12 can be mentioned.
"High-stringent conditions" refer to, for example, conditions involving a sodium salt concentration of about 19 to about 40 mM, preferably about 19 to about 20 mM, and a temperature of about 50 to about 70°C, preferably about 60 to about 65°C. In particular, a case wherein the sodium salt concentration is about 19 mM and the temperature is about 65°C is preferred.

ARaiv mRNA is preferably human ARaiv2 mRNA containing a nucleotide sequence shown by SEQ ID NO: 5, or mRNA of human ARaiv1Δ1, ARaiv1Δ2 or ARaiv1Δ3 containing a nucleotide sequence shown by SEQ ID NO: 7, 9 or 11, respectively.

The "part of a nucleotide sequence complementary or substantially complementary to a nucleotide sequence of ARaiv mRNA" is not particularly limited as regards the length and position thereof as long as it can specifically bind to ARaiv mRNA and inhibit translation of protein from mRNA. From the aspect of sequence specificity, however, it contains not less than 10 bases, preferably not less than about 15 bases, more preferably not less than about 20 bases, of a region complementary or substantially complementary to a target sequence. In consideration of a simple synthesis, antigenic problem, and transitional problem in cell, oligonucleotide comprising about 10 to 40 bases, particularly about 15 to 30 bases is preferable. In addition, since ARaiv and normal splice variant (full-length AR) commonly have the 1st - 3rd exons, ARaiv desirably contains the whole 4th exon of ARaiv (nucleotide sequence shown by SEQ ID NO: 13 (the 155642 - 157044-position of NCBI RefSeq No. NG_009014.1 (GI:213385253)) for ARaivl, nucleotide sequence shown by SEQ ID NO: 14 (the 147929 - 149083-position of NG_009014.1) for ARaiv2) or a part thereof in at least a part of a target sequence, to specifically inhibit translation from ARaiv mRNA. However, since cancer cells express not only ARaiv mRNA but also mRNA encoding full-length AR, it is also preferable to set a region in the 1st - 3rd exons as a target sequence, to shut off signaling from both ARs.
Since expression of full-length AR corresponding to TAU5-deleted ARaiv has not been confirmed, translation from the TAU5-deleted ARaiv mRNA may also be specifically inhibited by setting the linking site of 5'-side and 3'-side flaking sequences of a deleted region resulting from the deletion of the whole TAU5 region or a part thereof as a target sequence.

Specifically, as a nucleic acid comprising a nucleotide sequence complementary or substantially complementary to a nucleotide sequence of ARaiv mRNA, or a part thereof, for example, the nucleic acid of any of the following (a) - (e) is preferable.
(a) antisense nucleic acid for ARaiv mRNA
(b) siRNA for ARaiv mRNA
(c) nucleic acid capable of generating siRNA for ARaiv mRNA
(d) miRNA for ARaiv mRNA
(e) nucleic acid capable of generating miRNA for ARaiv mRNA

### (a) antisense nucleic acid for ARaiv mRNA

The "antisense nucleic acid for ARaiv mRNA" in the invention is a nucleic acid comprising a nucleotide sequence complimentary or substantially complimentary to the nucleotide sequence of mRNA or a part thereof, which has a function of inhibiting the protein synthesis by binding specifically with the target mRNA to form a stable duplex.
As the antisense nucleic acid, a polydeoxyribonucleotide containing 2-deoxy-D-ribose, a polyribonucleotide containing D-ribose, another type of polynucleotide that is an N-glycoside of the purine or pyrimidine base, or another polymer having a non-nucleotide backbone (e.g., commercially available protein nucleic acids and synthetic sequence specific nucleic acid polymers) or another polymer having a special bond (however, this polymer contains a nucleotide having a configuration that allows base pairing or base attachment as found in DNA and RNA) and the like can be mentioned. These may be double-stranded DNAs, single-stranded DNAs, double-stranded RNAs or single-stranded RNAs, or DNA:RNA hybrids, and may also be non-modified polynucleotides (or non-modified oligonucleotides), those having a known modification added thereto, for example, those with a label known in the relevant field, those with a cap, those methylated, those having 1 or more naturally occurring nucleotides substituted by analogues, those having an intramolecular nucleotide modification, for example, those having a non-charge bond (e.g., methylphosphonate, phosphotriester, phosphoramidate, carbamate and the like), those having a charged bond or a sulfur-containing bond (e.g., phosphorothioate, phosphorodithioate and the like), for example, those having a side chain group of a protein (nuclease, nuclease inhibitor, toxin, antibody, signal peptide, poly-L-lysine and the like), those having a side chain group of a sugar (e.g., monosaccharide and the like) and the like, those having an intercalating compound (e.g., acridine, soralen and the like), those containing a chelate compound (e.g., metals, radioactive metals, boron, oxidizing metals and the like), or those containing an alkylating agent, those having a modified bond (e.g., α anomer type nucleic acid and the like). Here, "nucleoside", "nucleotide" and "nucleic acid" may include not only those containing the purine and pyrimidine bases, but also those containing another modified heterocycle type base. These modified products may contain a methylated purine and pyrimidine, an acylated purine and pyrimidine, or another heterocycle. The modified nucleoside and the modified nucleotide may also have their sugar portion modified by, for example, substitution of 1 or more hydroxyl groups by a halogen, an aliphatic group and the like, or conversion to a functional group such as an ether or an amine.

As described above, the antisense nucleic acid may be DNA or RNA, or DNA/RNA chimera. When the antisense nucleic acid is DNA, the RNA:DNA hybrid formed by the target RNA and the antisense DNA is recognized by endogenous RNase H, thereby directing the selective degradation of the target RNA.
Further, when not only ARaiv but also full-length AR are also the targets, the antisense nucleic acid may be a nucleic acid that inhibits the translation into a protein by hybridizing with mRNA of ARaiv, and it may as well as be the nucleic acid capable of forming a triplex by binding with the AR gene which is the double-stranded DNA and inhibiting the transcription into RNA (anti-gene).

The nucleotide molecule constituting the antisense nucleic acid may be natural DNA or RNA, but may also include various chemical modifications in order to improve the stability (chemical and/or against enzyme) and specific activity (affinity with RNA). For example, to prevent digestion with a hydrolase such as nuclease, etc., the phosphate residue (phosphate) of each nucleotide that constitutes the antisense nucleic acid may be substituted with chemically modified phosphate residues, e.g., phosphorothioate (PS), methyl phosphonate, phosphorodithionate, etc. Also, a hydroxyl group at the 2' position of the sugar (ribose) in each nucleotide may be replaced by -OR (R represents, e.g., CH₃ (2'-O-Me), CH₂CH₂OCH₃ (2'-O-MOE), CH₂CH₂NHC(NH)NH₂, CH₂CONHCH₃, CH₂CH₂CN, etc.). Further, the base part (pyrimidine, purine) may be chemically modified, for example by the introduction of a methyl group or a cationic functional group to 5-position of the pyrimidine base or the replacement of a C2 carbonyl group with thiocarbonyl, etc.

The conformation of sugar in RNA is dominant in two of C2'-endo (S-form) and C3'-endo (N-form) and exists in equilibrium of those two in single-stranded RNA, but restricted in N-form in the case of a double strand. Accordingly, in order to provide a strong binding ability to the target RNA, BNA(LNA) (Imanishi, T. et al., Chem. Commun., 1653-9, 2002; Jepsen, J.S. et al., Oligonucleotides, 14, 130-46, 2004) and ENA (Morita, K. et al., Nucleosides Nucleotides Nucleic Acids, 22, 1619-21, 2003), which are RNA derivatives of which the sugar conformation is restricted into N-form by linking 2'-oxygen with 4'-carbon, are preferably employed.

The antisense oligonucleotide of the invention can be prepared by determining a target sequence of mRNA on the basis of a cDNA sequence information of ARaiv, and synthesizing its complementary sequence with the use of a commercially available DNA/RNA automatic synthesizer (Applied Biosytem company, Beckman company, etc.). In addition, the aforementioned antisense nucleic acids including various modifications can be chemically synthesized by a technique known per se.

### (b) siRNA for ARaiv mRNA

In the present specification, a double-stranded RNA comprising oligoRNA complementary to ARaiv mRNA and a strand complementary to the oligoRNA, i.e., siRNA, is also defined to be included in a nucleic acid comprising a nucleotide sequence complementary or substantially complementary to a nucleotide sequence of ARaiv mRNA or a part thereof. The phenomenon of so-called RNA interference (RNAi) in which mRNA complementary to the RNA introduced is degraded by introducing short double-stranded RNA into a cell, is known to occur in nematode, an insect, plant, etc., but after it is confirmed that the phenomenon also occurs in animal cells [Nature, 411 (6836): 494-498 (2001)], it is widely used as an alternative technology of ribozyme. siRNA can be appropriately designed on the basis of nucleotide sequence information of target mRNA by using a commercially available software (e.g., RNAi Designer; Invitrogen corp.). In more specific, siRNAs used in EXAMPLES shown later are preferably exemplified as siRNA for ARaiv of the invention, but not limited by those.

The ribonucleoside molecule constituting siRNA may also be modified in the same manner as in the antisense nucleic acid described above so as to improve the stability and specific activity. However, in the case of siRNA, introduction of minimal modified nucleoside allowing the RISC complex to function is necessary, since substitution of all ribonucleoside molecules in natural RNA by modified type may result in the loss of RNAi activity.

siRNA can be prepared according a process comprising synthesizing a sense strand and an antisense strand of target sequence on mRNA each with the DNA/RNA automatic synthesizer, denaturing in a suitable annealing-buffer solution at about 90 to 95°C for about 1 minute, and annealing at about 30 to 70°C for about 1 to 8 hours. In addition, siRNA can also be prepared by synthesizing short hairpin RNA (shRNA) which is the precursor of siRNA and cleaving the shRNA with the use of a dicer.

### (c) Nucleic acid capable of producing siRNA for ARaiv mRNA

In the present specification, a nucleic acid designed to be able to generate siRNA for the above-mentioned ARaiv mRNA in vivo is also defined to be included in a nucleic acid comprising a nucleotide sequence complementary or substantially complementary to a nucleotide sequence of ARaiv mRNA, or a part thereof. Such nucleic acid can be exemplified by aforementioned shRNA, an expression vector constructed in a manner to express the shRNA, or the like. The shRNA can be prepared by designing oligoRNA comprising a nucleotide sequence in which a sense strand and an antisense strand of the target sequence on mRNA are linked by a spacer sequence of a suitable length to form a loop-structure (for example, about 15 to 25 bases) intervened between the strands, and synthesizing the same by a DNA/RNA automatic synthesizer. The expression vector comprising a shRNA expression cassette can be prepared by first preparing a double-stranded DNA encoding the aforementioned shRNA by a conventional method and then inserting into a suitable expression vector. As the shRNA expression vector, those having Pol III promoter such as U6 or H1 can be used. In this case, shRNA transcribed in an animal cell in which the expression vector had been introduced forms a loop by itself, and then is processed by an endogeneous enzyme dicer or the like to form mature siRNA.

### (d) miRNA for ARaiv mRNA

In the present specification, an endogenous single strand RNA having a short nucleotide sequence complementary to 3'-untranslated region of ARaiv mRNA, i.e., miRNA, is also defined to be included in a nucleic acid comprising a nucleotide sequence complementary or substantially complementary to a nucleotide sequence of ARaiv mRNA or a part thereof. More specifically, preferable examples of the miRNA for ARaiv of the present invention include, but are not limited to, hsa-mir-98, hsa-let-7a-1/2/3, hsa-let-7b, hsa-let-7c, hsa-let-7d, hsa-let-7e, hsa-let-7f-1/2, hsa-let-7g, hsa-let-7i, hsa-miR-125a-5p, hsa-miR-134, hsa-miR-149^{*}, hsa-miR-491-5p, hsa-miR-574-5p and the like.

### (e) Nucleic acid capable of generating miRNA for ARaiv mRNA

In the present specification, a nucleic acid designed to be able to generate miRNA for the above-mentioned ARaiv mRNA in vivo (pre-miRNA, pri-miRNA etc.) is also defined to be included in a nucleic acid comprising a nucleotide sequence complementary or substantially complementary to a nucleotide sequence of ARaiv mRNA, or a part thereof.

Other preferred example of the nucleic acid comprising a nucleotide sequence complimentary or substantially complimentary to the nucleotide sequence of ARaiv mRNA or a part of the nucleotide sequence includes a ribozyme capable of specifically cleaving the internal coding region of the mRNA. The "ribozyme" is narrowly-defined as RNA having enzymatic activity for cleaving nucleic acid, but the present specification also includes DNA as long as there is a sequence specific enzymatic activity for cleaving nucleic acid. Ribozyme with mostly high-generality includes self-splicing RNA which can be found in infectious RNA such as viroid, a virusoid, etc., and hammer-head type or hairpin type are known. The hammer-head type exhibits enzymatic activity by about 40 bases, and it is possible to specifically cleave only the target mRNA by arranging several bases (about 10 bases in total) of both ends adjacent to the part having a hammer-head structure, so as to be complimentary to the desired cleavage site in mRNA. This type of ribozyme has a further advantage that genomic DNA is never targeted as its substrate is only RNA. When ARaiv mRNA forms itself a double-stranded structure, the target sequence can be formed into a single-strand by using hybrid ribozyme coupled with RNA motif derived from viral nucleic acid which can specifically bind to RNA helicase [Proc. Natl. Acad. Sci. USA, 98 (10): 5572-5577 (2001)]. Also, in a case where ribozyme is used in the form of an expression vector having DNA which encodes the ribozyme, the ribozyme can be hybrid ribozyme further coupled with the sequence of modified tRNA so as to promote transport to cytoplasm of a transcription product [Nucleic Acids Res., 29 (13): 2780-2788 (2001)].

A nucleic acid comprising a nucleotide sequence complementary or substantially complementary to a nucleotide sequence of ARaiv mRNA, or a part thereof can be supplied in a special form like liposome or microspheres, or can be given in an adduct form with a polycation like polylysine, which acts to neutralize the charge of the phosphate backbone, or a hydrophobic compound like a lipid that enhances the interaction with cellular membrane or increases nucleic acid uptake (e.g., phospholipid, cholesterol and the like). As lipids preferred for addition, cholesterol and derivatives thereof (e.g., cholesterylchloroformate, cholic acid and the like) can be mentioned. These can be attached to the 3' end or the 5' end of nucleic acid, and can be attached via a base, a sugar or an intramolecular nucleoside bond. As other groups, a capping group specifically arranged at the 3' end or 5' end of nucleic acid to prevent degradation by a nuclease such as exonuclease or RNase can be mentioned. As such a capping group, hydroxyl group protecting groups known in the relevant field, including glycols such as polyethylene glycol and tetraethylene glycol can be mentioned, which, however, are not to be construed as limitative.

### [Medicament containing antisense nucleic acid, siRNA, precursor nucleic acid thereof, miRNA, precursor nucleic acid thereof]

An antisense nucleic acid that complementarily binds to an ARaiv transcription product and can suppress translation of protein from the transcription product, siRNA (or ribozyme) capable of cleaving an ARaiv transcription product by using a homologous (or complementary) nucleotide sequence in the ARaiv transcription product as a target, shRNA which is a precursor of the siRNA, miRNA that complementarily binds to an ARaiv transcription product and suppresses translation of protein from the transcription product or promotes degradation of the transcription product, and pre-miRNA which is a precursor of the miRNA and the like (hereinafter sometimes to be comprehensively referred to as "the nucleic acid of the present invention") can be administered as they are in the form of liquid preparations, or as pharmaceutical compositions in suitable dosage forms to human or non-human mammals (e.g., mouse, rats, rabbits, sheep, swine, bovine, feline, canine, simian, etc.) orally or parenterally (e.g., intravascularly, subcutaneously, etc.).

When the nucleic acid of the present invention is used as an agent for the prophylaxis or treatment of relapsed prostate cancer, it can be prepared into a pharmaceutical preparation according to a method known per se and administered. That is, the nucleic acid of the present invention can be formulated singly, or formulated according to a conventional means after operably inserting into a suitable expression vector for mammalian cell such as retrovirus vector, adenovirus vector, adenovirus associated virus vector, lentivirus vector and the like. The nucleic acid can also be administered as it stands, or can be administered together with an auxiliary agent to assist its uptake, by a gene gun or through a catheter such as a catheter with a hydrogel. Alternatively, the antisense nucleic acid may be prepared into an aerosol, which is topically administered into the trachea as an inhaler.
Further for the purposes of improving pharmacokinetics, prolonging a half-life, and improving intracellular uptake efficiency, the nucleic acid described above may be prepared into a pharmaceutical preparation (injectable preparation) alone or together with a carrier such as liposome, etc. and the preparations may be administered intravenously, subcutaneously, or the like.

The pharmaceutical composition containing the nucleic acid of the present invention may contain the nucleic acid of the present invention and a pharmacologically acceptable carrier, a diluent or an excipient. Such a pharmaceutical composition is provided in the form of a pharmaceutical preparation suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations can be prepared by methods publicly known. For example, the injectable preparations may be prepared by dissolving, suspending or emulsifying the nucleic acid of the present invention described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc.
As the oily medium, there are employed, e.g., sesame oil, soybean oil, etc., which may be used in combination with a solubilizing agent such as benzyl benzoate, benzyl alcohol, etc. The injectable thus prepared is usually filled in an appropriate ampoule. The suppository used for rectal administration may be prepared by blending the nucleic acid described above with a conventional base for suppository.

The composition for oral administration includes solid or liquid preparations, particularly, tablets (including dragees and film-coated tablets), pills, granules, powdery preparations, capsules (including soft capsules), syrup, emulsions, suspensions, etc. Such a composition is manufactured by publicly known methods and contains a carrier, a diluent or an excipient conventionally used in the field of pharmaceutical preparations. Examples of the carrier or excipient for tablets are lactose, starch, sucrose and magnesium stearate.

Advantageously, the pharmaceutical compositions for parenteral or oral use described above are prepared into pharmaceutical preparations with a unit dose formed to fit a dose of the active components. Such unit dose preparations include, for example, tablets, pills, capsules, injections (ampoules) and suppositories. The nucleic acid of the present invention is generally contained in 5 to 500 mg per dosage unit form, in 5 to 100 mg especially in the form of injection, and in 10 to 250 mg for the other forms.

The dose of the above-mentioned medicament comprising the nucleic acid of the present invention varies depending on the subject of administration, target disease, symptom, administration route and the like. When, for example, the medicament is used for the treatment or prophylaxis of prostate cancer in adult, the nucleic acid of the present invention is conveniently administered at, per one dose, generally about 0.01 - 20 mg/kg body weight, preferably about 0.1 - 10 mg/kg body weight, more preferably about 0.1 - 5 mg/kg body weight, about 1 - 5 times, preferably 1 - 3 times, per day, by intravenous injection. When other parenteral or oral administration is employed, an amount similar thereto can be administered. When the symptom is particularly severe, the amount may be increased according to the symptom.

Each composition described above may further contain other active ingredients (e.g., HSP90 inhibitor, pyrvinium pamoate etc. to be mentioned later) as long as blending thereof does not cause any adverse effect on the nucleic acid of the present invention.
Furthermore, the nucleic acid of the present invention may be used in combination with other medicaments, for example, alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etoposide, irinotecan, Taxotere, androgen lowering agents (e.g., LH-RH analogue (e.g., leuprorelin acetate etc.), LH-RH antagonists (e.g., degarelix)), androgen synthesis inhibitors (e.g., abiraterone etc.), anti-androgenic agents (e.g., flutamide, bicartamide etc.), synthetic adrenal cortex hormone drugs such as estrogen agents, prednisolone and the like, antibody drugs (e.g., trastuzumab, cetuximab, pertuzumab etc.), low-molecular-weight target drugs (e.g., lapatinib, imatinib, rapamycin, everolimus, temsirolimus etc.), cancer vaccine and the like. The nucleic acid and the above-mentioned medicament in the present invention can be administered to patients simultaneously or at different times.

### (2-3) Diagnostic reagent for ARaiv expressing cancer

In the present invention, ARaiv1 (here means a generic term of ARaiv containing C-terminal amino acid sequence shown by SEQ ID NO: 1) and ARaiv2 (here means a generic term of ARaiv containing C-terminal amino acid sequence shown by SEQ ID NO: 2) have a specific C-terminal amino acid sequence absent in full-length AR. Therefore, by examining the presence or absence of a protein having the amino acid sequence, or RNA containing a nucleotide sequence of the 4th exon of ARaiv1 and ARaiv2 encoding the amino acid sequence (SEQ ID NO: 13 and 14, respectively) in a cancer cell, whether the cancer cell expresses ARaiv1 or ARaiv2 can be determined.
Thus, the present invention also provides a diagnostic reagent for cancer that expresses ARaiv1 or ARaiv2, comprising a substance that specifically recognizes an amino acid sequence shown by SEQ ID NO: 1 or 2 or a nucleotide sequence shown by SEQ ID NO: 13 or 14. While the cancer patients to be the test subject are not particularly limited, they are preferably patients with prostate cancer, testis cancer, breast cancer, uterine cancer, ovarian cancer, lung cancer, colorectal cancer or pancreatic cancer, more preferably patients with relapsed androgen-independent prostate cancer. As the sample, biopsy and circulating tumor cell (CTC) can be used.
Examples of the substance that specifically recognizes an amino acid sequence shown by SEQ ID NO: 1 or 2 include an antibody for which the amino acid sequence is an epitope. Such antibody can be obtained by preparing a polyclonal antibody or monoclonal antibody according to a conventional method by chemically synthesizing a peptide comprising an amino acid sequence shown by SEQ ID NO: 1 or 2, immunizing a rabbit, mouse or the like with the peptide as an immunogen. ARaiv1 or ARaiv2 protein can be detected by an immunological method known per se (e.g., Western blotting, ELISA, immunohistochemistry, flow cytometry etc.). In addition, CTC detection system such as Cell Search System and the like may also be used in combination.
Examples of the substance that specifically recognizes a nucleotide sequence shown by SEQ ID NO: 13 or 14 include a nucleic acid containing a nucleotide sequence complementary to the whole or a part of the nucleotide sequence and/or a complementary chain thereof. Such nucleic acid can be obtained by chemical synthesis using a commercially available DNA/RNA automatic synthesizer. ARaiv1 or ARaiv2 mRNA can be detected by a nucleic acid detection method known per se such as Northern blotting, dot blotting, RT-PCR, in situ hybridization and the like. In addition, CTC detection system such as Cell Search System and the like can also be used in combination.
Patients judged to have cancer expressing ARaiv1 or ARaiv2 as a result of the above-mentioned diagnosis are preferable subjects of administration for the ARaiv expression/activity inhibitor of the present invention. Accordingly, the present invention also provides an agent for the prophylaxis or treatment of cancer containing an ARaiv expression/activity inhibitor, which is administered to patients diagnosed with cancer expressing ARaiv1 by using the above-mentioned diagnostic reagent.

### (3) Other ARaiv expression/activity inhibitor

### (3-1) HSP90 inhibitor

The ARaiv expression inhibitor in the invention is not limited by the aforementioned nucleic acid comprising a nucleotide sequence complimentary or substantially complimentary to the nucleotide sequence of ARaiv mRNA or a part of the nucleotide sequence, and any other substances such as low-molecular-weight compounds may also be employed as long as it inhibits directly or indirectly the production of ARaiv protein. Examples of such low-molecular-weight compound include the following HSP90 inhibitor and the like.
The HSP90 inhibitor to be used in the present invention is not particularly limited as regards the pattern of HSP90 inhibition and the like as long as it can inhibit function of ARaiv, that is, a transcription regulating action of an androgen responsive gene (e.g., activation of transcription of prostate specific antigen (PSA) gene, TMPRSS2 gene and the like, suppression of transcription of SERPIN B5 gene and the like) and, for example, a medicament that binds to ATP binding pocket of HSP90 to inhibit its chaperone function (e.g., geldanamycin and a derivative thereof), a medicament that binds to HSP90 at a site other than the ATP binding pocket to inhibit its function (e.g., celastrol, gedunin and the like) and the like can be mentioned.

Examples of the geldanamycin derivative include compounds described in Tian ZQ et al., Bioorg. Med. Chem. 12 (2004) 5317-5329, US Patent No. 5,387,584, US Patent No. 4,261,989, US Patent No. 3,987,035, WO 00/03737, WO 02/079167, WO 99/21552, and WO 99/22761. Preferable examples of the geldanamycin derivative include a compound obtained by substituting the 17-position methoxy group of geldanamycin by an alkylamino group (17-alkylamino-17-demethoxy geldanamycin). Such compound is advantageous in that it can reduce the toxicity of geldanamycin. Specifically, for example, 17-allylamino-17-demethoxy geldanamycin (17-AAG), 17-(2-dimethylaminoethyl)amino-17-demethoxy geldanamycin (17-DMAG), 17-(4-(dimethylamino)butyl)amino-17-demethoxy geldanamycin, 17-(2-(carboxy)ethyl)amino-17-demethoxy geldanamycin, 17-(2-(N-methylethylamino)ethyl)amino-17-demethoxy geldanamycin, 17-(2-(pyrrolidin-1-yl)ethyl)amino-17-demethoxy geldanamycin, 17-(2-(piperazin-1-yl)ethyl)amino-demethoxy geldanamycin, 17-(4-(dimethylamino)butyl)amino-17-demethoxy geldanamycin and the like can be used.

Examples of other HSP90 inhibitor include, but are not limited to, radicicol and a derivative thereof, adenosine derivative PU3 (WO02/036075), a 1,3-dihydroxybenzene derivative wherein a pyrazole ring is bonded (WO03/055860, WO2004/050087, WO2004/056782), a 1,3-dihydroxybenzene derivative wherein an isoxazole ring is bonded (WO2004/072051), a 1,3-dihydroxybenzene derivative wherein a pyrazole ring having a nitrogen-containing substituent directly bonded at the nitrogen atom is bonded (JP-A-2006-306755), celastrol, gedunin (Cancer Cell, 2006 10(4): 321-30) and the like.

The HSP90 inhibitor may be in the form of a salt of any of the above-mentioned compounds. Examples of the salt include hydrohalic acid salt (specifically hydrofluoride, hydrochloride, hydrobromide, hydroiodide etc.), inorganic acid salt (specifically sulfate, nitrate, perchlorate, phosphate, carbonate, bicarbonate etc.), organic carboxylic acid salt (specifically acetate, trifluoroacetate, oxalate, maleate, tartrate, fumarate, citrate etc.), organic sulfonate (specifically methanesulfonate, trifluoromethanesulfonate, ethanesulfonate, benzenesulfonate, toluenesulfonate, camphorsulfonate etc.), amino acid salt (specifically aspartate, glutamate etc.), quaternary amine salt, alkali metal salt (specifically sodium salt, potassium salt etc.), alkaline earth metal salt (specifically magnesium salt, calcium salt etc.) and the like.

Since an HSP90 inhibitor can inhibit the function of ARaiv (transcription regulating action on androgen responsive gene), it can be used as a growth inhibitor for an ARaiv1 expressing cancer cell or an agent for the prophylaxis or treatment of ARaiv1 expressing cancer (e.g., colorectal cancer, breast cancer, lung cancer, prostate cancer, esophagus cancer, gastric cancer, liver cancer, biliarycancer, spleen cancer, kidney cancer, urinary bladder cancer, uterine cancer, ovarian cancer, testis cancer, thyroid cancer, pancreatic cancer, brain tumor, blood tumor and the like, preferably, prostate cancer, testis cancer, breast cancer, uterine cancer, ovarian cancer, lung cancer, colorectal cancer, pancreatic cancer and the like, more preferably relapsed androgen-independent prostate cancer).

An ARaiv transcription regulating action inhibitor containing an HSP90 inhibitor can be administered directly as a liquid or a pharmaceutical composition in a suitable dosage form to a human or mammal (e.g., rat, mouse, rabbit, sheep, swine, bovine, cat, dog, monkey and the like) orally or parenterally (e.g., intravascular administration, subcutaneous administration and the like). The pharmaceutical composition to be used for the administration may contain an HSP90 inhibitor and a pharmacologically acceptable carrier, diluent or excipient.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations can be prepared by methods publicly known. For example, the injectable preparations may be prepared by dissolving, suspending or emulsifying HSP90 inhibitor in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], and the like. As an oily medium, for example, sesame oil, soybean oil and the like are used. It may be concurrently used with a solubilizing agent such as benzyl benzoate, benzyl alcohol and the like. A prepared injection is preferably filled in a suitable ampoule. A suppository to be used for rectal administration can also be prepared by mixing HSP90 inhibitor with a general base for suppository.

As a composition for oral administration, solid or liquid dosage form, specifically tablet (including sugar-coated tablet, film-coated tablet), pill, granule, powder, capsule (including soft capsule), syrup, emulsion, suspension and the like can be mentioned. Such composition is produced by a known method, and may contain a carrier, diluent or excipient generally used in the pharmaceutical field. As carrier or excipient for tablets, for example, lactose, starch, saccharose or magnesium stearate is used.

The above-mentioned parenteral or oral pharmaceutical composition is conveniently prepared in a dosage form of a dose unit suitable for the dose of an active ingredient. Examples of the dosage form for such dose unit include, for example, tablet, pill, capsule, injection (ampoule), and suppository. The HSP90 inhibitor is preferably contained in an amount of generally 5 - 500 mg per dose unit dosage form, particularly 5 - 100 mg for injection and 10 - 250 mg for other dosage form.

While the dose of the above-mentioned preparation containing the HSP90 inhibitor varies depending on the subject of administration, target disease, symptom, administration route and the like, for example, the HSP90 inhibitor is conveniently administered at, per one dose, generally about 0.01 - 20 mg/kg body weight, preferably about 0.1 - 10 mg/kg body weight, more preferably about 0.1 - 5 mg/kg body weight, for the treatment or prophylaxis of prostate cancer in adult, about 1 to 5 times, preferably about 1 to 3 times, per day by intravenous injection. In other parenteral administration and oral administration, the amounts similar thereto can be administered. When the symptom is particularly severe, the dose may be increased depending on the symptom.

The pharmaceutical composition containing an HSP90 inhibitor may contain other active ingredients as long as blending with an HSP90 inhibitor does not cause any unpreferable interaction.
Furthermore, the HSP90 inhibitor may be used in combination with other medicaments, for example, alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etoposide, irinotecan, LH-RH analogues (e.g., leuprorelin acetate etc.), anti-androgenic agents (e.g., flutamide, bicartamide etc.), androgen synthesis inhibitors (e.g., abiraterone etc.), antibody drugs (e.g., trastuzumab, cetuximab etc.) and the like. The HSP90 inhibitor and the above-mentioned medicaments may be administered to patients simultaneously or at different times. When it is used in combination with an antibody drug, the HSP90 inhibitor and the antibody are conjugated by a method known per se to give a molecular target drug. The HSP90 inhibitor and the antibody are preferably bound via a linker. The linker contains, for example, substituted or unsubstituted aliphatic alkylene chain, and contains, at the both terminals, a group that can be bound to a functional group of the HSP90 inhibitor or antibody, for example, an N-hydroxysuccinimide group, an ester group, a thiol group, an imidecarbonate group, an aldehyde group and the like (Koutai Kougaku Nyumon, Chijin Shokan, 1994).

### (3-2) ARaiv activity inhibitor

The ARaiv activity inhibitor in the present invention may be any as long as it inhibits ARaiv protein, once operably produced, from exhibiting an androgen responsive gene transcription regulating action and includes, for example, a substance that inhibits formation of an ARaiv-cofactor complex, a substance that inhibits binding of an ARaiv complex to a target gene, a substance that inhibits nuclear translocation of ARaiv, a substance that promotes degradation of ARaiv and the like.

In specific, the substance inhibiting the activity of ARaiv protein can be exemplified by antibodies against ARaiv protein. The antibody may be any of polyclonal antibody and monoclonal antibody. These antibodies can be manufactured in accordance with the method of manufacturing antibody or antisera, which is known per se. In this case, to obtain an antibody that specifically recognizes ARaiv from AR variants, it is desirable to use a partial peptide of a region encoded by the 4th exon of ARaiv, a junction region between 3rd exon and 4th exon as an immunogen, or manufacture a conformational antibody by a DNA immunization method. The isotype of the antibody is not particularly limited, but it is preferably IgG, IgM or IgA, particularly preferably IgG. The antibody is not particularly subject to limitation, as long as it has at least a complementality determining region (CDR) for specifically recognizing and binding to the target antigen; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like, and the like.
In a preferred mode of embodiment, since the antibody to the ARaiv protein is used as a pharmaceutical product targeting humans as the subject of administration thereof, the antibody (preferably a monoclonal antibody) is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the usual method. Although a fully human antibody can also be produced from human-human (or -mouse) hybridoma, it is desirable to produce it using a human antibody-producing mouse or the phage display method in order to stably supply the antibody in large amounts at low costs.

Since ARaiv is a nuclear receptor variant and plays a key role in the transcription regulation of an androgen responsive gene, an ARaiv activity inhibitor is desirably a substance superior in intracellular transferability and nuclear translocation. Therefore, a more preferred substance for inhibiting the activity of ARaiv is a low-molecular-weight compound obeying Lipinski's Rule. Such compound can be obtained by, for example, the aforementioned screening method of the present invention. When the above-mentioned anti-ARaiv antibody is used as an ARaiv activity inhibitor, it can be encapsulated in a liposome to enhance intracellular transferability. Preferable liposome includes positively-charged liposome, positively-charged cholesterol, membrane permeable peptide-bound liposome and the like (Mamoru Nakanishi et al., protein nucleic acid enzyme 44:1590-1596 (1999), Shiro Niki, Kagaku to Seibutsu 43:649-653 (2005), Clinical Cancer Research 59:4325-4333 (1999) and the like). To deliver an anti-ARaiv antibody selectively to the target cancer cell, moreover, the antibody can also be prepared as a bispecific antibody to a surface antigen molecule that is specifically highly expressed in the cancer cell.

Since the substance that inhibits a function of ARaiv in the present invention shuts off AR signal by inhibiting regulation of the transcription of an androgen responsive gene by ARaiv, it can be used as an agent for the prophylaxis and/or treatment of cancer, particularly ARaiv highly expressing cancer (e.g., colorectal cancer, breast cancer, lung cancer, prostate cancer, esophagus cancer, gastric cancer, liver cancer, biliarycancer, spleen cancer, kidney cancer, urinary bladder cancer, uterine cancer, ovarian cancer, testis cancer, thyroid cancer, pancreatic cancer, brain tumor, blood tumor and the like, preferably, prostate cancer, testis cancer, breast cancer, uterine cancer, ovarian cancer, lung cancer, colorectal cancer, pancreatic cancer and the like, more preferably relapsed androgen-independent prostate cancer).

### [Medicament containing an antibody to ARaiv or a low-molecular-weight compound inhibiting ARaiv expression or activity and the like]

A medicament containing an antibody to ARaiv or a low-molecular-weight compound inhibiting ARaiv expression or activity (e.g., HSP90 inhibitor, pyrvinium pamoate etc.) can be administered directly as a liquid or a pharmaceutical composition in a suitable dosage form to a human or mammal (e.g., mouse, rat, rabbit, sheep, swine, bovine, cat, dog, monkey and the like) orally or parenterally (e.g., intravascular administration, subcutaneous administration and the like).

The pharmaceutical composition comprising the above-mentioned antibody or low-molecular-weight compound may contain the above-mentioned antibody or low-molecular-weight compound or a salt thereof, and a pharmacologically acceptable carrier, diluent or excipient. Such pharmaceutical composition is provided in a dosage form suitable for oral or parenteral administration. Here, as the pharmacologically acceptable carrier, diluent and excipient, those similar to the carrier, diluent and excipient recited above for a preparation containing an HSP90 inhibitor can be preferably used.

The dose of the above-mentioned medicament containing the above-mentioned antibody or low-molecular-weight compound or a salt thereof varies depending on the subject of administration, target disease, symptom, administration route and the like. When used for the treatment or prophylaxis of prostate cancer in adult, the antibody or low-molecular-weight compound is preferably administered at, per one dose, generally about 0.01 - 20 mg/kg body weight, preferably about 0.1 - 10 mg/kg body weight, more preferably about 0.1 - 5 mg/kg body weight, about 1 - 5 times, preferably about 1 - 3 times, per day by intravenous injection. In other parenteral administration and oral administration, the amounts similar thereto can be administered. When the symptom is particularly severe, the dose may be increased depending on the symptom.

Each of the aforementioned compositions may contain other active ingredients as long as blending with the above-mentioned antibody or low-molecular-weight compound does not cause any unpreferable interaction.
Furthermore, the above-mentioned antibody or low-molecular-weight compound may be used in combination with other medicaments similar to those recited above for a medicament containing the nucleic acid of the present invention. The above-mentioned antibody or low-molecular-weight compound and other medicaments can be administered to patients simultaneously or at different times.

### (4) Combined use of ARaiv expression/activity inhibitor and AR signal inhibitor for ARaiv expressing prostate cancer

The substance that inhibits a function of ARaiv (i.e., the above-mentioned ARaiv expression or activity inhibitor) of the present invention can shut off at least the AR signal from ARaiv. In ARaiv expressing cancer cell, generally, full-length AR (androgen dependent AR) is also expressed simultaneously. As shown in the below-mentioned Examples, the ARaiv expressing cancer cell growth suppressive effect is higher by inhibition of both full-length AR and ARaiv than by specific inhibition of ARaiv alone. However, inhibition of not only ARaiv but also full-length AR by an ARaiv expression/activity inhibitor alone possibly requires increased doses. For example, since HSP90 is expressed in cancer cell as well as normal cell and responsible for various physiological functions, administration at a high dose is associated with feared expression of side effects.
Full-length AR has a ligand binding domain, and expresses AR activity in an androgen-dependent manner. Thus, transcription regulation of an androgen responsive gene can be inhibited by shutting off the supply of androgen itself, or co-presence of a substance that acts on AR competitively with androgen to shut off AR signal from the full-length AR. Therefore, by combining an AR signal inhibitor with the ARaiv expression/activity inhibitor of the present invention, the ARaiv expression/activity inhibitor can selectively act for inhibition of the ARaiv expression or activity. Consequently, the dose of the ARaiv expression/activity inhibitor can be reduced and the side effects can be decreased.
Accordingly, the present invention also provides an agent for the prophylaxis or treatment of ARaiv expressing cancer based on a combined use of an ARaiv expression/activity inhibitor of the present invention and an AR signal inhibitor. The subject of administration of the concomitant drug is not particularly limited as long as it is an animal having cancer expressing ARaiv. For example, it is an animal diagnosed using the diagnostic reagent of (4) below to have cancer expressing ARaiv.
While the ARaiv expression/activity inhibitor used here is not particularly limited as long as it is among those mentioned above, for example, a substance that specifically or preferentially inhibits expression or activity of ARaiv is preferable. In the case of an HSP90 inhibitor, for example, since it can exhibit an expression inhibitory effect at a lower concentration on ARaiv than on full-length AR, as shown in the below-mentioned Examples, HSP90 inhibitor is one preferable example of an ARaiv expression/activity inhibitor.
Examples of the AR signal inhibitor used here include, but are not limited to, androgen lowering agents (e.g., LH-RH analogue (e.g., leuprorelin acetate etc.), LH-RH antagonists (e.g., degarelix)), androgen synthesis inhibitors (e.g., abiraterone etc.), 5α reductase inhibitors (e.g., dutasteride etc.), AR antagonists (anti-androgen) (e.g., bicartamide, flutamide etc.), estrogen agent, etc., and the like.

In the above-mentioned combined use, the ARaiv expression/activity inhibitor can be administered to patients in an amount equal to or appropriately lower than the amount mentioned above for single administration. In addition, AR signal inhibitors can be each administered to patients in an amount equal to or appropriately lower than the dose known per se for single administration. The ARaiv expression/activity inhibitor and the AR signal inhibitor can be administered to patients simultaneously or at different times.

Abbreviations for bases, amino acids and the like used in the present specification are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA:: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDTA:: Ethylenediaminetetraacetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyroglutamic acid
- Sec:: Selenocysteine

The sequence identification numbers in the sequence listing of the present specification indicate the following sequences.
[SEQ ID NO: 1]
   This shows a C-terminal amino acid sequence specific to ARaiv1.
[SEQ ID NO: 2]
   This shows a C-terminal amino acid sequence specific to ARaiv2.
[SEQ ID NO: 3]
   This shows the nucleotide sequence of ARaiv1 cDNA.
[SEQ ID NO: 4]
   This shows the amino acid sequence of ARaiv1.
[SEQ ID NO: 5]
   This shows the nucleotide sequence of ARaiv2 cDNA.
[SEQ ID NO: 6]
   This shows the amino acid sequence of ARaiv2.
[SEQ ID NO: 7]
   This shows the nucleotide sequence of ARaiv1Δ1 cDNA.
[SEQ ID NO: 8]
   This shows the amino acid sequence of ARaiv1Δ1.
[SEQ ID NO: 9]
   This shows the nucleotide sequence of ARaiv1Δ2 cDNA.
[SEQ ID NO: 10]
   This shows the amino acid sequence of ARaiv1Δ2.
[SEQ ID NO: 11]
   This shows the nucleotide sequence of ARaiv1Δ3 cDNA.
[SEQ ID NO: 12]
   This shows the amino acid sequence of ARaiv1Δ3.
[SEQ ID NO: 13]
   This shows the nucleotide sequence of the 4th exon of ARaiv1 mRNA.
[SEQ ID NO: 14]
   This shows the nucleotide sequence of the 4th exon of ARaiv2 mRNA.
[SEQ ID NO: 15]
   This shows the CDS sequence of wild-type AR.
[SEQ ID NO: 16]
   This shows the amino acid sequence of wild-type AR.
[SEQ ID NO: 17]
   This shows the ARE sequence in human PSA promoter.
[SEQ ID NO: 18]
   This shows the ARE sequence in human PSA promoter.
[SEQ ID NO: 32]
   This shows a consensus ARE sequence.
Refer to the Examples for SEQ ID NO: 19 - 31, 33 - 52.

Escherichia coli TOP10/pcDNA3.1-hARaiv1 harboring ARaiv1 clone obtained in Example 3 has been deposited at the International Patent Organism Depositary (IPOD), National Institute of Advanced Industrial Science and Technology (Chuo 6, 1-1-1, Higashi, Tsukuba, Ibaraki, 305-8566 Japan) since July 15, 2009 (accession number: FERM BP-11144).
Escherichia coli TOP10/pcDNA3.1-hARaiv1Δ1 harboring ARaiv1Δ1 clone obtained in Example 3 has been deposited at the International Patent Organism Depositary (IPOD), National Institute of Advanced Industrial Science and Technology (Chuo 6, 1-1-1, Higashi, Tsukuba, Ibaraki, 305-8566 Japan) since July 15, 2009 (accession number: FERM BP-11145).
Escherichia coli TOP10/pcDNA3.1-hARaiv1Δ2 harboring ARaiv1Δ2 clone obtained in Example 3 has been deposited at the International Patent Organism Depositary (IPOD), National Institute of Advanced Industrial Science and Technology (Chuo 6, 1-1-1, Higashi, Tsukuba, Ibaraki, 305-8566 Japan) since July 15, 2009 (accession number: FERM BP-11146).
Escherichia coli TOP10/pcDNA3.1-hARaiv1Δ3 harboring ARaiv1Δ3 clone obtained in Example 3 has been deposited at the International Patent Organism Depositary (IPOD), National Institute of Advanced Industrial Science and Technology (Chuo 6, 1-1-1, Higashi, Tsukuba, Ibaraki, 305-8566 Japan) since July 15, 2009 (accession number: FERM BP-11147).
Escherichia coli TOP10/pGEM-T easy-hARaiv2-3'cDNA harboring ARaiv2 clone obtained in Example 2 has been deposited at the International Patent Organism Depositary (IPOD), National Institute of Advanced Industrial Science and Technology (Chuo 6, 1-1-1, Higashi, Tsukuba, Ibaraki, 305-8566 Japan) since July 15, 2009 (accession number: FERM BP-11148). Examples

The present invention is explained in more detail in the following by referring to Examples and Reference Examples, which are not to be construed as limitative.

### Example 1:

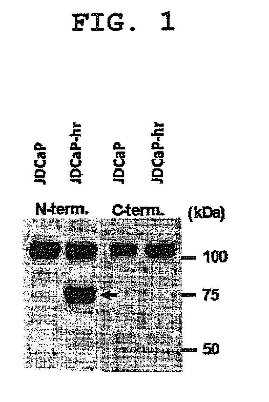

A biopsy sample obtained from the skin metastatic focus of prostate cancer patient was cut in thickness 2 - 3 mm, and the tumor fragments were subcutaneously transplanted to male nude mice (BALB-nu/nu, Charles River Japan). The tumor developed in the cancer-bearing mouse was named JDCaP tumor. The JDCaP tumor was maintained by repeated subcutaneous transplantation into the male nude mice. The JDCaP tumors were completely regressed by castrating the cancer-bearing mice , and the mice were reared for about 6 months to allow formation of relapsed tumor. The relapsed tumor was named JDCaP-hr (hormone refractory).
The JDCaP-hr cell line was established from JDCaP-hr tumor. The JDCaP-hr tumor was minced, and cultured in phenol red-free RPMI1640 (Invitrogen) containing 10% DCC-FBS. After several passages, the medium was changed to RPMI1640 containing 3% DCC-FBS and 100 ng/ml IGF (JRH Biosciences) for purification by suppressing the growth of mouse-derived stromal cells.
AR proteins in the JDCaP tumor and JDCaP-hr tumor were analyzed by Western blotting by using a specific antibody to the N-terminal (N-20) or C-terminal (C-19) portion of AR (Santa Cruz). As a loading control, β-actin was detected by using an AC-15 antibody (Sigma). The results are shown in Fig. 1. C-terminally defective-type AR protein (about 75 kDa) was found in an AIPC strain, JDCaP-hr.

### Example 2:

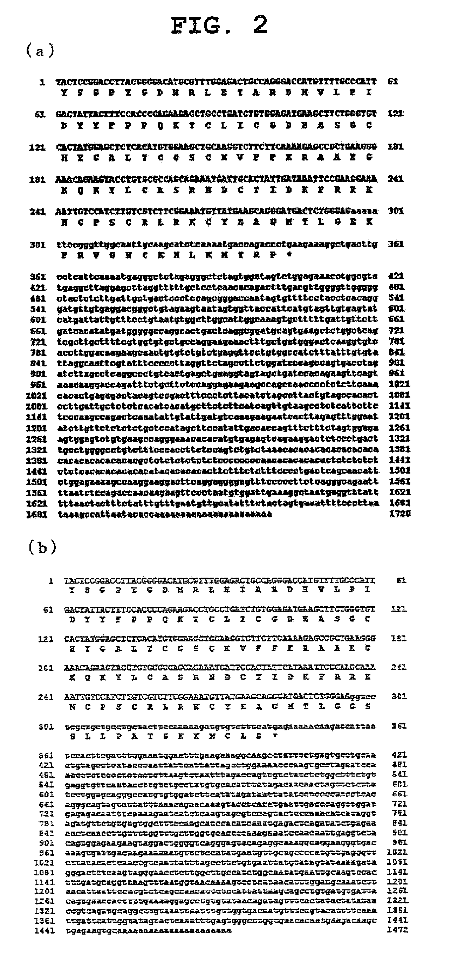

Total RNA was extracted from cultured cells and xenograft tumor of JDCaP-hr by using RNeasy Mini kit (Invitrogen) and RNase-Free DNase set (Qiagen), and 3' rapid amplification of cDNA end (RACE) was performed using GeneRacer kit (Invitrogen). For primary PCR, a forward primer specific to AR exon 1 (5'-gcatggtgagcagagtgccctat-3'; SEQ ID NO: 19) and a reverse primer attached to the kit were used. Then, using a diluted solution of the primary PCR reaction mixture as a template, and a nested forward primer specific to exon 1 (5'-tactccggaccttacggggacat-3'; SEQ ID NO: 20) and a reverse primer attached to the kit, nested PCR was performed. The obtained 3'-RACE product was cloned, and the nucleotide sequence was analyzed by Big Dye Terminator v3.1 and ABI PRISM7700 (Applied Biosystems). As a result, two kinds of clones in which a part of AR intron 3 was linked to immediate downstream of AR exon 3 to produce an in-frame stop codon were found in addition to known AR 3'sequence clone (Figs. 2a and b). As translation products of AR variants having these 3' sequences, molecules having AF1, DNA binding domain and a part of hinge region and lacking ligand binding domain were predicted. Since AR lacking ligand binding domain is expected to be androgen-independent, an AR variant having the 3' terminal of SEQ ID NO: 1, and characterized by the C-terminal amino acid sequence EKFRVGNCKHLKMTRP (SEQ ID NO: 1) was named ARaiv1 (AR androgen-independent variant 1), and an AR variant having the 3' terminal of GSSLLPATSKKMCLS (SEQ ID NO: 2) was named ARaiv2 (AR androgen-independent variant 2).

### Example 3:

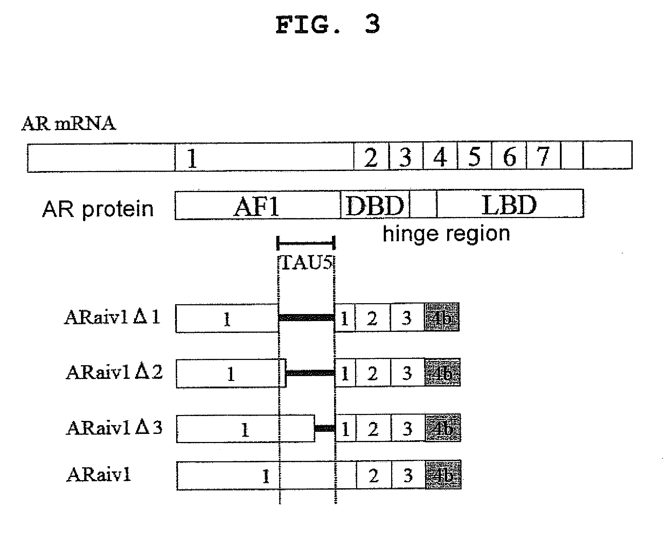

cDNA was prepared from JDCaP-hr RNA by using SuperScript III First-Strand Synthesis System for RT-PCR (Invitrogen). Using PfuUltra II Fusion HS DNA Polymerase (Stratagene), a forward primer containing AR initiation codon (5'-atggaagtgcagttagggctgggaag-3'; SEQ ID NO: 21) and a reverse primer specific to exon 4b encoding the 3' terminal of ARaiv1 transcript (5'-ggtctggtcattttgagatgcttgcaatt-3'; SEQ ID NO: 22), ARaiv1 ORF was amplified from the obtained cDNA. The obtained ORF fragment was cloned to a pGEM-T easy vector (Promega), and the sequence was analyzed. As a result, three kinds of clones having an in-frame defect in the TAU5 (transcriptional activation unit 5) region of exon 1 were obtained (Fig. 3). These ARaiv1 clones were named ARaiv1Δ1, ARaiv1Δ2 and ARaiv1Δ3, respectively.

### Example 4:

cDNA was prepared from RNA of CWR22Rv1 cell by using SuperScript III First-Strand Synthesis System for RT-PCR (Invitrogen). Using PfuUltra II Fusion HS DNA Polymerase (Stratagene), a forward primer containing AR initiation codon (5'-atggaagtgcagttagggctgggaag-3'; SEQ ID NO: 21) and a reverse primer specific to exon 4b (5'-ggtctggtcattttgagatgcttgcaatt-3'; SEQ ID NO: 22), ARaiv1 ORF was amplified from the obtained cDNA. The obtained ORF fragment was cloned to a pGEM-T easy vector (Promega), and the sequence was analyzed. As a result, ORF clone of ARaiv1 having intact exon 1 and no defect on the N-terminal side was obtained (Fig. 3).

### Example 5:

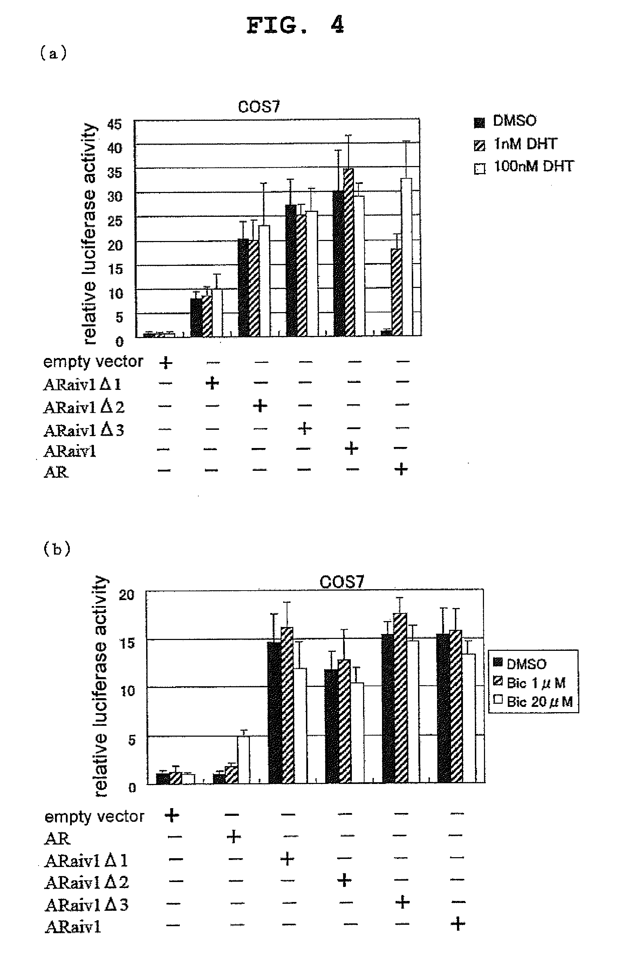

The above-mentioned 4 kinds of ARaiv1 clones (ARaiv1 and ARaiv1Δ1, ARaiv1Δ2 and ARaiv1Δ3) were respectively subcloned to a pcDNA3.1 expression vector, and the transcription activity was examined by a reporter gene assay. The ARaiv1 clone expression vector and pGL3-2xPSA reporter were cotransfected into COS7 cell. After 24 hr, DHT or an AR antagonist bicalutamide, was added, and 24 hr later, a luciferase assay was performed. As a result, the 4 kinds of ARaiv1 clones all showed a DHT independent increase of the reporter activity (Fig. 4a). It has also been clarified that the transcription activity of 4 kinds of ARaiv1 clones is not inhibited by bicalutamide (Fig. 4b).

### Example 6:

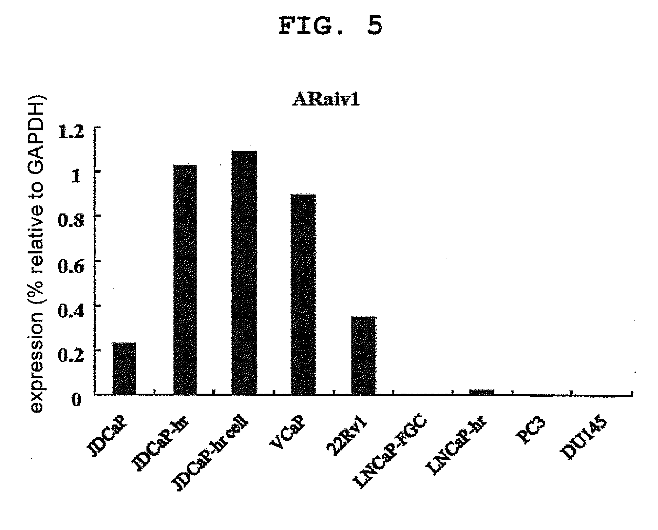

The expression of ARaiv1 and AR in various prostate cancer cell lines was examined by a quantitative RT-PCR method. The quantitative RT-PCR was carried out using ABI PRISM7700 (Applied Biosystems), QuantiTect Probe RT-PCR kit (Qiagen) and gene specific primers and probes. The primers and probes used were AR (forward primer: 5'-aaatgttatgaagcagggatgactct-3' (SEQ ID NO: 23); reverse primer: 5'-gcttctgggttgtctcctcagt-3' (SEQ ID NO: 24); probe: 5'-FAM-actacaggaggaaggaga-MGB-3' (SEQ ID NO: 25)), ARaiv1 (forward primer: 5'-catcttgtcgtcttcggaaatg-3' (SEQ ID NO: 26); reverse primer: 5'-tgccaacccggaatttttct-3' (SEQ ID NO: 27); probe: 5'-FAM-cagggatgactctggg-MGB-3' (SEQ ID NO: 28)), and GAPDH (Hs99999905, TaqMan^{R} Gene Expression Assays, Applied Biosystems). As a result, high expression of ARaiv1 was found in JDCaP-hr cells, 22Rv1 cells and VCaP cells (Fig. 5) .

### Example 7:

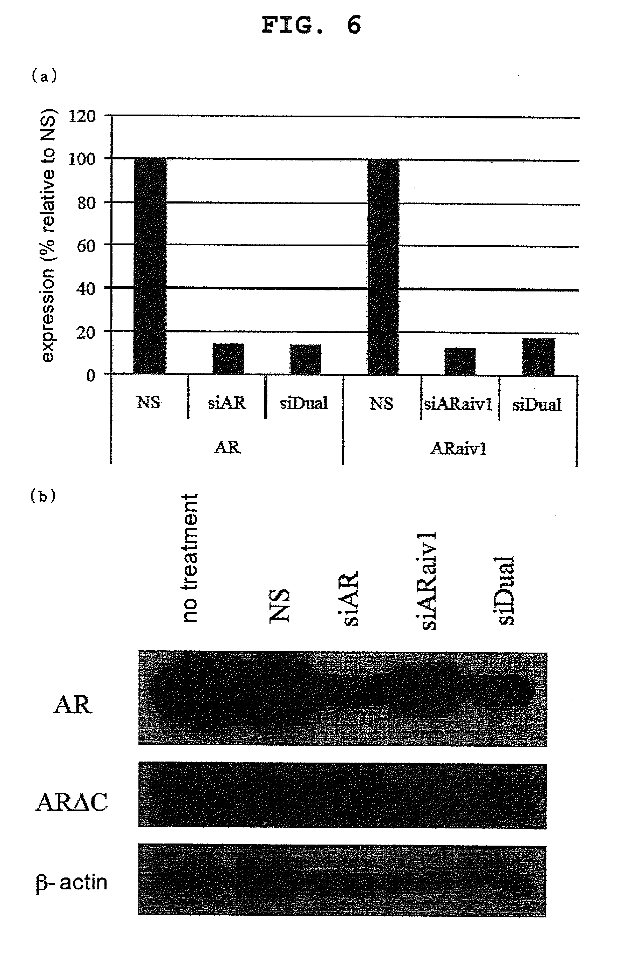

An siRNA targeting exon 4b specific to ARaiv1 (siARaiv1: 5'-gattgttcttgatcacata-3' (SEQ ID NO: 29)) was designed and synthesized by Qiagen. In addition, an siRNA targeting exon 8 specific to AR (siAR: Hs_AR_6_HP Validated siRNA: 5'-ggaacucgaucguaucauu-3' (SEQ ID NO: 30)) and an siRNA cocktail containing siRNA targeting exon 1 common to AR and ARaiv1 (siDual: OTP SMART pool, L-003400-00-0005) were purchased from Qiagen and Dharmacon, respectively. siAR, siARaiv1 or siDual was transfected into JDCaP-hr cells. RNA was extracted from the cells after 48 hr from the siRNA transfection, and the expression level of the target gene was analyzed by quantitative RT-PCR. As a result, it was confirmed that gene knockdown by each siRNA was carried out sufficiently efficiently (Fig. 6a). To examine changes in the C-terminally defective-type AR protein production, which were caused by the ARaiv1 knockdown, an extract was prepared from the cells after 48 hr from the siRNA transfection, and the production of AR and β-actin was examined by Western blotting. The production of only full-length AR in the siAR transfection group, only C-terminally defective-type AR in the siARaiv1 transfection group, and the both in the siDual transfection group decreased (Fig. 6b). These results showed that ARaiv1 actually encodes C-terminally defective-type AR.

### Example 8:

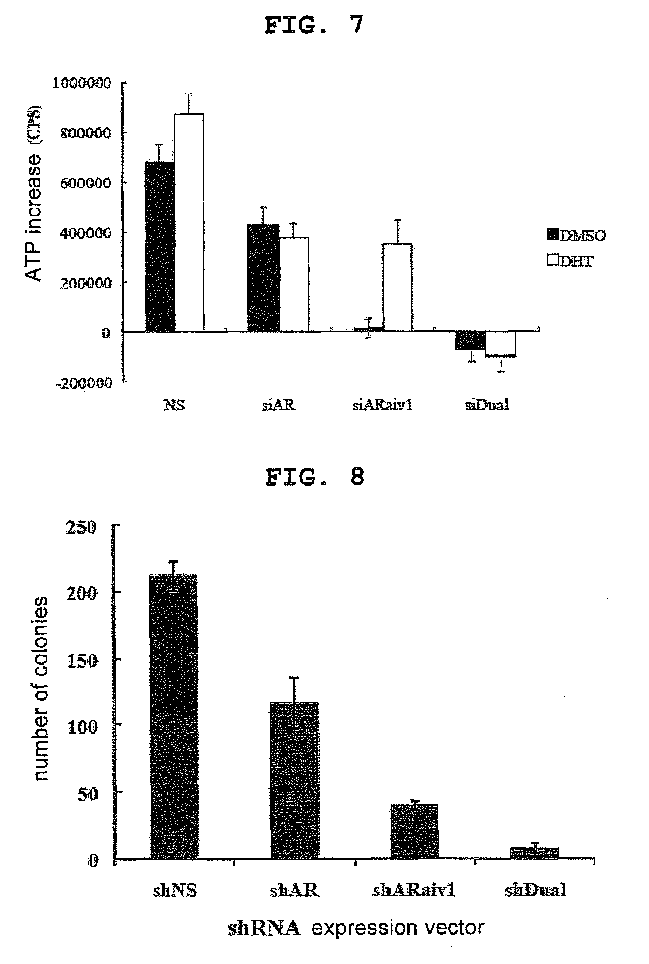

siRNA targeting AR-specific exon 8 (siAR), siRNA targeting ARaiv1-specific exon 4b (siARaiv1) or siRNA targeting exon 1 common to the both (siDual) was transfected into JDCaP-hr cells, the cells were cultured for 5 days in an androgen-free medium added with DHT or DMSO, and the cell proliferation was measured (Fig. 7). Under the androgen-free conditions, a remarkable growth inhibition was observed in the siARaiv1 and siDual transfection groups. On the other hand, the growth inhibitory effect was partial in the siAR transfection group. When DHT was added, a growth promotion due to DHT was observed in the siARaiv1 transfection group, whereas a growth promotion due to DHT was remarkably inhibited in the siAR and siDual transfection groups. These results show that ARaiv1 is essential for the growth of the JDCaP-hr cell in the absence of androgen.

### Example 9:

Expression plasmids of shRNA targeting AR-specific exon 8 (shAR), shRNA targeting ARaiv1-specific exon 4b (shARaiv1) and shRNA targeting exon 1 common to the both (shDual) were constructed using siSTRIKE U6 hairpin cloning system (Promega). The target sequences of shAR and shΔRaiv1 were the same as those of the above-mentioned siAR and siARaiv1. As the target sequence of shRNA specific to exon 1 (shDual), the sequence of exon 1-specific siRNA (5'-gcagaaatggattgcactat-3' (SEQ ID NO: 31)) contained in siDual was used.
The expression vector of shAR, shARaiv1 or shDual was transfected into 22Rv1 cells, and a colony formation assay was carried out under androgen-free conditions. The results are shown in Fig. 8. In the shARaiv1 transfection group, the number of colonies decreased to 19% of that of the non-specific shRNA (shNS) transfection group (NS group). In the shDual transfection group, the number of colonies decreased to 4% of that of the NS group, and remarkable suppression of colony formation was observed. On the other hand, the colony formation ability of the shAR transfection group was 54% of the NS group, and the decrease was weaker as compared to the ARaiv1 knockdown group. These results show that ARaiv1 plays an important role in the growth of 22Rv1 cells. In addition, the results show that inhibition of both ARaiv1 and full-length AR more strongly suppresses the growth of AIPC.

### Example 10:

A homology analysis was carried out between the 3'-UTR nucleotide sequence of ARaiv1 and not less than 400 kinds of the nucleotide sequences of human miRNA. As a result, hsa-mir-98, hsa-let-7a-1/2/3, hsa-let-7b, hsa-let-7c, hsa-let-7d, hsa-let-7e, hsa-let-7f-1/2, hsa-let-7g, hsa-let-7i, hsa-miR-125a-5p, hsa-miR-134, hsa-miR-149*, hsa-miR-491-5p, and hsa-miR-574-5p were considered to be able to bind to 3'-UTR of ARaiv1 (Tables 1-1 and 1-2).

**[Table 1-1]**

| microRNA ID | microRNA sequence (SEQ ID NO:) | position on ARaiv1 3'-UTR | | microRNA sequence [3'⇒5'] alignment ARaiv1 3'-UTR sequmm [5'⇒3'] (SEQ ID NO:) |
|---|---|---|---|---|
| hsa-mir-98 | UGAGGUAGUAAGUUGUAUUGUU (33) | 169 | 191 | 3' UUGUUAU-GUUGAAUGAUGGAGU 5' ; ;;;;;  :: :;; ;;;;;;;; 5' ACCAATAGTGTTTTCCTACCTCA 3'   (47) |
| hsa-let-7a-1/2/3 | UGAGGUAGUAGGUUGUAUAGUU (34) | 169 | 191 | 3' UUGAUAU-GUUGGAUGAUGGAGU 5' ; ; ;;;  :: ::; ;;;;;;;; 5' ACCAATAGTGTTTTCCTACCTCA 3' |
| hsa-let-7b | UGAGGUAGUAGGUUGUGUGGUU (35) | 169 | 191 | 3' UUGGUGU-GUUGGAUGAUGGAGU 5' ; ; ;:; :: ::; ;;;;;;;; 5' ACCAATAGTGTTTTCCTACCTCA 3' |
| hsa-let-7c | UGAGGUAGUAGGUUGUAUGGUU (36) | 169 | 191 | 3' UUGGUAU-GUUGGAUGAUGGAGU 5' ; ; ;;; :: ::; ;;;;;;;; 5' ACCAATAGTGTTTTCCTACCTCA 3' |
| hsa-let-7d | AGAGGUAGUAGGUUGCAUAGU (37) | 169 | 191 | 3' UG-AUA-CGUUGGAUGAUGGAGA 5' ;;  ;; ;:: ::; ;;;;;;; 5' ACCAATAGTGTTTTCCTACCTCA 3' |
| hsa-let-7e | UGAGGUAGGAGGUUGUAUAGU (38) | 169 | 191 | 3' UG-AUAU-GUUGGAGGAUGGAGU 5' ;;  ;;; :: ::;;;;;;;;;; 5' ACCAATAGTGTTTTCCTACCTCA 3' |
| hsa-let-7f-1/2 | UGAGGUAGUAGAUUGUAUAGUU (39) | 169 | 191 | 3' UUGAUAU-GUUAGAUGAUGGAGU 5' ; ; ;;;  :: ;:; ;;;;;;;; 5' ACCAATAGTGTTTTCCTACCTCA 3' |

**[Table 1-2]**

| microRNA ID | microRNA sequence (SEQ ID NO:) | position on ARaiv1 3'-UTR | | microRNA sequence [3'⇒5] alignment ARaiv1 3'-UTR sequence [5'⇒3'] (SEQ ID NO:) |
|---|---|---|---|---|
| hsa-let-7g | UGAGGUAGUAGUUUGUACAGU (40) | 169 | 191 | 3' UG-ACAU-GUUUGAUGAUGGAGU 5' ;;   ;; ::  :; ;;;;;;;; 5' ACCAATAGTGTTTTCCTACCTCA 3'   (47) |
| hsa-let-7i | UGAGGUAGUAGUUUUGUGCUGU (41) | 169 | 191 | 3' UG-UCGU-GUUUGAUGAUGGAGU 5' ;; ; :; ::  :; ;;;;;;;; 5' ACCAATAGTGTTTTCCTACCTCA 3' |
| hsa-miR-125a-5p | UCCCUGAGACCCUUUAACUGA (42) | 1178 | 1209 | 3' aguGUCCAAUUUCCC————————AGAGUCCCU 5' ;;;;   ;:;;;          ;;;;;;;; 5' cttCAGG——AGGGGAGTTTCCCCCTTCTCAGGGC 3'  (48) |
| hsa-miR-134 | UGUGACUGGUGACCAGAGGGG (43) | 528 | 552 | 3'  ggGGAGACC-A---GUUGGUCAGUGU  5' ;:;;;;; ;  ;;:;;;;; ;; 5'  gcCTTCTGGATCCCAGCCAGTGACC  3'   (49) |
| hsa-miR-149* | AGGGAGGGACGGGGGCUCUGC (44) | 1045 | 1069 | 3'  cgUGU-CG---GGGGCAGGGAGGGa  5' ;;;  ;;      ;:;:  ;;:;;;;; 5'  acACACGCTCTCTCTCTCTCTCCCc  3'   (50) |
| hsa-miR-491-5p | AGUGGGGAACCCUUCCAUGAGG (45) | 1178 | 1201 | 3'  ggAGUACCUUCCC---AAGGGGuga 5' ;;; ;;;:;;;    ;;;;;; 5'  ctTCA-GGAGGGGAGTTTCCCCctt 3'  (51) |
| hsa-miR-574-5p | UGAGUGUGUGUGUGUGAGUGUGU (46) | 1015 | 1037 | 3'  ugUGUGAGUGUGUGUGUGUGAGU  5' ;;;;  ;;;;;;;;;;;;;  ;; 5'  aaACACACACACACACACACACACA  3'    (52) |

### Example 11:

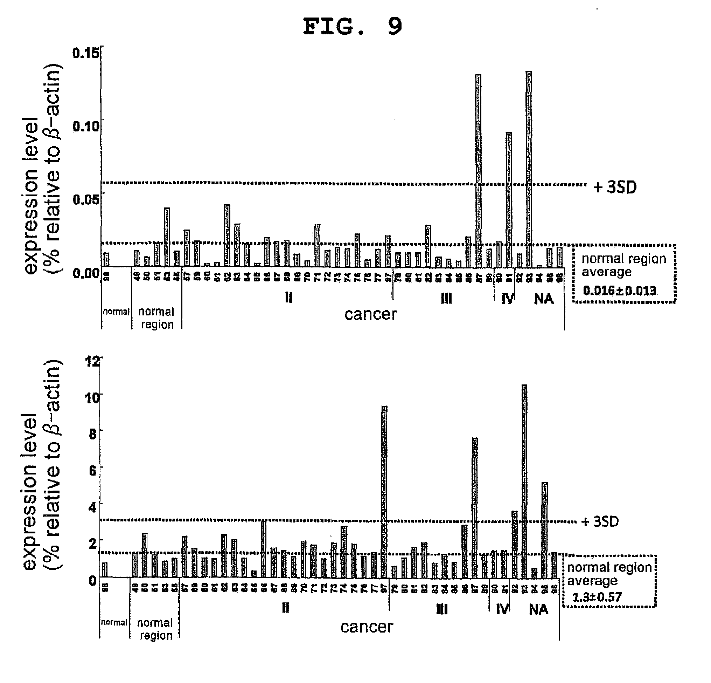

Using Tissue Scan^{™} Real-Time, Prostate Cancer Array II (Origene) as cDNA of clinical prostate cancer, the expression levels of AR, ARaiv1 and β-actin were measured by quantitative RT-PCR. The quantitative RT-PCR was carried out using ABI PRISM7700 (Applied Biosystems), QuantiTect Probe RT-PCR kit (Qiagen) and gene specific primers and probes. The primers and probes used were AR (forward primer: 5'-aaatgttatgaagcagggatgactct-3' (SEQ ID NO: 23); reverse primer: 5'-gcttctgggttgtctcctcagt-3' (SEQ ID NO: 24); probe: 5'-FAM-actacaggaggaaggaga-MGB-3' (SEQ ID NO: 25)), ARaiv1 (forward primer: 5'-catcttgtcgtcttcggaaatg-3' (SEQ ID NO: 26); reverse primer: 5'-tgccaacccggaatttttct-3' (SEQ ID NO: 27); probe: 5'-FAM-cagggatgactctggg-MGB-3' (SEQ ID NO: 28)), and β-actin (Human ACTB, Pre-Developed TaqMan^{R} Assay Reagents, PE Biosystems). The results are shown in Fig. 9. Of the 38 samples measured, 3 samples (7.9%) showed a remarkable increase of ARaiv1 expression, and 5 samples (13.2%) showed an increase of AR expression. Among those, 2 samples (5.3%) showed an increase of both ARaiv1 and AR expression.

### Example 12:

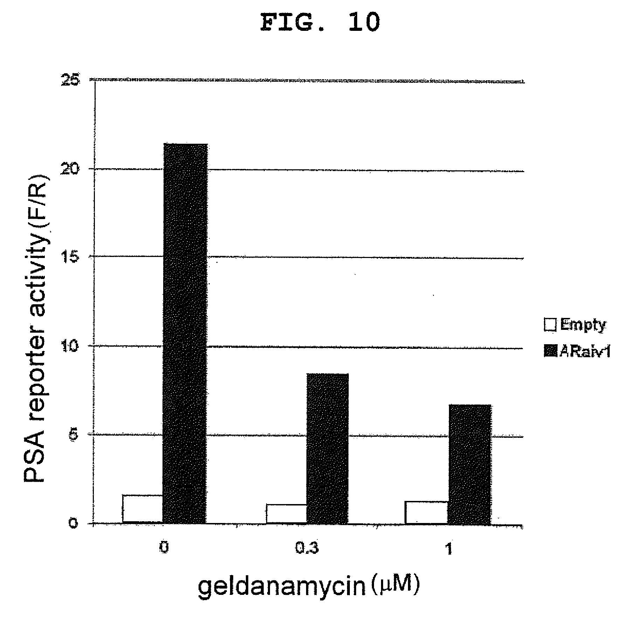

COS7 cells were seeded at 10⁶ cells/6 cm diameter culture dish and, 24 hr later, 200 ng of ARaiv1 expression plasmid and 7 µg of 2xPSA reporter plasmid were transfected using lipofectoamine2000. After 6 hr, the cells were detached therefrom, and seeded again in RPMI1640 medium containing 10% steroid-free fetal bovine serum in a 96 well culture plate. After 24 hr, geldanamycin or solvent DMSO was added and the culture was continued. After 24 hr, the luciferase activity was measured by Bright-Glo luciferase assay system (Promega). As a result, transcription activity of ARaiv1 was inhibited by geldanamycin (Fig. 10).

### Example 13:

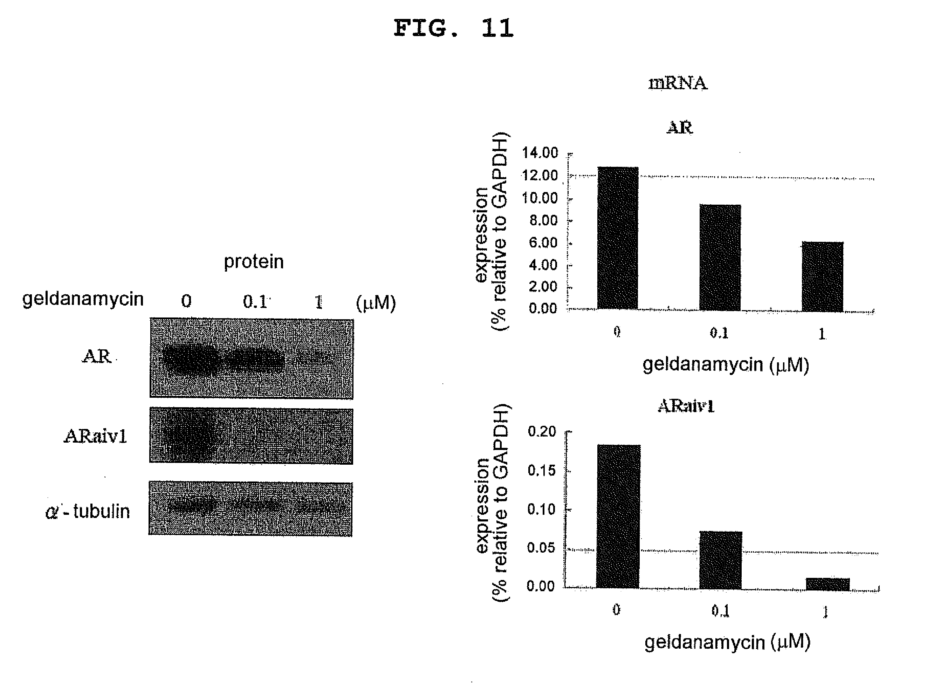

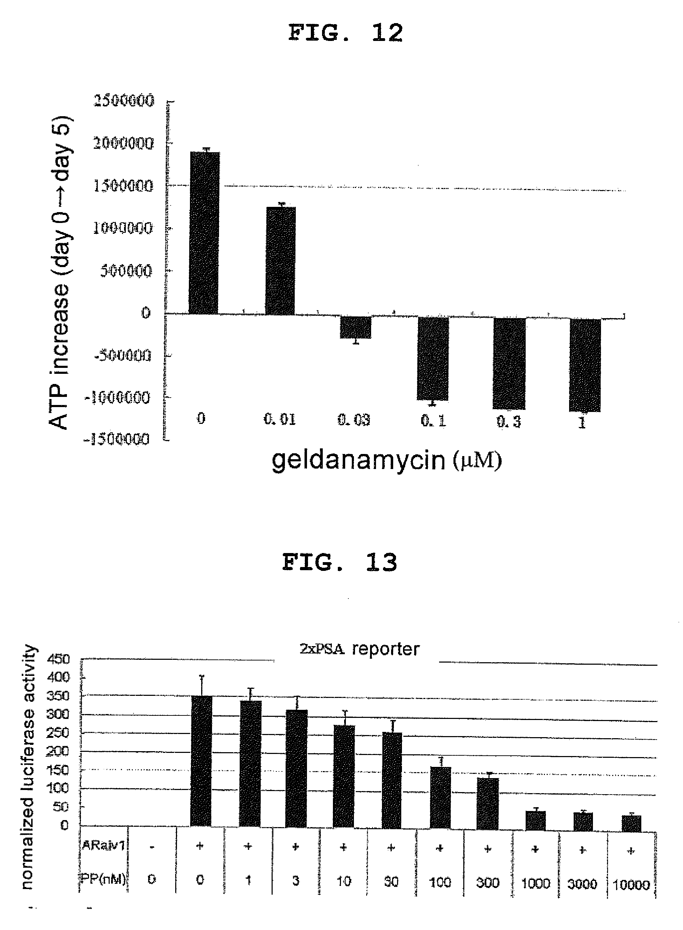

Geldanamycin was added to JDCaP-hr cells, and an influence on the cell proliferation, ARaiv1 mRNA expression and ARaiv1 protein production were examined. It was clarified that geldanamycin remarkably reduced ARaiv1 mRNA and protein production (Fig. 11), and remarkably inhibited the growth of JDCaP-hr (Fig. 12). Geldanamycin showed a more preferential and remarkable mRNA expression/protein production lowering action on ARaiv1 than on full-length AR (Fig. 11).

### Example 14:

Pyrvinium pamoate (PP) has been reported to inhibit the transcription activity of AR according to an action mechanism different from androgen antagonism. An influence of PP on the transcription activity of ARaiv1 was examined by a reporter gene assay. An empty vector or ARaiv1 expression vector was transfected into LNCaP-FGC with 2x PSA reporter, PP was added 24 hr later, and a luciferase assay was carried out 24 hr later. As a result, it was clarified that PP inhibits transcription by ARaiv1 in a concentration-dependent manner (Fig. 13).

### Example 15:

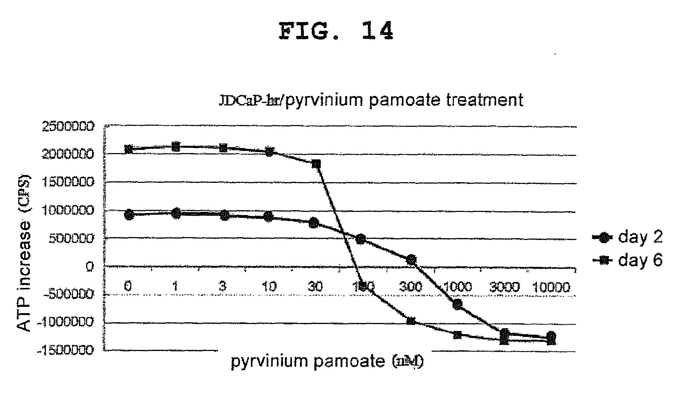

Pyrvinium pamoate (PP) was added to JDCaP-hr cells and an influence on the cell proliferation was examined. It was clarified that PP remarkably inhibits the growth of JDCaP-hr (Fig. 14).

### Industrial Applicability

Using inhibition of transcriptional activation by ARaiv and the like as an index, a substance effective for the prophylaxis or treatment of ARaiv expressing cancer can be screened for. An antisense nucleic acid, siRNA, miRNA, or HSP90 inhibitor capable of specifically inhibiting expression of ARaiv and other substance that inhibits expression or activity of ARaiv are useful for the treatment and prevention of recurrence of ARaiv expressing cancer. In addition, detection of the C-terminal sequence of ARaiv enables diagnosis of ARaiv expressing cancer.

This application is based on a patent application No. 2009-225541 filed in Japan on September 29, 2009, the contents of which are incorporated in full herein.

## Claims

1. A method of screening for a substance for the prophylaxis or treatment of cancer, comprising
a step of contacting a cell comprising a nucleic acid encoding a variant androgen receptor protein comprising the amino acid sequence shown by SEQ ID NO: 6, 8, 10 or 12, and a reporter gene under control of an androgen responsive promoter with a test compound,
a step of measuring the expression level of the reporter gene, and
a step of selecting a compound that suppresses the expression of the reporter gene as compared to a control without contact with the test compound.

2. A method of screening for a substance for the prophylaxis or treatment of cancer, comprising
a step of contacting a variant androgen receptor protein comprising the amino acid sequence shown by SEQ ID NO: 6, 8, 10 or 12 with a test compound,
a step of measuring the activity of the protein, and
a step of selecting a compound that suppresses the activity of the protein as compared to a control without contact with the test compound.

3. A variant androgen receptor protein comprising the amino acid sequence shown by SEQ ID NO: 6, 8, 10 or 12.

4. A nucleic acid encoding the protein of claim 3.

5. A cell transduced with the nucleic acid of claim 4.

6. A nucleic acid of any of the following (a) - (e),
(a) an antisense nucleic acid for a nucleic acid encoding the protein of claim 3
(b) siRNA for RNA encoding the protein of claim 3
(c) a nucleic acid capable of generating siRNA for RNA encoding the protein of claim 3
(d) miRNA for RNA encoding the protein of claim 3.
(e) a nucleic acid capable of generating miRNA for RNA encoding the protein of claim 3.

7. A method of evaluating the efficacy of a substance for the prophylaxis or treatment of cancer, comprising a step of contacting a test compound with an animal or tissue thereof into which the nucleic acid of claim 4 has been transduced, and measuring growth, differentiation or cell death, canceration or cancer growth of the tissue.

8. A diagnostic reagent for cancer expressing a variant androgen receptor lacking a ligand binding domain, which comprises a substance that specifically recognizes the amino acid sequence shown by SEQ ID NO: 2 or the nucleotide sequence shown by SEQ ID NO: 14.
